(19) Europäisches Patentamt
European Patent Office
Office européen des brevets

(11) EP 0 871 765 B1

(12) EUROPEAN PATENT SPECIFICATION

(45) Date of publication and mention of the grant of the patent: **28.11.2001 Bulletin 2001/48**

(21) Application number: **95912584.0**

(22) Date of filing: **23.02.1995**

(51) Int Cl.[7]: **C12Q 1/68**

(86) International application number: **PCT/US95/02203**

(87) International publication number: **WO 95/23874 (08.09.1995 Gazette 1995/38)**

(54) **DETERMINATION OF CANCEROUS CONDITIONS BY MAGE GENE EXPRESSION**

BESTIMMUNG KREBSARTIGER BESCHAFFENHEITEN DURCH DIE GENEXPRESSION EINES MITGLIEDES DER MELANOMARTIGENGRUPPE, MAGE

DETERMINATION D'ETATS CANCEREUX PAR L'EXPRESSION DE GENES MAGE

(84) Designated Contracting States: **AT BE CH DE DK ES FR GB GR IE IT LI LU MC NL PT SE**

(30) Priority: **01.03.1994 US 204727**
**10.03.1994 US 209172**
**01.09.1994 US 299849**
**30.11.1994 US 346774**

(43) Date of publication of application: **21.10.1998 Bulletin 1998/43**

(73) Proprietor: **LUDWIG INSTITUTE FOR CANCER RESEARCH New York, New York 10105 (US)**

(72) Inventors:
- **DE PLAEN, Etienne B-1200 Brussels (BE)**
- **BOON-FALLEUR, Thierry B-1200 Brussels (BE)**
- **LETHE, Bernard B-1200 Brussels (BE)**
- **SZIKORA, Jean-Pierre B-1200 Brussels (BE)**
- **DE SMET, Charles B-1200 Brussels (BE)**
- **CHOMEZ, Patrick B-1200 Brussels (BE)**
- **GAUGLER, Beatrice B-1200 Brussels (BE)**
- **VAN DEN EYNDE, Benoit B-1200 Brussels (BE)**
- **BRASSEUR, Francis B-1200 Brussels (BE)**
- **PATARD, Jean-Jacques, Hôpital Henry Mondor 94010 Creteil Cedex (FR)**
- **WEYNANTS, P. B-1200 Brussels (BE)**
- **MARCHAND, M. B-1200 Brussels (BE)**
- **VAN DER BRUGGEN, Pierre B-1200 Brussels (BE)**

(74) Representative: **Harrison Goddard Foote Belgrave Hall Belgrave Street Leeds LS2 8DD (GB)**

(56) References cited:
**WO-A-92/20356** **US-A- 5 342 774**

- **ZAKUT R ET AL: "DIFFERENTIAL EXPRESSION OF MAGE-1, -2, AND -3 MESSENGER RNA IN TRANSFORMED AND NORMAL HUMAN CELL LINES" CANCER RESEARCH, vol. 53, no. 1, 1 January 1993, pages 5-8, XP000578279**
- **VAN DER BRUGGEN ET AL: "A GENE ENCODING AN ANTIGEN RECOGNIZED BY CYTOLYTIC T LYMPHOCYTES ON A HUMAN MELANOMA" SCIENCE, no. 254, 14 December 1991, pages 1643-1647, XP002077831**
- **TRAVERSARI C ET AL: "A NONAPEPTIDE ENCODED BY HUMAN GENE MAGE-1 IS RECOGNIZED ON HLA-A1 BY CYTOLYTIC T LYMPHOCYTES DIRECTED AGAINST TUMOR ANTIGEN MZ2-E" JOURNAL OF EXPERIMENTAL MEDICINE, vol. 176, 1 November 1992, pages 1453-1457, XP000579148**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- PLAEN DE E ET AL: "STRUCTURE, CHROMOSOMAL LOCALIZATION, AND EXPRESSION OF 12 GENES OF THE MAGE FAMILY" IMMUNOGENETICS, vol. 40, no. 5, June 1994, pages 360-369, XP000614537
- GAUGLER B ET AL: "HUMAN GENE MAGE-3 CODES FOR AN ANTIGEN RECOGNIZED ON A MELANOMA BY AUTOLOGOUS CYTOLYTIC T LYMPHOCYTES" JOURNAL OF EXPERIMENTAL MEDICINE, vol. 179, no. 3, 1 March 1994, pages 921-930, XP002023774
- IMMUNOGENETICS, Volume 39, issued 1994, SMET et al., "Sequence and Expression Pattern of Human Mage2 Gene", pages 121-129.
- INTERNATIONAL JOURNAL OF CANCER, issued 1994, WEYNANTS et al., "Expression of Mage Genes by Non-Small-Cell Lung Carcinomas", pages 826-829.

(1) GENERAL INFORMATION:

(i) APPLICANTS: De Plaen, Etienne; Boon-Falleur, Thierry; Lethé, Bernard; Szikora, Jean-Pierre; De Smet, Charles; Chomez, Patrick; Weynants, P.; Brasseur, Francis; Marchand, M.; Gaugler, Béatrice; Van den Eynde, Benoit; van der Bruggen, Pierre; Patard, Jean-Jacques

(ii) TITLE OF INVENTION: Method For Determining A Cancerous Condition by Assaying For Expression Of One Or More Mage Tumor Rejection Antigen Precursors

(iii) NUMBER OF SEQUENCES: 56

(iv) CORRESPONDENCE ADDRESS:

(A) ADDRESSEE: Felfe & Lynch
(B) STREET: 805 Third Avenue
(C) CITY: New York City
(D) STATE: New York
(F) ZIP: 10022

(v) COMPUTER READABLE FORM:

(A) MEDIUM TYPE: Diskette, 5.25 inch, 360 kb storage
(B) COMPUTER: IBM
(C) OPERATING SYSTEM: PC-DOS
(D) SOFTWARE: Wordperfect

(vi) CURRENT APPLICATION DATA:

(A) APPLICATION NUMBER:
(B) FILING DATE:
(C) CLASSIFICATION:

(viii) ATTORNEY/AGENT INFORMATION:

(A) NAME: Hanson, Norman D.
(B) REGISTRATION NUMBER: 30,946
(C) REFERENCE/DOCKET NUMBER: LUD 5356-PCT

(ix) TELECOMMUNICATION INFORMATION:

(A) TELEPHONE: (212) 688-9200
(B) TELEFAX: (212) 838-3884

(2) INFORMATION FOR SEQUENCE ID NO: 1:

(i) SEQUENCE CHARACTERISTICS:

(A) LENGTH: 462 base pairs
(B) TYPE: nucleic acid
(D) TOPOLOGY: linear

(ii) MOLECULE TYPE: genomic DNA
(xi) SEQUENCE DESCRIPTION: SEQ ID NO: 1:

```
ACCACAGGAG AATGAAAAGA ACCCGGGACT CCCAAAGACG CTAGATGTGT         50
GAAGATCCTG ATCACTCATT GGGTGTCTGA GTTCTGCGAT ATTCATCCCT        100
CAGCCAATGA GCTTACTGTT CTCGTGGGGG GTTTGTGAGC CTTGGGTAGG        150
AAGTTTTGCA AGTTCCGCCT ACAGCTCTAG CTTGTGAATT TGTACCCTTT        200
CACGTAAAAA AGTAGTCCAG AGTTTACTAC ACCCTCCCTC CCCCCTCCCA        250
CCTCGTGCTG TGCTGAGTTT AGAAGTCTTC CTTATAGAAG TCTTCCGTAT        300
AGAACTCTTC CGGAGGAAGG AGGGAGGACC CCCCCCCTTT GCTCTCCCAG        350
CATGCATTGT GTCAACGCCA TTGCACTGAG CTGGTCGAAG AAGTAAGCCG        400
CTAGCTTGCG ACTCTACTCT TATCTTAACT TAGCTCGGCT TCCTGCTGGT        450
ACCCTTTGTG CC                                                 462
```

(2) INFORMATION FOR SEQUENCE ID NO: 2:

(i) SEQUENCE CHARACTERISTICS:

(A) LENGTH: 675 base pairs
(B) TYPE: nucleic acid
(D) TOPOLOGY: linear

(ii) MOLECULE TYPE: genomic DNA
(xi) SEQUENCE DESCRIPTION: SEQ ID NO: 2:

```
ATG TCT GAT AAC AAG AAA CCA GAC AAA GCC CAC AGT GGC TCA GGT GGT     48
Met Ser Asp Asn Lys Lys Pro Asp Lys Ala His Ser Gly Ser Gly Gly
                  5                  10                  15
GAC GGT GAT GGG AAT AGG TGC AAT TTA TTG CAC CGG TAC TCC CTG GAA     96
Asp Gly Asp Gly Asn Arg Cys Asn Leu Leu His Arg Tyr Ser Leu Glu
             20                  25                  30
GAA ATT CTG CCT TAT CTA GGG TGG CTG GTC TTC GCT GTT GTC ACA ACA    144
Glu Ile Leu Pro Tyr Leu Gly Trp Leu Val Phe Ala Val Val Thr Thr
         35                  40                  45
AGT TTT CTG GCG CTC CAG ATG TTC ATA GAC GCC CTT TAT GAG GAG CAG    192
Ser Phe Leu Ala Leu Gln Met Phe Ile Asp Ala Leu Tyr Glu Glu Gln
     50                  55                  60
TAT GAA AGG GAT GTG GCC TGG ATA GCC AGG CAA AGC AAG CGC ATG TCC    240
Tyr Glu Arg Asp Val Ala Trp Ile Ala Arg Gln Ser Lys Arg Met Ser
 65                  70                  75                  80
TCT GTC GAT GAG GAT GAA GAC GAT GAG GAT GAT GAG GAT GAC TAC TAC    288
Ser Val Asp Glu Asp Glu Asp Asp Glu Asp Asp Glu Asp Asp Tyr Tyr
                 85                  90                  95
GAC GAC GAG GAC GAC GAC GAC GAT GCC TTC TAT GAT GAT GAG GAT GAT    336
Asp Asp Glu Asp Asp Asp Asp Asp Ala Phe Tyr Asp Asp Glu Asp Asp
            100                 105                 110
GAG GAA GAA GAA TTG GAG AAC CTG ATG GAT GAT GAA TCA GAA GAT GAG    384
Glu Glu Glu Glu Leu Glu Asn Leu Met Asp Asp Glu Ser Glu Asp Glu
        115                 120                 125
GCC GAA GAA GAG ATG AGC GTG GAA ATG GGT GCC GGA GCT GAG GAA ATG    432
Ala Glu Glu Glu Met Ser Val Glu Met Gly Ala Gly Ala Glu Glu Met
    130                 135                 140
GGT GCT GGC GCT AAC TGT GCC TGT GTT CCT GGC CAT CAT TTA AGG AAG    480
Gly Ala Gly Ala Asn Cys Ala Cys Val Pro Gly His His Leu Arg Lys
145                 150                 155                 160
AAT GAA GTG AAG TGT AGG ATG ATT TAT TTC TTC CAC GAC CCT AAT TTC    528
Asn Glu Val Lys Cys Arg Met Ile Tyr Phe Phe His Asp Pro Asn Phe
                165                 170                 175
CTG GTG TCT ATA CCA GTG AAC CCT AAG GAA CAA ATG GAG TGT AGG TGT    576
Leu Val Ser Ile Pro Val Asn Pro Lys Glu Gln Met Glu Cys Arg Cys
            180                 185                 190
```

```
GAA AAT GCT GAT GAA GAG GTT GCA ATG GAA GAG GAA GAA GAA GAA GAG   624
Glu Asn Ala Asp Glu Glu Val Ala Met Glu Glu Glu Glu Glu Glu Glu
        195                 200                 210
GAG GAG GAG GAG GAA GAG GAA ATG GGA AAC CCG GAT GGC TTC TCA CCT   672
Glu Glu Glu Glu Glu Glu Glu Met Gly Asn Pro Asp Gly Phe Ser Pro
220                 225                 230                 235
TAG                                                               675
```

(2) INFORMATION FOR SEQUENCE ID NO: 3:

(i) SEQUENCE CHARACTERISTICS:

(A) LENGTH: 228 base pairs
(B) TYPE: nucleic acid
(D) TOPOLOGY: linear

(ii) MOLECULE TYPE: genomic DNA
(xi) SEQUENCE DESCRIPTION: SEQ ID NO: 3:

```
GCATGCAGTT GCAAAGCCCA GAAGAAAGAA ATGGACAGCG GAAGAAGTGG TTGTTTTTTT    60
TTCCCCTTCA TTAATTTTCT AGTTTTTAGT AATCCAGAAA ATTTGATTTT GTTCTAAAGT   120
TCATTATGCA AAGATGTCAC CAACAGACTT CTGACTGCAT GGTGAACTTT CATATGATAC   180
ATAGGATTAC ACTTGTACCT GTTAAAAATA AAAGTTTGAC TTGCATAC                228
```

(2) INFORMATION FOR SEQUENCE ID NO: 4:

(i) SEQUENCE CHARACTERISTICS:

(A) LENGTH: 1365 base pairs
(B) TYPE: nucleic acid
(D) TOPOLOGY: linear

(ii) MOLECULE TYPE: genomic DNA
(xi) SEQUENCE DESCRIPTION: SEQ ID NO: 4:

```
ACCACAGGAG AATGAAAAGA ACCCGGGACT CCCAAAGACG CTAGATGTGT      50
GAAGATCCTG ATCACTCATT GGGTGTCTGA GTTCTGCGAT ATTCATCCCT     100
CAGCCAATGA GCTTACTGTT CTCGTGGGGG GTTTGTGAGC CTTGGGTAGG     150
AAGTTTTGCA AGTTCCGCCT ACAGCTCTAG CTTGTGAATT TGTACCCTTT     200
CACGTAAAAA AGTAGTCCAG AGTTTACTAC ACCCTCCCTC CCCCCTCCCA     250
CCTCGTGCTG TGCTGAGTTT AGAAGTCTTC CTTATAGAAG TCTTCCGTAT     300
AGAACTCTTC CGGAGGAAGG AGGGAGGACC CCCCCCCTTT GCTCTCCCAG     350
CATGCATTGT GTCAACGCCA TTGCACTGAG CTGGTCGAAG AAGTAAGCCG     400
CTAGCTTGCG ACTCTACTCT TATCTTAACT TAGCTCGGCT TCCTGCTGGT     450
ACCCTTTGTG CC                                              462
ATG TCT GAT AAC AAG AAA CCA GAC AAA GCC CAC AGT GGC TCA    504
GGT GGT GAC GGT GAT GGG AAT AGG TGC AAT TTA TTG CAC CGG    546
TAC TCC CTG GAA GAA ATT CTG CCT TAT CTA GGG TGG CTG GTC    588
TTC GCT GTT GTC ACA ACA AGT TTT CTG GCG CTC CAG ATG TTC    630
ATA GAC GCC CTT TAT GAG GAG CAG TAT GAA AGG GAT GTG GCC    672
TGG ATA GCC AGG CAA AGC AAG CGC ATG TCC TCT GTC GAT GAG    714
GAT GAA GAC GAT GAG GAT GAT GAG GAT GAC TAC TAC GAC GAC    756
GAG GAC GAC GAC GAC GAT GCC TTC TAT GAT GAT GAG GAT GAT    798
GAG GAA GAA GAA TTG GAG AAC CTG ATG GAT GAT GAA TCA GAA    840
GAT GAG GCC GAA GAA GAG ATG AGC GTG GAA ATG GGT GCC GGA    882
GCT GAG GAA ATG GGT GCT GGC GCT AAC TGT GCC TGT GTT CCT    924
GGC CAT CAT TTA AGG AAG AAT GAA GTG AAG TGT AGG ATG ATT    966
TAT TTC TTC CAC GAC CCT AAT TTC CTG GTG TCT ATA CCA GTG   1008
AAC CCT AAG GAA CAA ATG GAG TGT AGG TGT GAA AAT GCT GAT   1050
GAA GAG GTT GCA ATG GAA GAG GAA GAA GAA GAA GAG GAG GAG   1092
GAG GAG GAA GAG GAA ATG GGA AAC CCG GAT GGC TTC TCA CCT   1134
TAG                                                       1137
GCATGCAGTT GCAAAGCCCA GAAGAAAGAA ATGGACAGCG GAAGAAGTGG    1187
TTGTTTTTTT TTCCCCTTCA TTAATTTTCT AGTTTTTAGT AATCCAGAAA    1237
ATTTGATTTT GTTCTAAAGT TCATTATGCA AAGATGTCAC CAACAGACTT    1287
CTGACTGCAT GGTGAACTTT CATATGATAC ATAGGATTAC ACTTGTACCT    1337
GTTAAAAATA AAAGTTTGAC TTGCATAC                            1365
```

(2) INFORMATION FOR SEQUENCE ID NO: 5:

(i) SEQUENCE CHARACTERISTICS:

(A) LENGTH: 4698 base pairs
(B) TYPE: nucleic acid
(D) TOPOLOGY: linear

(ii) MOLECULE TYPE: genomic DNA
(xi) SEQUENCE DESCRIPTION: SEQ ID NO: 5:

```
ACCACAGGAG AATGAAAAGA ACCCGGGACT CCCAAAGACG CTAGATGTGT     50
GAAGATCCTG ATCACTCATT GGGTGTCTGA GTTCTGCGAT ATTCATCCCT    100
CAGCCAATGA GCTTACTGTT CTCGTGGGGG GTTTGTGAGC CTTGGGTAGG    150
AAGTTTTGCA AGTTCCGCCT ACAGCTCTAG CTTGTGAATT TGTACCCTTT    200
CACGTAAAAA AGTAGTCCAG AGTTTACTAC ACCCTCCCTC CCCCCTCCCA    250
CCTCGTGCTG TGCTGAGTTT AGAAGTCTTC CTTATAGAAG TCTTCCGTAT    300
AGAACTCTTC CGGAGGAAGG AGGGAGGACC CCCCCCCTTT GCTCTCCCAG    350
CATGCATTGT GTCAACGCCA TTGCACTGAG CTGGTCGAAG AAGTAAGCCG    400
CTAGCTTGCG ACTCTACTCT TATCTTAACT TAGCTCGGCT TCCTGCTGGT    450
ACCCTTTGTG CC                                             462
ATG TCT GAT AAC AAG AAA CCA GAC AAA GCC CAC AGT GGC TCA   504
GGT GGT GAC GGT GAT GGG AAT AGG TGC AAT TTA TTG CAC CGG   546
TAC TCC CTG GAA GAA ATT CTG CCT TAT CTA GGG TGG CTG GTC   588
TTC GCT GTT GTC ACA ACA AGT TTT CTG GCG CTC CAG ATG TTC   630
ATA GAC GCC CTT TAT GAG GAG CAG TAT GAA AGG GAT GTG GCC   672
TGG ATA GCC AGG CAA AGC AAG CGC ATG TCC TCT GTC GAT GAG   714
GAT GAA GAC GAT GAG GAT GAT GAG GAT GAC TAC TAC GAC GAC   756
GAG GAC GAC GAC GAC GAT GCC TTC TAT GAT GAT GAG GAT GAT   798
GAG GAA GAA GAA TTG GAG AAC CTG ATG GAT GAT GAA TCA GAA   840
GAT GAG GCC GAA GAA GAG ATG AGC GTG GAA ATG GGT GCC GGA   882
GCT GAG GAA ATG GGT GCT GGC GCT AAC TGT GCC T             916
GTGAGTAACC CGTGGTCTTT ACTCTAGATT CAGGTGGGGT GCATTCTTTA    966
CTCTTGCCCA CATCTGTAGT AAAGACCACA TTTTGGTTGG GGGTCATTGC   1016
TGGAGCCATT CCTGGCTCTC CTGTCCACGC CTATCCCCGC TCCTCCCATC   1066
CCCCACTCCT TGCTCCGCTC TCTTTCCTTT TCCCACCTTG CCTCTGGAGC   1116
TTCAGTCCAT CCTGCTCTGC TCCCTTTCCC CTTTGCTCTC CTTGCTCCCC   1166
TCCCCCTCGG CTCAACTTTT CGTGCCTTCT GCTCTCTGAT CCCCACCCTC   1216
TTCAGGCTTC CCCATTTGCT CCTCTCCCGA AACCCTCCCC TTCCTGTTCC   1266
CCTTTTCGCG CCTTTTCTTT CCTGCTCCCC TCCCCCTCCC TATTTACCTT   1316
TCACCAGCTT TGCTCTCCCT GCTCCCCTCC CCCTTTTGCA CCTTTTCTTT   1366
TCCTGCTCCC CTCCCCCTCC CCTCCCTGTT TACCCTTCAC CGCTTTTCCT   1416
CTACCTGCTT CCCTCCCCCT TGCTGCTCCC TCCCTATTTG CATTTTCGGG   1466
TGCTCCTCCC TCCCCCTCCC CCTCCCTCCC TATTTGCATT TTCGGGTGCT   1516
CCTCCCTCCC CCTCCCCAGG CCTTTTTTTT TTTTTTTTTT TTTTTTTTTT   1566
TTGGTTTTTC GAGACAGGGT TTCTCTTTGT ATCCCTGGCT GTCCTGGCAC   1616
TCACTCTGTA GACCAGGCTG GCCTCAAACT CAGAAATCTG CCTGCCTCTG   1666
CCTCCCAAAT GCTGGGATTA AAGGCTTGCA CCAGGACTGC CCCAGTGCAG   1716
GCCTTTCTTT TTTCTCCTCT CTGGTCTCCC TAATCCCTTT TCTGCATGTT   1766
AACTCCCCTT TTGGCACCTT TCCTTTACAG GACCCCCTCC CCCTCCCTGT   1816
TTCCCTTCCG GCACCCTTCC TAGCCCTGCT CTGTTCCCTC TCCCTGCTCC   1866
CCTCCCCCTC TTTGCTCGAC TTTTAGCAGC CTTACCTCTC CCTGCTTTCT   1916
GCCCCGTTCC CCTTTTTTGT GCCTTTCCTC CTGGCTCCCC TCCACCTTCC   1966
AGCTCACCTT TTTGTTTGTT TGGTTGTTTG GTTGTTTGGT TTGCTTTTTT   2016
TTTTTTTTTT GCACCTTGTT TTCCAAGATC CCCCTCCCCC TCCGGCTTCC   2066
CCTCTGTGTG CCTTTCCTGT TCCCTCCCCC TCGCTGGCTC CCCCTCCCTT   2116
TCTGCCTTTC CTGTCCCTGC TCCCTTCTCT GCTAACCTTT TAATGCCTTT   2166
CTTTTCTAGA CTCCCCCCTC CAGGCTTGCT GTTTGCTTCT GTGCACTTTT   2216
CCTGACCCTG CTCCCCTTCC CCTCCCAGCT CCCCCCTCTT TTCCCACCTC   2266
CCTTTCTCCA GCCTGTCACC CCTCCTTCTC TCCTCTCTGT TTCTCCCACT   2316
TCCTGCTTCC TTTACCCCTT CCCTCTCCCT ACTCTCCTCC CTGCCTGCTG   2366
GACTTCCTCT CCAGCCGCCC AGTTCCCTGC AGTCCTGGAG TCTTTCCTGC   2416
CTCTCTGTCC ATCACTTCCC CCTAGTTTCA CTTCCCTTTC ACTCTCCCCT   2466
ATGTGTCTCT CTTCCTATCT ATCCCTTCCT TTCTGTCCCC TCTCCTCTGT   2516
CCATCACCTC TCTCCTCCCT TCCCTTTCCT CTCTCTTCCA TTTTCTTCCA   2566
CCTGCTTCTT TACCCTGCCT CTCCCATTGC CCTCTTACCT TTATGCCCAT   2616
TCCATGTCCC CTCTCAATTC CCTGTCCCAT TGTGCTCCCT CACATCTTCC   2666
ATTTCCCTCT TTCTCCCTTA GCCTCTTCTT CCTCTTCTCT TGTATCTCCC   2716
```

```
TTCCCTTTGC TTCTCCCTCC TCCTTTCCCC TTCCCCTATG CCCTCTACTC      2766
TACTTGATCT TCTCTCCTCT CCACATACCC TTTTTCCTTT CCACCCCTGCC     2816
CTTTGTCCCC AGACCCTACA GTATCCTGTG CACAGGAAGT GGGAGGTGCC      2866
ATCAACAACA AGGAGGCAAG AAACAGAGCA AAATCCCAAA ATCAGCAGGA      2916
AAGGCTGGAT GAAAATAAGG CCAGGTTCTG AGGACAGCTG GAATCTAGCC      2966
AAGTGGCTCC TATAACCCTA AGTACCAAGG GAGAAAGTGA TGGTGAAGTT      3016
CTTGATCCTT GCTGCTTCTT TTACATATGT TGGCACATCT TTCTCAAATG      3066
CAGGCCATGC TCCATGCTTG GCGCTTGCTC AGCGTGGTTA AGTAATGGGA      3116
GAATCTGAAA ACTAGGGGCC AGTGGTTTGT TTTGGGGACA AATTAGCACG      3166
TAGTGATATT TCCCCCTAAA AATTATAACA AACAGATTCA TGATTTGAGA      3216
TCCTTCTACA GGTGAGAAGT GGAAAAATTG TCACTATGAA GTTCTTTTTA      3266
GGCTAAAGAT ACTTGGAACC ATAGAAGCGT TGTTAAAATA CTGCTTTCTT      3316
TTGCTAAAAT ATTCTTTCTC ACATATTCAT ATTCTCCAG                  3355
 GT GTT CCT GGC CAT CAT TTA AGG AAG AAT GAA GTG AAG TGT     3396
AGG ATG ATT TAT TTC TTC CAC GAC CCT AAT TTC CTG GTG TCT     3438
ATA CCA GTG AAC CCT AAG GAA CAA ATG GAG TGT AGG TGT GAA     3480
AAT GCT GAT GAA GAG GTT GCA ATG GAA GAG GAA GAA GAA GAA     3522
GAG GAG GAG GAG GAG GAA GAG GAA ATG GGA AAC CCG GAT GGC     3564
TTC TCA CCT TAG                                             3576
GCATGCAGGT ACTGGCTTCA CTAACCAACC ATTCCTAACA TATGCCTGTA      3626
GCTAAGAGCA TCTTTTTAAA AAATATTATT GGTAAACTAA ACAATTGTTA      3676
TCTTTTTACA TTAATAAGTA TTAAATTAAT CCAGTATACA GTTTTAAGAA      3726
CCCTAAGTTA AACAGAAGTC AATGATGTCT AGATGCCTGT TCTTTAGATT      3776
GTAGTGAGAC TACTTACTAC AGATGAGAAG TTGTTAGACT CGGGAGTAGA      3826
GACCAGTAAA AGATCATGCA GTGAAATGTG GCCATGGAAA TCGCATATTG      3876
TTCTTATAGT ACCTTTGAGA CAGCTGATAA CAGCTGACAA AAATAAGTGT      3926
TTCAAGAAAG ATCACACGCC ATGGTTCACA TGCAAATTAT TATTTTGTCG      3976
TTCTGATTTT TTTCATTTCT AGACCTGTGG TTTTAAAGAG ATGAAAATCT      4026
CTTAAAATTT CCTTCATCTT TAATTTTCCT TAACTTTAGT TTTTTTCACT      4076
TAGAATTCAA TTCAAATTCT TAATTCAATC TTAATTTTTA GATTTCTTAA      4126
AATGTTTTTT AAAAAAAATG CAAATCTCAT TTTTAAGAGA TGAAAGCAGA      4176
GTAACTGGGG GGCTTAGGGA ATCTGTAGGG TTGCGGTATA GCAATAGGGA      4226
GTTCTGGTCT CTGAGAAGCA GTCAGAGAGA ATGGAAAACC AGGCCCTTGC      4276
CAGTAGGTTA GTGAGGTTGA TATGATCAGA TTATGGACAC TCTCCAAATC      4326
ATAAATACTC TAACAGCTAA GGATCTCTGA GGGAAACACA ACAGGGAAAT      4376
ATTTTAGTTT CTCCTTGAGA AACAATGACA AGACATAAAA TTGGCAAGAA      4426
AGTCAGGAGT GTATTCTAAT AAGTGTTGCT TATCTCTTAT TTTCTTCTAC      4476
AGTTGCAAAG CCCAGAAGAA AGAAATGGAC AGCGGAAGAA GTGGTTGTTT      4526
TTTTTTCCCC TTCATTAATT TTCTAGTTTT TAGTAATCCA GAAAATTTGA      4576
TTTTGTTCTA AAGTTCATTA TGCAAAGATG TCACCAACAG ACTTCTGACT      4626
GCATGGTGAA CTTTCATATG ATACATAGGA TTACACTTGT ACCTGTTAAA      4676
AATAAAAGTT TGACTTGCAT AC                                    4698
```

(2) INFORMATION FOR SEQUENCE ID NO: 6:

(i) SEQUENCE CHARACTERISTICS:

(A) LENGTH: 9 amino acids
(B) TYPE: amino acid
(D) TOPOLOGY: linear

(ii) MOLECULE TYPE: protein
(xi) SEQUENCE DESCRIPTION: SEQ ID NO: 6:

```
Leu Pro Tyr Leu Gly Trp Leu Val Phe
                    5
```

(2) INFORMATION FOR SEQUENCE ID NO: 7:

(i) SEQUENCE CHARACTERISTICS:

(A) LENGTH: 2418 base pairs
(B) TYPE: nucleic acid
(D) TOPOLOGY: linear

(ii) MOLECULE TYPE: genomic DNA
(xi) SEQUENCE DESCRIPTION: SEQ ID NO: 7:

```
GGATCCAGGC CCTGCCAGGA AAAATATAAG GGCCCTGCGT GAGAACAGAG      50
GGGGTCATCC ACTGCATGAG AGTGGGGATG TCACAGAGTC CAGCCCACCC     100
```

```
TCCTGGTAGC ACTGAGAAGC CAGGGCTGTG CTTGCGGTCT GCACCCTGAG    150
GGCCCGTGGA TTCCTCTTCC TGGAGCTCCA GGAACCAGGC AGTGAGGCCT    200
TGGTCTGAGA CAGTATCCTC AGGTCACAGA GCAGAGGATG CACAGGGTGT    250
GCCAGCAGTG AATGTTTGCC CTGAATGCAC ACCAAGGGCC CCACCTGCCA    300
CAGGACACAT AGGACTCCAC AGAGTCTGGC CTCACCTCCC TACTGTCAGT    350
CCTGTAGAAT CGACCTCTGC TGGCCGGCTG TACCCTGAGT ACCCTCTCAC    400
TTCCTCCTTC AGGTTTTCAG GGGACAGGCC AACCCAGAGG ACAGGATTCC    450
CTGGAGGCCA CAGAGGAGCA CCAAGGAGAA GATCTGTAAG TAGGCCTTTG    500
TTAGAGTCTC CAAGGTTCAG TTCTCAGCTG AGGCCTCTCA CACACTCCCT    550
CTCTCCCCAG GCCTGTGGGT CTTCATTGCC CAGCTCCTGC CCACACTCCT    600
GCCTGCTGCC CTGACGAGAG TCATCATGTC TCTTGAGCAG AGGAGTCTGC    650
ACTGCAAGCC TGAGGAAGCC CTTGAGGCCC AACAAGAGGC CCTGGGCCTG    700
GTGTGTGTGC AGGCTGCCAC CTCCTCCTCC TCTCCTCTGG TCCTGGGCAC    750
CCTGGAGGAG GTGCCCACTG CTGGGTCAAC AGATCCTCCC CAGAGTCCTC    800
AGGGAGCCTC CGCCTTTCCC ACTACCATCA ACTTCACTCG ACAGAGGCAA    850
CCCAGTGAGG GTTCCAGCAG CCGTGAAGAG GAGGGGCCAA GCACCTCTTG    900
TATCCTGGAG TCCTTGTTCC GAGCAGTAAT CACTAAGAAG GTGGCTGATT    950
TGGTTGGTTT TCTGCTCCTC AAATATCGAG CCAGGGAGCC AGTCACAAAG   1000
GCAGAAATGC TGGAGAGTGT CATCAAAAAT TACAAGCACT GTTTTCCTGA   1050
GATCTTCGGC AAAGCCTCTG AGTCCTTGCA GCTGGTCTTT GGCATTGACG   1100
TGAAGGAAGC AGACCCCACC GGCCACTCCT ATGTCCTTGT CACCTGCCTA   1150
GGTCTCTCCT ATGATGGCCT GCTGGGTGAT AATCAGATCA TGCCCAAGAC   1200
AGGCTTCCTG ATAATTGTCC TGGTCATGAT TGCAATGGAG GGCGGCCATG   1250
CTCCTGAGGA GGAAATCTGG GAGGAGCTGA GTGTGATGGA GGTGTATGAT   1300
GGGAGGGAGC ACAGTGCCTA TGGGGAGCCC AGGAAGCTGC TCACCCAAGA   1350
TTTGGTGCAG GAAAAGTACC TGGAGTACGG CAGGTGCCGG ACAGTGATCC   1400
CGCACGCTAT GAGTTCCTGT GGGGTCCAAG GGCCCTCGCT GAAACCAGCT   1450
ATGTGAAAGT CCTTGAGTAT GTGATCAAGG TCAGTGCAAG AGTTCGCTTT   1500
TTCTTCCCAT CCCTGCGTGA AGCAGCTTTG AGAGAGGAGG AAGAGGGAGT   1550
CTGAGCATGA GTTGCAGCCA AGGCCAGTGG GAGGGGGACT GGGCCAGTGC   1600
ACCTTCCAGG GCCGCGTCCA GCAGCTTCCC CTGCCTCGTG TGACATGAGG   1650
CCCATTCTTC ACTCTGAAGA GAGCGGTCAG TGTTCTCAGT AGTAGGTTTC   1700
TGTTCTATTG GGTGACTTGG AGATTTATCT TTGTTCTCTT TTGGAATTGT   1750
TCAAATGTTT TTTTTTAAGG GATGGTTGAA TGAACTTCAG CATCCAAGTT   1800
TATGAATGAC AGCAGTCACA CAGTTCTGTG TATATAGTTT AAGGGTAAGA   1850
GTCTTGTGTT TTATTCAGAT TGGGAAATCC ATTCTATTTT GTGAATTGGG   1900
ATAATAACAG CAGTGGAATA AGTACTTAGA AATGTGAAAA ATGAGCAGTA   1950
AAATAGATGA GATAAAGAAC TAAAGAAATT AAGAGATAGT CAATTCTTGC   2000
CTTATACCTC AGTCTATTCT GTAAAATTTT TAAAGATATA TGCATACCTG   2050
GATTTCCTTG GCTTCTTTGA GAATGTAAGA GAAATTAAAT CTGAATAAAG   2100
AATTCTTCCT GTTCACTGGC TCTTTTCTTC TCCATGCACT GAGCATCTGC   2150
TTTTTGGAAG GCCCTGGGTT AGTAGTGGAG ATGCTAAGGT AAGCCAGACT   2200
CATACCCACC CATAGGGTCG TAGAGTCTAG GAGCTGCAGT CACGTAATCG   2250
AGGTGGCAAG ATGTCCTCTA AAGATGTAGG GAAAAGTGAG AGAGGGGTGA   2300
GGGTGTGGGG CTCCGGGTGA GAGTGGTGGA GTGTCAATGC CCTGAGCTGG   2350
GGCATTTTGG GCTTTGGGAA ACTGCAGTTC CTTCTGGGGG AGCTGATTGT   2400
AATGATCTTG GGTGGATCC                                      2418
```

(2) INFORMATION FOR SEQUENCE ID NO: 8:

(i) SEQUENCE CHARACTERISTICS:

(A) LENGTH: 5724 base pairs
(B) TYPE: nucleic acid
(D) TOPOLOGY: linear

(ii) MOLECULE TYPE: genomic DNA
(ix) FEATURE:

(A) NAME/KEY: MAGE-1 gene

(xi) SEQUENCE DESCRIPTION: SEQ ID NO: 8:

```
CCCGGGGCAC CACTGGCATC CCTCCCCCTA CCACCCCCAA TCCCTCCCTT       50
TACGCCACCC ATCCAAACAT CTTCACGCTC ACCCCCAGCC CAAGCCAGGC      100
AGAATCCGGT TCCACCCCTG CTCTCAACCC AGGGAAGCCC AGGTGCCCAG      150
ATGTGACGCC ACTGACTTGA GCATTAGTGG TTAGAGAGAA GCGAGGTTTT      200
CGGTCTGAGG GGCGGCTTGA GATCGGTGGA GGGAAGCGGG CCCAGCTCTG      250
```

TAAGGAGGCA AGGTGACATG CTGAGGGAGG ACTGAGGACC CACTTACCCC 300
AGATAGAGGA CCCCAAATAA TCCCTTCATG CCAGTCCTGG ACCATCTGGT 350
GGTGGACTTC TCAGGCTGGG CCACCCCCAG CCCCCTTGCT GCTTAAACCA 400
CTGGGGACTC GAAGTCAGAG CTCCGTGTGA TCAGGGAAGG GCTGCTTAGG 450
AGAGGGCAGC GTCCAGGCTC TGCCAGACAT CATGCTCAGG ATTCTCAAGG 500
AGGGCTGAGG GTCCCTAAGA CCCCACTCCC GTGACCCAAC CCCCACTCCA 550
ATGCTCACTC CCGTGACCCA ACCCCCTCTT CATTGTCATT CCAACCCCCA 600
CCCCACATCC CCCACCCCAT CCCTCAACCC TGATGCCCAT CCGCCCAGCC 650
ATTCCACCCT CACCCCCACC CCCACCCCCA CGCCCACTCC CACCCCCACC 700
CAGGCAGGAT CCGGTTCCCG CCAGGAAACA TCCGGGTGCC CGGATGTGAC 750
GCCACTGACT TGCGCATTGT GGGGCAGAGA GAAGCGAGGT TTCCATTCTG 800
AGGGACGGCG TAGAGTTCGG CCGAAGGAAC CTGACCCAGG CTCTGTGAGG 850
AGGCAAGGTG AGAGGCTGAG GGAGGACTGA GGACCCCGCC ACTCCAAATA 900
GAGAGCCCCA AATATTCCAG CCCCGCCCTT GCTGCCAGCC CTGGCCCACC 950
CGCGGGAAGA CGTCTCAGCC TGGGCTGCCC CCAGACCCCT GCTCCAAAAG 1000
CCTTGAGAGA CACCAGGTTC TTCTCCCCAA GCTCTGGAAT CAGAGGTTGC 1050
TGTGACCAGG GCAGGACTGG TTAGGAGAGG GCAGGGCACA GGCTCTGCCA 1100
GGCATCAAGA TCAGCACCCA AGAGGGAGGG CTGTGGGCCC CCAAGACTGC 1150
ACTCCAATCC CCACTCCCAC CCCATTCGCA TTCCCATTCC CCACCCAACC 1200
CCCATCTCCT CAGCTACACC TCCACCCCCA TCCCTACTCC TACTCCGTCA 1250
CCTGACCACC ACCCTCCAGC CCCAGCACCA GCCCCAACCC TTCTGCCACC 1300
TCACCCTCAC TGCCCCCAAC CCCACCCTCA TCTCTCTCAT GTGCCCCACT 1350
CCCATCGCCT CCCCCATTCT GGCAGAATCC GGTTTGCCCC TGCTCTCAAC 1400
CCAGGGAAGC CCTGGTAGGC CCGATGTGAA ACCACTGACT TGAACCTCAC 1450
AGATCTGAGA GAAGCCAGGT TCATTTAATG GTTCTGAGGG GCGGCTTGAG 1500
ATCCACTGAG GGGAGTGGTT TTAGGCTCTG TGAGGAGGCA AGGTGAGATG 1550
CTGAGGGAGG ACTGAGGAGG CACACACCCC AGGTAGATGG CCCCAAAATG 1600
ATCCAGTACC ACCCCTGCTG CCAGCCCTGG ACCACCCGGC CAGGACAGAT 1650
GTCTCAGCTG GACCACCCCC CGTCCCGTCC CACTGCCACT TAACCCACAG 1700
GGCAATCTGT AGTCATAGCT TATGTGACCG GGGCAGGGTT GGTCAGGAGA 1750
GGCAGGGCCC AGGCATCAAG GTCCAGCATC CGCCCGGCAT TAGGGTCAGG 1800
ACCCTGGGAG GGAACTGAGG GTTCCCCACC CACACCTGTC TCCTCATCTC 1850
CACCGCCACC CCACTCACAT TCCCATACCT ACCCCCTACC CCCAACCTCA 1900
TCTTGTCAGA ATCCCTGCTG TCAACCCACG GAAGCCACGG GAATGGCGGC 1950
CAGGCACTCG GATCTTGACG TCCCCATCCA GGGTCTGATG GAGGGAAGGG 2000
GCTTGAACAG GGCCTCAGGG GAGCAGAGGG AGGGCCCTAC TGCGAGATGA 2050
GGGAGGCCTC AGAGGACCCA GCACCCTAGG ACACCGCACC CCTGTCTGAG 2100
ACTGAGGCTG CCACTTCTGG CCTCAAGAAT CAGAACGATG GGGACTCAGA 2150
TTGCATGGGG GTGGGACCCA GGCCTGCAAG GCTTACGCGG AGGAAGAGGA 2200
GGGAGGACTC AGGGGACCTT GGAATCCAGA TCAGTGTGGA CCTCGGCCCT 2250
GAGAGGTCCA GGGCACGGTG GCCACATATG GCCCATATTT CCTGCATCTT 2300
TGAGGTGACA GGACAGAGCT GTGGTCTGAG AAGTGGGGCC TCAGGTCAAC 2350
AGAGGGAGGA GTTCCAGGAT CCATATGGCC CAAGATGTGC CCCCTTCATG 2400
AGGACTGGGG ATATCCCCGG CTCAGAAAGA AGGGACTCCA CACAGTCTGG 2450
CTGTCCCCTT TTAGTAGCTC TAGGGGGACC AGATCAGGGA TGGCGGTATG 2500
TTCCATTCTC ACTTGTACCA CAGGCAGGAA GTTGGGGGGC CCTCAGGGAG 2550
ATGGGGTCTT GGGGTAAAGG GGGGATGTCT ACTCATGTCA GGGAATTGGG 2600
GGTTGAGGAA GCACAGGCGC TGGCAGGAAT AAAGATGAGT GAGACAGACA 2650
AGGCTATTGG AATCCACACC CCAGAACCAA AGGGGTCAGC CCTGGACACC 2700
TCACCCAGGA TGTGGCTTCT TTTTCACTCC TGTTTCCAGA TCTGGGGCAG 2750
GTGAGGACCT CATTCTCAGA GGGTGACTCA GGTCAACGTA GGGACCCCCA 2800
TCTGGTCTAA AGACAGAGCG GTCCCAGGAT CTGCCATGCG TTCGGGTGAG 2850
GAACATGAGG GAGGACTGAG GGTACCCCAG GACCAGAACA CTGAGGGAGA 2900
CTGCACAGAA ATCAGCCCTG CCCCTGCTGT CACCCCAGAG AGCATGGGCT 2950
GGGCCGTCTG CCGAGGTCCT TCCGTTATCC TGGGATCATT GATGTCAGGG 3000
ACGGGGAGGC CTTGGTCTGA GAAGGCTGCG CTCAGGTCAG TAGAGGGAGC 3050
GTCCCAGGCC CTGCCAGGAG TCAAGGTGAG GACCAAGCGG GCACCTCACC 3150
CAGGACACAT TAATTCCAAT GAATTTTGAT ATCTCTTGCT GCCCTTCCCC 3200
AAGGACCTAG GCACGTGTGG CCAGATGTTT GTCCCCTCCT GTCCTTCCAT 3250
TCCTTATCAT GGATGTGAAC TCTTGATTTG GATTTCTCAG ACCAGCAAAA 3300
GGGCAGGATC CAGGCCCTGC CAGGAAAAAT ATAAGGGCCC TGCGTGAGAA 3350
CAGAGGGGGT CATCCACTGC ATGAGAGTGG GGATGTCACA GAGTCCAGCC 3400
CACCCTCCTG GTAGCACTGA GAAGCCAGGG CTGTGCTTGC GGTCTGCACC 3450
CTGAGGGCCC GTGGATTCCT CTTCCTGGAG CTCCAGGAAC CAGGCAGTGA 3500
GGCCTTGGTC TGAGACAGTA TCCTCAGGTC ACAGAGCAGA GGATGCACAG 3550
GGTGTGCCAG CAGTGAATGT TTGCCCTGAA TGCACACCAA GGGCCCCACC 3600

```
TGCCACAGGA CACATAGGAC TCCACAGAGT CTGGCCTCAC CTCCCTACTG        3650
TCAGTCCTGT AGAATCGACC TCTGCTGGCC GGCTGTACCC TGAGTACCCT        3700
CTCACTTCCT CCTTCAGGTT TTCAGGGGAC AGGCCAACCC AGAGGACAGG        3750
ATTCCCTGGA GGCCACAGAG GAGCACCAAG GAGAAGATCT GTAAGTAGGC        3800
CTTTGTTAGA GTCTCCAAGG TTCAGTTCTC AGCTGAGGCC TCTCACACAC        3850
TCCCTCTCTC CCCAGGCCTG TGGGTCTTCA TTGCCCAGCT CCTGCCCACA        3900
CTCCTGCCTG CTGCCCTGAC GAGAGTCATC                              3930
ATG TCT CTT GAG CAG AGG AGT CTG CAC TGC AAG CCT GAG GAA       3972
GCC CTT GAG GCC CAA CAA GAG GCC CTG GGC CTG GTG TGT GTG       4014
CAG GCT GCC ACC TCC TCC TCC TCT CCT CTG GTC CTG GGC ACC       4056
CTG GAG GAG GTG CCC ACT GCT GGG TCA ACA GAT CCT CCC CAG       4098
AGT CCT CAG GGA GCC TCC GCC TTT CCC ACT ACC ATC AAC TTC       4140
ACT CGA CAG AGG CAA CCC AGT GAG GGT TCC AGC AGC CGT GAA       4182
GAG GAG GGG CCA AGC ACC TCT TGT ATC CTG GAG TCC TTG TTC       4224
CGA GCA GTA ATC ACT AAG AAG GTG GCT GAT TTG GTT GGT TTT       4266
CTG CTC CTC AAA TAT CGA GCC AGG GAG CCA GTC ACA AAG GCA       4308
GAA ATG CTG GAG AGT GTC ATC AAA AAT TAC AAG CAC TGT TTT       4350
CCT GAG ATC TTC GGC AAA GCC TCT GAG TCC TTG CAG CTG GTC       4392
TTT GGC ATT GAC GTG AAG GAA GCA GAC CCC ACC GGC CAC TCC       4434
TAT GTC CTT GTC ACC TGC CTA GGT CTC TCC TAT GAT GGC CTG       4476
CTG GGT GAT AAT CAG ATC ATG CCC AAG ACA GGC TTC CTG ATA       4518
ATT GTC CTG GTC ATG ATT GCA ATG GAG GGC GGC CAT GCT CCT       4560
GAG GAG GAA ATC TGG GAG GAG CTG AGT GTG ATG GAG GTG TAT       4602
GAT GGG AGG GAG CAC AGT GCC TAT GGG GAG CCC AGG AAG CTG       4644
CTC ACC CAA GAT TTG GTG CAG GAA AAG TAC CTG GAG TAC GGC       4686
AGG TGC CGG ACA GTG ATC CCG CAC GCT ATG AGT TCC TGT GGG       4728
GTC CAA GGG CCC TCG CTG AAA CCA GCT ATG TGA                   4761
AAGTCCTTGA GTATGTGATC AAGGTCAGTG CAAGAGTTC                    4800
GCTTTTTCTT CCCATCCCTG CGTGAAGCAG CTTTGAGAGA GGAGGAAGAG        4850
GGAGTCTGAG CATGAGTTGC AGCCAAGGCC AGTGGGAGGG GGACTGGGCC        4900
AGTGCACCTT CCAGGGCCGC GTCCAGCAGC TTCCCCTGCC TCGTGTGACA        4950
TGAGGCCCAT TCTTCACTCT GAAGAGAGCG GTCAGTGTTC TCAGTAGTAG        5000
GTTTCTGTTC TATTGGGTGA CTTGGAGATT TATCTTTGTT CTCTTTTGGA        5050
ATTGTTCAAA TGTTTTTTTT TAAGGGATGG TTGAATGAAC TTCAGCATCC        5100
AAGTTTATGA ATGACAGCAG TCACACAGTT CTGTGTATAT AGTTTAAGGG        5150
TAAGAGTCTT GTGTTTTATT CAGATTGGGA AATCCATTCT ATTTTGTGAA        5200
TTGGGATAAT AACAGCAGTG GAATAAGTAC TTAGAAATGT GAAAAATGAG        5250
CAGTAAAATA GATGAGATAA AGAACTAAAG AAATTAAGAG ATAGTCAATT        5300
CTTGCCTTAT ACCTCAGTCT ATTCTGTAAA ATTTTTAAAG ATATATGCAT        5350
ACCTGGATTT CCTTGGCTTC TTTGAGAATG TAAGAGAAAT TAAATCTGAA        5400
TAAAGAATTC TTCCTGTTCA CTGGCTCTTT TCTTCTCCAT GCACTGAGCA        5450
TCTGCTTTTT GGAAGGCCCT GGGTTAGTAG TGGAGATGCT AAGGTAAGCC        5500
AGACTCATAC CCACCCATAG GGTCGTAGAG TCTAGGAGCT GCAGTCACGT        5550
AATCGAGGTG GCAAGATGTC CTCTAAAGAT GTAGGGAAAA GTGAGAGAGG        5600
GGTGAGGGTG TGGGGCTCCG GGTGAGAGTG GTGGAGTGTC AATGCCCTGA        5650
GCTGGGGCAT TTTGGGCTTT GGGAAACTGC AGTTCCTTCT GGGGGAGCTG        5700
ATTGTAATGA TCTTGGGTGG ATCC                                    5724
```

(2) INFORMATION FOR SEQUENCE ID NO: 9:

(i) SEQUENCE CHARACTERISTICS:

(A) LENGTH: 4157 base pairs
(B) TYPE: nucleic acid
(D) TOPOLOGY: linear

(ii) MOLECULE TYPE: genomic DNA
(ix) FEATURE:

(A) NAME/KEY: MAGE-2 gene

(xi) SEQUENCE DESCRIPTION: SEQ ID NO: 9:

```
CCCATCCAGA TCCCCATCCG GGCAGAATCC GGTTCCACCC TTGCCGTGAA     50
CCCAGGGAAG TCACGGGCCC GGATGTGACG CCACTGACTT GCACATTGGA    100
GGTCAGAGGA CAGCGAGATT CTCGCCCTGA GCAACGGCCT GACGTCGGCG    150
GAGGGAAGCA GGCGCAGGCT CCGTGAGGAG GCAAGGTAAG ACGCCGAGGG    200
AGGACTGAGG CGGGCCTCAC CCCAGACAGA GGGCCCCCAA TTAATCCAGC    250
```

GCTGCCTCTG CTGCCGGGCC TGGACCACCC TGCAGGGGAA GACTTCTCAG 300
GCTCAGTCGC CACCACCTCA CCCCGCCACC CCCCGCCGCT TTAACCGCAG 350
GGAACTCTGG CGTAAGAGCT TTGTGTGACC AGGGCAGGGC TGGTTAGAAG 400
TGCTCAGGGC CCAGACTCAG CCAGGAATCA AGGTCAGGAC CCCAAGAGGG 450
GACTGAGGGC AACCCACCCC CTACCCTCAC TACCAATCCC ATCCCCCAAC 500
ACCAACCCCA CCCCCATCCC TCAAACACCA ACCCCACCCC CAAACCCCAT 550
TCCCATCTCC TCCCCCACCA CCATCCTGGC AGAATCCGGC TTTGCCCCTG 600
CAATCAACCC ACGGAAGCTC CGGGAATGGC GGCCAAGCAC GCGGATCCTG 650
ACGTTCACAT GTACGGCTAA GGGAGGGAAG GGGTTGGGTC TCGTGAGTAT 700
GGCCTTTGGG ATGCAGAGGA AGGGCCCAGG CCTCCTGGAA GACAGTGGAG 750
TCCTTAGGGG ACCCAGCATG CCAGGACAGG GGGCCCACTG TACCCCTGTC 800
TCAAACTGAG CCACCTTTTC ATTCAGCCGA GGGAATCCTA GGGATGCAGA 850
CCCACTTCAG GGGGTTGGGG CCCAGCCTGC GAGGAGTCAA GGGGAGGAAG 900
AAGAGGGAGG ACTGAGGGGA CCTTGGAGTC CAGATCAGTG GCAACCTTGG 950
GCTGGGGGAT CCTGGGCACA GTGGCCGAAT GTGCCCCGTG CTCATTGCAC 1000
CTTCAGGGTG ACAGAGAGTT GAGGGCTGTG GTCTGAGGGC TGGGACTTCA 1050
GGTCAGCAGA GGGAGGAATC CCAGGATCTG CCGGACCCAA GGTGTGCCCC 1100
CTTCATGAGG ACTCCCCATA CCCCCGGCCC AGAAAGAAGG GATGCCACAG 1150
AGTCTGGAAG TAAATTGTTC TTAGCTCTGG GGGAACCTGA TCAGGGATGG 1200
CCCTAAGTGA CAATCTCATT TGTACCACAG GCAGGAGGTT GGGGAACCCT 1250
CAGGGAGATA AGGTGTTGGT GTAAAGAGGA GCTGTCTGCT CATTTCAGGG 1300
GGTTCCCCCT TGAGAAAGGG CAGTCCCTGG CAGGAGTAAA GATGAGTAAC 1350
CCACAGGAGG CCATCATAAC GTTCACCCTA GAACCAAAGG GGTCAGCCCT 1400
GGACAACGCA CGTGGGGTAA CAGGATGTGG CCCCTCCTCA CTTGTCTTTC 1450
CAGATCTCAG GGAGTTGATG ACCTTGTTTT CAGAAGGTGA CTCAGTCAAC 1500
ACAGGGGCCC CTCTGGTCGA CAGATGCAGT GGTTCTAGGA TCTGCCAAGC 1550
ATCCAGGTGG AGAGCCTGAG GTAGGATTGA GGGTACCCCT GGGCCAGAAT 1600
GCAGCAAGGG GGCCCCATAG AAATCTGCCC TGCCCCTGCG GTTACTTCAG 1650
AGACCCTGGG CAGGGCTGTC AGCTGAAGTC CCTCCATTAT CTGGGATCTT 1700
TGATGTCAGG GAAGGGGAGG CCTTGGTCTG AAGGGGCTGG AGTCAGGTCA 1750
GTAGAGGGAG GGTCTCAGGC CCTGCCAGGA GTGGACGTGA GGACCAAGCG 1800
GACTCGTCAC CCAGGACACC TGGACTCCAA TGAATTTGAC ATCTCTCGTT 1850
GTCCTTCGCG GAGGACCTGG TCACGTATGG CCAGATGTGG GTCCCCTCTA 1900
TCTCCTTCTG TACCATATCA GGGATGTGAG TTCTTGACAT GAGAGATTCT 1950
CAAGCCAGCA AAAGGGTGGG ATTAGGCCCT ACAAGGAGAA AGGTGAGGGC 2000
CCTGAGTGAG CACAGAGGGG ACCCTCCACC CAAGTAGAGT GGGGACCTCA 2050
CGGAGTCTGG CCAACCCTGC TGAGACTTCT GGGAATCCGT GGCTGTGCTT 2100
GCAGTCTGCA CACTGAAGGC CCGTGCATTC CTCTCCCAGG AATCAGGAGC 2150
TCCAGGAACC AGGCAGTGAG GCCTTGGTCT GAGTCAGTGC CTCAGGTCAC 2200
AGAGCAGAGG GGACGCAGAC AGTGCCAACA CTGAAGGTTT GCCTGGAATG 2250
CACACCAAGG GCCCCACCCG CCCAGAACAA ATGGGACTCC AGAGGGCCTG 2300
GCCTCACCCT CCCTATTCTC AGTCCTGCAG CCTGAGCATG TGCTGGCCGG 2350
CTGTACCCTG AGGTGCCCTC CCACTTCCTC CTTCAGGTTC TGAGGGGGAC 2400
AGGCTGACAA GTAGGACCCG AGGCACTGGA GGAGCATTGA AGGAGAAGAT 2450
CTGTAAGTAA GCCTTTGTCA GAGCCTCCAA GGTTCAGTTC AGTTCTCACC 2500
TAAGGCCTCA CACACGCTCC TTCTCTCCCC AGGCCTGTGG GTCTTCATTG 2550
CCCAGCTCCT GCCCGCACTC CTGCCTGCTG CCCTGACCAG AGTCATC 2597
ATG CCT CTT GAG CAG AGG AGT CAG CAC TGC AAG CCT GAA GAA 2639
GGC CTT GAG GCC CGA GGA GAG GCC CTG GGC CTG GTG GGT GCG 2681
CAG GCT CCT GCT ACT GAG GAG CAG CAG ACC GCT TCT TCC TCT 2723
TCT ACT CTA GTG GAA GTT ACC CTG GGG GAG GTG CCT GCT GCC 2765
GAC TCA CCG AGT CCT CCC CAC AGT CCT CAG GGA GCC TCC AGC 2807
TTC TCG ACT ACC ATC AAC TAC ACT CTT TGG AGA CAA TCC GAT 2849
GAG GGC TCC AGC AAC CAA GAA GAG GAG GGG CCA AGA ATG TTT 2891
CCC GAC CTG GAG TCC GAG TTC CAA GCA GCA ATC AGT AGG AAG 2933
ATG GTT GAG TTG GTT CAT TTT CTG CTC CTC AAG TAT CGA GCC 2975
AGG GAG CCG GTC ACA AAG GCA GAA ATG CTG GAG AGT GTC CTC 3017
AGA AAT TGC CAG GAC TTC TTT CCC GTG ATC TTC AGC AAA GCC 3059
TCC GAG TAC TTG CAG CTG GTC TTT GGC ATC GAG GTG GTG GAA 3101
GTG GTC CCC ATC AGC CAC TTG TAC ATC CTT GTC ACC TGC CTG 3143
GGC CTC TCC TAC GAT GGC CTG CTG GGC GAC AAT CAG GTC ATG 3185
CCC AAG ACA GGC CTC CTG ATA ATC GTC CTG GCC ATA ATC GCA 3227
ATA GAG GGC GAC TGT GCC CCT GAG GAG AAA ATC TGG GAG GAG 3269
CTG AGT ATG TTG GAG GTG TTT GAG GGG AGG GAG GAC AGT GTC 3311
TTC GCA CAT CCC AGG AAG CTG CTC ATG CAA GAT CTG GTG CAG 3353
GAA AAC TAC CTG GAG TAC CGG CAG GTG CCC GGC AGT GAT CCT 3395

GCA TGC TAC GAG TTC CTG TGG GGT CCA AGG GCC CTC ATT GAA 3437
ACC AGC TAT GTG AAA GTC CTG CAC CAT ACA CTA AAG ATC GGT 3479
GGA GAA CCT CAC ATT TCC TAC CCA CCC CTG CAT GAA CGG GCT 3521
TTG AGA GAG GGA GAA GAG TGA 3542
GTCTCAGCAC ATGTTGCAGC CAGGGCCAGT GGGAGGGGGT CTGGGCCAGT 3592
GCACCTTCCA GGGCCCCATC CATTAGCTTC CACTGCCTCG TGTGATATGA 3642
GGCCCATTCC TGCCTCTTTG AAGAGAGCAG TCAGCATTCT TAGCAGTGAG 3692
TTTCTGTTCT GTTGGATGAC TTTGAGATTT ATCTTTCTTT CCTGTTGGAA 3742
TTGTTCAAAT GTTCCTTTTA ACAAATGGTT GGATGAACTT CAGCATCCAA 3792
GTTTATGAAT GACAGTAGTC ACACATAGTG CTGTTTATAT AGTTTAGGGG 3842
TAAGAGTCCT GTTTTTTATT CAGATTGGGA AATCCATTCC ATTTTGTGAG 3892
TTGTCACATA ATAACAGCAG TGGAATATGT ATTTGCCTAT ATTGTGAACG 3942
AATTAGCAGT AAAATACATG ATACAAGGAA CTCAAAAGAT AGTTAATTCT 3992
TGCCTTATAC CTCAGTCTAT TATGTAAAAT TAAAAATATG TGTATGTTTT 4042
TGCTTCTTTG AGAATGCAAA AGAAATTAAA TCTGAATAAA TTCTTCCTGT 4092
TCACTGGCTC ATTTCTTTAC CATTCACTCA GCATCTGCTC TGTGGAAGGC 4142
CCTGGTAGTA GTGGG 4157

(2) INFORMATION FOR SEQUENCE ID NO: 10:

(i) SEQUENCE CHARACTERISTICS:

(A) LENGTH: 662 base pairs
(B) TYPE: nucleic acid
(D) TOPOLOGY: linear

(ii) MOLECULE TYPE: genomic DNA
(ix) FEATURE:

(A) NAME/KEY: MAGE-21 gene

(xi) SEQUENCE DESCRIPTION: SEQ ID NO: 10:

GGATCCCCAT GGATCCAGGA AGAATCCAGT TCCACCCCTG CTGTGAACCC 50
AGGGAAGTCA CGGGGCCGGA TGTGACGCCA CTGACTTGCG CGTTGGAGGT 100
CAGAGAACAG CGAGATTCTC GCCCTGAGCA ACGGCCTGAC GTCGGCGGAG 150
GGAAGCAGGC GCAGGCTCCG TGAGGAGGCA AGGTAAGATG CCGAGGGAGG 200
ACTGAGGCGG GCCTCACCCC AGACAGAGGG CCCCCAATAA TCCAGCGCTG 250
CCTCTGCTGC CAGGCCTGGA CCACCCTGCA GGGGAAGACT TCTCAGGCTC 300
AGTCGCCACC ACCTCACCCC GCCACCCCCC GCCGCTTTAA CCGCAGGGAA 350
CTCTGGTGTA AGAGCTTTGT GTGACCAGGG CAGGGCTGGT TAGAAGTGCT 400
CAGGGCCCAG ACTCAGCCAG GAATCAAGGT CAGGACCCCA AGAGGGGACT 450
GAGGGTAACC CCCCCGCACC CCCACCACCA TTCCCATCCC CCAACACCAA 500
CCCCACCCCC ATCCCCCAAC ACCAAACCCA CCACCATCGC TCAAACATCA 550
ACGGCACCCC CAAACCCCGA TTCCCATCCC CACCCATCCT GGCAGAATCG 600
GAGCTTTGCC CCTGCAATCA ACCCACGGAA GCTCCGGGAA TGGCGGCCAA 650
GCACGCGGAT CC 662

(2) INFORMATION FOR SEQUENCE ID NO: 11:

(i) SEQUENCE CHARACTERISTICS:

(A) LENGTH: 1640 base pairs
(B) TYPE: nucleic acid
(D) TOPOLOGY: linear

(ii) MOLECULE TYPE: cDNA to mRNA
(ix) FEATURE:

(A) NAME/KEY: cDNA MAGE-3

(xi) SEQUENCE DESCRIPTION: SEQ ID NO: 11:

```
GCCGCGAGGG AAGCCGGCCC AGGCTCGGTG AGGAGGCAAG GTTCTGAGGG        50
GACAGGCTGA CCTGGAGGAC CAGAGGCCCC CGGAGGAGCA CTGAAGGAGA       100
AGATCTGCCA GTGGGTCTCC ATTGCCCAGC TCCTGCCCAC ACTCCCGCCT       150
GTTGCCCTGA CCAGAGTCAT C                                      171
ATG CCT CTT GAG CAG AGG AGT CAG CAC TGC AAG CCT GAA GAA      213
GGC CTT GAG GCC CGA GGA GAG GCC CTG GGC CTG GTG GGT GCG      255
CAG GCT CCT GCT ACT GAG GAG CAG GAG GCT GCC TCC TCC TCT      297
TCT ACT CTA GTT GAA GTC ACC CTG GGG GAG GTG CCT GCT GCC      339

GAG TCA CCA GAT CCT CCC CAG AGT CCT CAG GGA GCC TCC AGC      381
CTC CCC ACT ACC ATG AAC TAC CCT CTC TGG AGC CAA TCC TAT      423
GAG GAC TCC AGC AAC CAA GAA GAG GAG GGG CCA AGC ACC TTC      465
CCT GAC CTG GAG TCC GAG TTC CAA GCA GCA CTC AGT AGG AAG      507
GTG GCC GAG TTG GTT CAT TTT CTG CTC CTC AAG TAT CGA GCC      549
AGG GAG CCG GTC ACA AAG GCA GAA ATG CTG GGG AGT GTC GTC      591
GGA AAT TGG CAG TAT TTC TTT CCT GTG ATC TTC AGC AAA GCT      633
TCC AGT TCC TTG CAG CTG GTC TTT GGC ATC GAG CTG ATG GAA      675
GTG GAC CCC ATC GGC CAC TTG TAC ATC TTT GCC ACC TGC CTG      717
GGC CTC TCC TAC GAT GGC CTG CTG GGT GAC AAT CAG ATC ATG      759
CCC AAG GCA GGC CTC CTG ATA ATC GTC CTG GCC ATA ATC GCA      801
AGA GAG GGC GAC TGT GCC CCT GAG GAG AAA ATC TGG GAG GAG      843
CTG AGT GTG TTA GAG GTG TTT GAG GGG AGG GAA GAC AGT ATG      885
TTG GGG GAT CCC AAG AAG CTG CTC ACC CAA CAT TTC GTG CAG      927
GAA AAC TAC CTG GAG TAC CGG CAG GTC CCC GGC AGT GAT CCT      969
GCA TGT TAT GAA TTC CTG TGG GGT CCA AGG GCC CTC GTT GAA     1011
ACC AGC TAT GTG AAA GTC CTG CAC CAT ATG GTA AAG ATC AGT     1053
GGA GGA CCT CAC ATT TCC TAC CCA CCC CTG CAT GAG TGG GTT     1095
TTG AGA GAG GGG GAA GAG TGA                                 1116
GTCTGAGCAC GAGTTGCAGC CAGGGCCAGT GGGAGGGGGT CTGGGCCAGT      1166
GCACCTTCCG GGGCCGCATC CCTTAGTTTC CACTGCCTCC TGTGACGTGA      1216
GGCCCATTCT TCACTCTTTG AAGCGAGCAG TCAGCATTCT TAGTAGTGGG      1266
TTTCTGTTCT GTTGGATGAC TTTGAGATTA TTCTTTGTTT CCTGTTGGAG      1316
TTGTTCAAAT GTTCCTTTTA ACGGATGGTT GAATGAGCGT CAGCATCCAG      1366
GTTTATGAAT GACAGTAGTC ACACATAGTG CTGTTTATAT AGTTTAGGAG      1416
TAAGAGTCTT GttTTTTACT CAAATTgGGA AATCCATTCC ATTTTGTGAA      1466
TTGTGACATA ATAATAGCAG TGGTAAAAGT ATTTGCTTAA AATTGTGAGC      1516
GAATTAGCAA TAACATACAT GAGATAACTC AAGAAATCAA AAGATAGTTG      1566
ATTCTTGCCT TGTACCTCAA TCTATTCTGT AAAATTAAAC AAATATGCAA      1616
ACCAGGATTT CCTTGACTTC TTTG                                  1640
```

(2) INFORMATION FOR SEQUENCE ID NO: 12:

(i) SEQUENCE CHARACTERISTICS:

(A) LENGTH: 943 base pairs
(B) TYPE: nucleic acid
(D) TOPOLOGY: linear

(ii) MOLECULE TYPE: genomic DNA

(ix) FEATURE:

(A) NAME/KEY: MAGE-31 gene

(xi) SEQUENCE DESCRIPTION: SEQ ID NO: 12:

```
GGATCCTCCA CCCCAGTAGA GTGGGGACCT CACAGAGTCT GGCCAACCCT    50
CCTGACAGTT CTGGGAATCC GTGGCTGCGT TTGCTGTCTG CACATTGGGG   100
GCCCGTGGAT TCCTCTCCCA GGAATCAGGA GCTCCAGGAA CAAGGCAGTG   150
AGGACTTGGT CTGAGGCAGT GTCCTCAGGT CACAGAGTAG AGGGGgCTCA   200
GATAGTGCCA ACGGTGAAGG TTTGCCTTGG ATTCAAACCA AGGGCCCCAC   250
CTGCCCCAGA ACACATGGAC TCCAGAGCGC CTGGCCTCAC CCTCAATACT   300
TTCAGTCCTG CAGCCTCAGC ATGCGCTGGC CGGATGTACC CTGAGGTGCC   350
CTCTCACTTC CTCCTTCAGG TTCTGAGGGG ACAGGCTGAC CTGGAGGACC   400
AGAGGCCCCC GGAGGAGCAC TGAAGGAGAA GATCTGTAAG TAAGCCTTTG   450
TTAGAGCCTC CAAGGTTCCA TTCAGTACTC AGCTGAGGTC TCTCACATGC   500
TCCCTCTCTC CCCAGGCCAG TGGGTCTCCA TTGCCCAGCT CCTGCCCACA   550
CTCCCGCCTG TTGCCCTGAC CAGAGTCATC                         580
ATG CCT CTT GAG CAG AGG AGT CAG CAC TGC AAG CCT GAA GAA  622
GGC CTT GAG GCC CGA GGA GAg GCC CTG GGC CTG GTG GGT GCG  664
CAG GCT CCT GCT ACT GAG GAG CAG GAG GCT GCC TCC TCC TCT  706
TCT AGT GTA GTT GAA GTC ACC CTG GGG GAG GTG CCT GCT GCC  748
GAG TCA CCA GAT CCT CCC CAG AGT CCT CAG GGA GCC TCC AGC  790
CTC CCC ACT ACC ATG AAC TAC CCT CTC TGG AGC CAA TCC TAT  832
GAG GAC TCC AGC AAC CAA GAA GAG GAG GGG CCA AGC ACC TTC  874
CCT GAC CTG GAG TCT GAG TTC CAA GCA GCA CTC AGT AGG AAG  916
GTG GCC AAG TTG GTT CAT TTT CTG CTC                      943
```

(2) INFORMATION FOR SEQUENCE ID NO: 13:

(i) SEQUENCE CHARACTERISTICS:

(A) LENGTH: 2531 base pairs
(B) TYPE: nucleic acid
(D) TOPOLOGY: linear

(ii) MOLECULE TYPE: genomic DNA
(ix) FEATURE:

(A) NAME/KEY: MAGE-4 gene

(xi) SEQUENCE DESCRIPTION: SEQ ID NO: 13:

```
GGATCCAGGC CCTGCCTGGA GAAATGTGAG GGCCCTGAGT GAACACAGTG        50
GGGATCATCC ACTCCATGAG AGTGGGGACC TCACAGAGTC CAGCCTACCC       100
TCTTGATGGC ACTGAGGGAC CGGGGCTGTG CTTACAGTCT GCACCCTAAG       150
GGCCCATGGA TTCCTCTCCT AGGAGCTCCA GGAACAAGGC AGTGAGGCCT       200
TGGTCTGAGA CAGTGTCCTC AGGTTACAGA GCAGAGGATG CACAGGCTGT       250
GCCAGCAGTG AATGTTTGCC CTGAATGCAC ACCAAGGGCC CCACCTGCCA       300
CAAGACACAT AGGACTCCAA AGAGTCTGGC CTCACCTCCC TACCATCAAT       350
CCTGCAGAAT CGACCTCTGC TGGCCGGCTA TACCCTGAGG TGCTCTCTCA       400
CTTCCTCCTT CAGGTTCTGA GCAGACAGGC CAACCGGAGA CAGGATTCCC       450
TGGAGGCCAC AGAGGAGCAC CAAGGAGAAG ATCTGTAAGT AAGCCTTTGT       500
TAGAGCCTCT AAGATTTGGT TCTCAGCTGA GGTCTCTCAC ATGCTCCCTC       550
TCTCCGTAGG CCTGTGGGTC CCCATTGCCC AGCTTTTGCC TGCACTCTTG       600
CCTGCTGCCC TGACCAGAGT CATC                                   624
ATG TCT TCT GAG CAG AAG AGT CAG CAC TGC AAG CCT GAG GAA      666
GGC GTT GAG GCC CAA GAA GAG GCC CTG GGC CTG GTG GGT GCA      708
CAG GCT CCT ACT ACT GAG GAG CAG GAG GCT GCT GTC TCC TCC      750
TCC TCT CCT CTG GTC CCT GGC ACC CTG GAG GAA GTG CCT GCT      792
GCT GAG TCA GCA GGT CCT CCC CAG AGT CCT CAG GGA GCC TCT      834
GCC TTA CCC ACT ACC ATC AGC TTC ACT TGC TGG AGG CAA CCC      876
AAT GAG GGT TCC AGC AGC CAA GAA GAG GAG GGG CCA AGC ACC      918
TCG CCT GAC GCA GAG TCC TTG TTC CGA GAA GCA CTC AGT AAC      960
AAG GTG GAT GAG TTG GCT CAT TTT CTG CTC CGC AAG TAT CGA     1002
GCC AAG GAG CTG GTC ACA AAG GCA GAA ATG CTG GAG AGA GTC     1044
ATC AAA AAT TAC AAG CGC TGC TTT CCT GTG ATC TTC GGC AAA     1086
GCC TCC GAG TCC CTG AAG ATG ATC TTT GGC ATT GAC GTG AAG     1128
GAA GTG GAC CCC GCC AGC AAC ACC TAC ACC CTT GTC ACC TGC     1170
CTG GGC CTT TCC TAT GAT GGC CTG CTG GGT AAT AAT CAG ATC     1212
TTT CCC AAG ACA GGC CTT CTG ATA ATC GTC CTG GGC ACA ATT     1254
GCA ATG GAG GGC GAC AGC GCC TCT GAG GAG GAA ATC TGG GAG     1296
GAG CTG GGT GTG ATG GGG GTG TAT GAT GGG AGG GAG CAC ACT     1338
GTC TAT GGG GAG CCC AGG AAA CTG CTC ACC CAA GAT TGG GTG     1380
CAG GAA AAC TAC CTG GAG TAC CGG CAG GTA CCC GGC AGT AAT     1422
CCT GCG CGC TAT GAG TTC CTG TGG GGT CCA AGG GCT CTG GCT     1464
GAA ACC AGC TAT GTG AAA GTC CTG GAG CAT GTG GTC AGG GTC     1506
AAT GCA AGA GTT CGC ATT GCC TAC CCA TCC CTG CGT GAA GCA     1548
GCT TTG TTA GAG GAG GAA GAG GGA GTC TGA                     1578
GCATGAGTTG CAGCCAGGGC TGTGGGGAAG GGGCAGGGCT GGGCCAGTGC      1628
ATCTAACAGC CCTGTGCAGC AGCTTCCCTT GCCTCGTGTA ACATGAGGCC      1678
CATTCTTCAC TCTGTTTGAA GAAAATAGTC AGTGTTCTTA GTAGTGGGTT      1728
TCTATTTTGT TGGATGACTT GGAGATTTAT CTCTGTTTCC TTTTACAATT      1778
GTTGAAATGT TCCTTTTAAT GGATGGTTGA ATTAACTTCA GCATCCAAGT      1828
TTATGAATCG TAGTTAACCT ATATTGCTGT TAATATAGTT TAGGAGTAAG      1878
AGTCTTGTTT TTTATTCAGA TTGGGAAATC CGTTCTATTT TGTGAATTTG      1928
GGACATAATA ACAGCAGTGG AGTAAGTATT TAGAAGTGTG AATTCACCGT      1978
GAAATAGGTG AGATAAATTA AAAGATACTT AATTCCCGCC TTATGCCTCA      2028
GTCTATTCTG TAAAATTTAA AAATATATAT GCATACCTGG ATTTCCTTGG      2078
CTTCGTGAAT GTAAGAGAAA TTAAATCTGA ATAAATAATT CTTTCTGTTA      2128
ACTGGCTCAT TTCTTCTCTA TGCACTGAGC ATCTGCTCTG TGGAAGGCCC      2178
AGGATTAGTA GTGGAGATAC TAGGGTAAGC CAGACACACA CCTACCGATA      2228
GGGTATTAAG AGTCTAGGAG CGCGGTCATA TAATTAAGGT GACAAGATGT      2278
CCTCTAAGAT GTAGGGGAAA AGTAACGAGT GTGGGTATGG GGCTCCAGGT      2328
GAGAGTGGTC GGGTGTAAAT TCCCTGTGTG GGCCCTTTTG GGCTTTGGGA      2378
AACTGCATTT TCTTCTGAGG GATCTGATTC TAATGAAGCT TGGTGGGTCC      2428
AGGGCCAGAT TCTCAGAGGG AGAGGGAAAA GCCCAGATTG GAAAAGTTGC      2478

TCTGAGCAGT TCCTTTGTGA CAATGGATGA ACAGAGAGGA GCCTCTACCT      2528
GGG                                                         2531
```

(2) INFORMATION FOR SEQUENCE ID NO: 14:

(i) SEQUENCE CHARACTERISTICS:

(A) LENGTH: 2531 base pairs
(B) TYPE: nucleic acid
(D) TOPOLOGY: linear

(ii) MOLECULE TYPE: genomic DNA
(ix) FEATURE:

(A) NAME/KEY: MA6B-41 gene

(xi) SEQUENCE DESCRIPTION: SEQ ID NO: 14:

```
GGATCCAGGC CCTGCCTGGA GAAATGTGAG GGCCCTGAGT GAACACAGTG      50
GGGATCATCC ACTCCATGAG AGTGGGGACC TCACAGAGTC CAGCCTACCC     100
TCTTGATGGC ACTGAGGGAC CGGGGCTGTG CTTACAGTCT GCACCCTAAG     150
GGCCCATGGA TTCCTCTCCT AGGAGCTCCA GGAACAAGGC AGTGAGGCCT     200
TGGTCTGAGA CAGTGTCCTC AGGTTACAGA GCAGAGGATG CACAGGCTGT     250
GCCAGCAGTG AATGTTTGCC CTGAATGCAC ACCAAGGGCC CCACCTGCCA     300
CAAGACACAT AGGACTCCAA AGAGTCTGGC CTCACCTCCC TACCATCAAT     350
CCTGCAGAAT CGACCTCTGC TGGCCGGCTA TACCCTGAGG TGCTCTCTCA     400
CTTCCTCCTT CAGGTTCTGA GCAGACAGGC CAACCGGAGA CAGGATTCCC     450
TGGAGGCCAC AGAGGAGCAC CAAGGAGAAG ATCTGTAAGT AAGCCTTTGT     500
TAGAGCCTCT AAGATTTGGT TCTCAGCTGA GGTCTCTCAC ATGCTCCCTC     550
TCTCCGTAGG CCTGTGGGTC CCCATTGCCC AGCTTTTGCC TGCACTCTTG     600
CCTGCTGCCC TGAGCAGAGT CATC                                 624
ATG TCT TCT GAG CAG AAG AGT CAG CAC TGC AAG CCT GAG GAA    666
GGC GTT GAG GCC CAA GAA GAG GCC CTG GGC CTG GTG GGT GCG    708
CAG GCT CCT ACT ACT GAG GAG CAG GAG GCT GCT GTC TCC TCC    750
TCC TCT CCT CTG GTC CCT GGC ACC CTG GAG GAA GTG CCT GCT    792
GCT GAG TCA GCA GGT CCT CCC CAG AGT CCT CAG GGA GCC TCT    834
GCC TTA CCC ACT ACC ATC AGC TTC ACT TGC TGG AGG CAA CCC    876
AAT GAG GGT TCC AGC AGC CAA GAA GAG GAG GGG CCA AGC ACC    918
TCG CCT GAC GCA GAG TCC TTG TTC CGA GAA GCA CTC AGT AAC    960
AAG GTG GAT GAG TTG GCT CAT TTT CTG CTC CGC AAG TAT CGA   1002
GCC AAG GAG CTG GTC ACA AAG GCA GAA ATG CTG GAG AGA GTC   1044
ATC AAA AAT TAC AAG CGC TGC TTT CCT GTG ATC TTC GGC AAA   1086
GCC TCC GAG TCC CTG AAG ATG ATC TTT GGC ATT GAC GTG AAG   1128
GAA GTG GAC CCC ACC AGC AAC ACC TAC ACC CTT GTC ACC TGC   1170
CTG GGC CTT TCC TAT GAT GGC CTG CTG GGT AAT AAT CAG ATC   1212
TTT CCC AAG ACA GGC CTT CTG ATA ATC GTC CTG GGC ACA ATT   1254
GCA ATG GAG GGC GAC AGC GCC TCT GAG GAG GAA ATC TGG GAG   1296
GAG CTG GGT GTG ATG GGG GTG TAT GAT GGG AGG GAG CAC ACT   1338
GTC TAT GGG GAG CCC AGG AAA CTG CTC ACC CAA GAT TGG GTG   1380
CAG GAA AAC TAC CTG GAG TAC CGG CAG GTA CCC GGC AGT AAT   1422
CCT GCG CGC TAT GAG TTC CTG TGG GGT CCA AGG GCT CTG GCT   1464
GAA ACC AGC TAT GTG AAA GTC CTG GAG CAT GTG GTC AGG GTC   1506
AAT GCA AGA GTT CGC ATT GCC TAC CCA TCC CTG CGT GAA GCA   1548
GCT TTG TTA GAG GAG GAA GAG GGA GTC TGA                   1578
GCATGAGTTG CAGCCAGGGC TGTGGGGAAG GGGCAGGGCT GGGCCAGTGC    1628
ATCTAACAGC CCTGTGCAGC AGCTTCCCTT GCCTCGTGTA ACATGAGGCC    1678
CATTCTTCAC TCTGTTTGAA GAAAATAGTC AGTGTTCTTA GTAGTGGGTT    1728
TCTATTTTGT TGGATGACTT GGAGATTTAT CTCTGTTTCC TTTTACAATT    1778
GTTGAAATGT TCCTTTTAAT GGATGGTTGA ATTAACTTCA GCATCCAAGT    1828
TTATGAATCG TAGTTAACGT ATATTGCTGT TAATATAGTT TAGGAGTAAG    1878
AGTCTTGTTT TTTATTCAGA TTGGGAAATC CGTTCTATTT TGTGAATTTG    1928
GGACATAATA ACAGCAGTGG AGTAAGTATT TAGAAGTGTG AATTCACCGT    1978
GAAATAGGTG AGATAAATTA AAAGATACTT AATTCCCGCC TTATGCCTCA    2028
GTCTATTCTG TAAAATTTAA AAATATATAT GCATACCTGG ATTTCCTTGG    2078
CTTCGTGAAT GTAAGAGAAA TTAAATCTGA ATAAATAATT CTTTCTGTTA    2128
ACTGGCTCAT TTCTTCTCTA TGCACTGAGC ATCTGCTCTG TGGAAGGCCC    2178
AGGATTAGTA GTGGAGATAC TAGGGTAAGC CAGACACACA CCTACCGATA    2228

GGGTATTAAG AGTCTAGGAG CGCGGTCATA TAATTAAGGT GACAAGATGT    2278
CCTCTAAGAT GTAGGGGAAA AGTAACGAGT GTGGGTATGG GGCTCCAGGT    2328
GAGAGTGGTC GGGTGTAAAT TCCCTGTGTG GGGCCTTTTG GGCTTTGGGA    2378
AACTCCATTT TCTTCTGAGG GATCTGATTC TAATGAAGCT TGGTGGGTCC    2428
AGGGCCAGAT TCTCAGAGGG AGAGGGAAAA GCCCAGATTG GAAAAGTTGC    2478
TCTGAGCGGT TCCTTTGTGA CAATGGATGA ACAGAGAGGA GCCTCTACCT    2528
GGG                                                       2531
```

(2) INFORMATION FOR SEQUENCE ID NO: 15:

(i) SEQUENCE CHARACTERISTICS:

(A) LENGTH: 1068 base pairs
(B) TYPE: nucleic acid
(D) TOPOLOGY: linear

(ii) MOLECULE TYPE: cDNA to mRNA
(ix) FEATURE:

(A) NAME/KEY: cDNA MAGE-4

(xi) SEQUENCE DESCRIPTION: SEQ ID NO: 15:

```
  G GGG CCA AGC ACC TCG CCT GAC GCA GAG TCC TTG TTC CGA      40
GAA GCA CTC AGT AAC AAG GTG GAT GAG TTG GCT CAT TTT CTG      82
CTC CGC AAG TAT CGA GCC AAG GAG CTG GTC ACA AAG GCA GAA     124
ATG CTG GAG AGA GTC ATC AAA AAT TAC AAG CGC TGC TTT CCT     166
GTG ATC TTC GGC AAA GCC TCC GAG TCC CTG AAG ATG ATC TTT     208
GGC ATT GAC GTG AAG GAA GTG GAC CCC GCC AGC AAC ACC TAC     250
ACC CTT GTC ACC TGC CTG GGC CTT TCC TAT GAT GGC CTG CTG     292
GGT AAT AAT CAG ATC TTT CCC AAG ACA GGC CTT CTG ATA ATC     334
GTC CTG GGC ACA ATT GCA ATG GAG GGC GAC AGC GCC TCT GAG     376
GAG GAA ATC TGG GAG GAG CTG GGT GTG ATG GGG GTG TAT GAT     418
GGG AGG GAG CAC ACT GTC TAT GGG GAG CCC AGG AAA CTG CTC     460
ACC CAA GAT TGG GTG CAG GAA AAC TAC CTG GAG TAC CGG CAG     502
GTA CCC GGC AGT AAT CCT GCG CGC TAT GAG TTC CTG TGG GGT     544
CCA AGG GCT CTG GCT GAA ACC AGC TAT GTG AAA GTC CTG GAG     586
CAT GTG GTC AGG GTC AAT GCA AGA GTT CGC ATT GCC TAC CCA     628
TCC CTG CGT GAA GCA GCT TTG TTA GAG GAG GAA GAG GGA GTC     670
TGAGCATGAG TTGCAGCCAG GGCTGTGGGG AAGGGGCAGG GCTGGGCCAG     720
TGCATCTAAC AGCCCTGTGC AGCAGCTTCC CTTGCCTCGT GTAACATGAG     770
GCCCATTCTT CACTCTGTTT GAAGAAAATA GTCAGTGTTC TTAGTAGTGG     820
GTTTCTATTT TGTTGGATGA CTTGGAGATT TATCTCTGTT TCCTTTTACA     870
ATTGTTGAAA TGTTCCTTTT AATGGATGGT TGAATTAACT TCAGCATCCA     920
AGTTTATGAA TCGTAGTTAA CGTATATTGC TGTTAATATA GTTTAGGAGT     970
AAGAGTCTTG TTTTTTATTC AGATTGGGAA ATCCGTTCTA TTTTGTGAAT    1020
TTGGGACATA ATAACAGCAG TGGAGTAAGT ATTTAGAAGT GTGAATTC      1068
```

(2) INFORMATION FOR SEQUENCE ID NO: 16:

(i) SEQUENCE CHARACTERISTICS:

(A) LENGTH: 2226 base pairs
(B) TYPE, nucleic acid
(D) TOPOLOGY: linear

(ii) MOLECULE TYPE: genomic DNA
(ix) FEATURE:

(A) NAME/KEY: MAGE-5 gene

(xi) SEQUENCE DESCRIPTION: SEQ ID NO: 16:

GGATCCAGGC CTTGCCAGGA GAAAGGTGAG GGCCCTGTGT GAGCACAGAG 50
GGGACCATTC ACCCCAAGAG GGTGGAGACC TCACAGATTC CAGCCTACCC 100
TCCTGTTAGC ACTGGGGGCC TGAGGCTGTG CTTGCAGTCT GCACCCTGAG 150
GGCCCATGCA TTCCTCTTCC AGGAGCTCCA GGAAACAGAC ACTGAGGCCT 200
TGGTCTGAGG CCGTGCCCTC AGGTCACAGA GCAGAGGAGA TGCAGACGTC 250
TAGTGCCAGC AGTGAACGTT TGCCTTGAAT GCACACTAAT GGCCCCCATC 300

GCCCCAGAAC ATATGGGACT CCAGAGCACC TGGCCTCACC CTCTCTACTG 350
TCAGTCCTGC AGAATCAGCC TCTGCTTGCT TGTGTACCCT GAGGTGCCCT 400
CTCACTTTTT CCTTCAGGTT CTCAGGGGAC AGGCTGACCA GGATCACCAG 450
GAAGCTCCAG AGGATCCCCA GGAGGCCCTA GAGGAGCACC AAAGGAGAAG 500
ATCTGTAAGT AAGCCTTTGT TAGAGCCTCC AAGGTTCAGT TTTTAGCTGA 550
GGCTTCTCAC ATGCTCCCTC TCTCTCCAGG CCAGTGGGTC TCCATTGCCC 600
AGCTCCTGCC CACACTCCTG CCTGTTGCGG TGACCAGAGT CGTC 644
ATG TCT CTT GAG CAG AAG AGT CAG CAC TGC AAG CCT GAG GAA 684
CTC CTC TGG TCC CAG GCA CCC TGG GGG AGG TGC CTG CTG CTG 728
GGT CAC CAG GTC CTC TCA AGA GTC CTC AGG GAG CCT CCG CCA 770
TCC CCA CTG CCA TCG ATT TCA CTC TAT GGA GGC AAT CCA TTA 812
AGG GCT CCA GCA ACC AAG AAG AGG AGG GGC CAA GCA CCT CCC 854
CTG ACC CAG AGT CTG TGT TCC GAG CAG CAC TCA GTA AGA AGG 896
TGG CTG ACT TGA 908
TTCATTTTCT GCTCCTCAAG TATTAAGTCA AGGAGCTGGT CACAAAGGCA 958
GAAATGCTGG AGAGCGTCAT CAAAAATTAC AAGCGCTGCT TTCCTGAGAT 1008
CTTCGGCAAA GCCTCCGAGT CCTTGCAGCT GGTCTTTGGC ATTGACGTGA 1058
AGGAAGCGGA CCCCACCAGC AACACCTACA CCCTTGTCAC CTGCCTGGGA 1108
CTCCTATGAT GGCCTGCTGG TTGATAATAA TCAGATCATG CCCAAGACGG 1158
GCCTCCTGAT AATCGTCTTG GGCATGATTG CAATGGAGGG CAAATGCGTC 1208
CCTGAGGAGA AAATCTGGGA GGAGCTGAGT GTGATGAAGG TGTATGTTGG 1258
GAGGGAGCAC AGTGTCTGTG GGGAGCCCAG GAAGCTGCTC ACCCAAGATT 1308
TGGTGCAGGA AAACTACCTG GAGTACCGGC AGGTGCCCAG CAGTGATCCC 1358
ATATGCTATG AGTTACTGTG GGGTCCAAGG GCACTCGCTG CTTGAAAGTA 1408
CTGGAGCACG TGGTCAGGGT CAATGCAAGA GTTCTCATTT CCTACCCATC 1458
CCTGCGTGAA GCAGCTTTGA GAGAGGAGGA AGAGGGAGTC TGAGCATGAG 1508
CTGCAGCCAG GGCCACTGCG AGGGGGGCTG GGCCAGTGCA CCTTCCAGGG 1558
CTCCGTCCAG TAGTTTCCCC TGCCTTAATG TGACATGAGG CCCATTCTTC 1608
TCTCTTTGAA GAGAGCAGTC AACATTCTTA GTAGTGGGTT TCTGTTCTAT 1658
TGGATGACTT TGAGATTTGT CTTTGTTTCC TTTTGGAATT GTTCAAATGT 1708
TTCTTTTAAT GGGTGGTTGA ATGAACTTCA GCATTCAAAT TTATGAATGA 1758
CAGTAGTCAC ACATAGTGCT GTTTATATAG TTTAGGAGTA AGAGTCTTGT 1808
TTTTTATTCA GATTGGGAAA TCCATTCCAT TTTGTGAATT GGGACATAGT 1858
TACAGCAGTG GAATAAGTAT TCATTTAGAA ATGTGAATGA GCAGTAAAAC 1908
TGATGACATA AAGAAATTAA AAGATATTTA ATTCTTGCTT ATACTCAGTC 1958
TATTCGGTAA AATTTTTTTT AAAAAATGTG CATACCTGGA TTTCCTTGGC 2008
TTCTTTGAGA ATGTAAGACA AATTAAATCT GAATAAATCA TTCTCCCTGT 2058
TCACTGGCTC ATTTATTCTC TATGCACTGA GCATTTGCTC TGTGGAAGGC 2108
CCTGGGTTAA TAGTGGAGAT GCTAAGGTAA GCCAGACTCA CCCCTACCCA 2158
CAGGGTAGTA AAGTCTAGGA GCAGCAGTCA TATAATTAAG GTGGAGAGAT 2208
GCCCTCTAAG ATGTAGAG 2226

(2) INFORMATION FOR SEQUENCE ID NO: 17:

(i) SEQUENCE CHARACTERISTICS:

(A) LENGTH: 2305 base pairs
(B) TYPE: nucleic acid
(D) TOPOLOGY: linear

(ii) MOLECULE TYPE: genomic DNA

(ix) FEATURE:

(A) NAME/KEY: MAGE-51 gene

(xi) SEQUENCE DESCRIPTION: SEQ ID NO: 17:

```
GGATCCAGGC CTTGCCAGGA GAAAGGTGAG GGCCCTGTGT GAGCACAGAG     50
GGGACCATTC ACCCCAAGAG GGTGGAGACC TCACAGATTC CAGCCTACCC    100
TCCTGTTAGC ACTGGGGGCC TGAGGCTGTG CTTGCAGTCT GCACCCTGAG    150
GGCCCATGCA TTCCTCTTCC AGGAGCTCCA GGAAACAGAC ACTGAGGCCT    200
TGGTCTGAGG CCGTGCCCTC AGGTCACAGA GCAGAGGAGA TGCAGACGTC    250
TAGTGCCAGC AGTGAACGTT TGCCTTGAAT GCACACTAAT GGCCCCCATC    300
GCCCCAGAAC ATATGGGACT CCAGAGCACC TGGCCTCACC CTCTCTACTG    350
TCAGTCCTGC AGAATCAGCC TCTGCTTGCT TGTGTACCCT GAGGTGCCCT    400
CTCACTTTTT CCTTCAGGTT CTCAGGGGAC AGGCTGACCA GGATCACCAG    450
GAAGCTCCAG AGGATCCCCA GGAGGCCCTA GAGGAGCACC AAAGGAGAAG    500
ATCTGTAAGT AAGCCTTTGT TAGAGCCTCC AAGGTTCAGT TTTTAGCTGA    550
```

```
GGCTTCTCAC ATGCTCCCTC TCTCTCCAGG CCAGTGGGTC TCCATTGCCC    600
AGCTCCTGCC CACACTCCTG CCTGTTGCGG TGACCAGAGT CGTC          644
ATG TCT CTT GAG CAG AAG AGT CAG CAC TGC AAG CCT GAG GAA   686
GGC CTT GAC ACC CAA GAA GAG CCC TGG GCC TGG TGG GTG TGC   728
AGG CTG CCA CTA CTG AGG AGC AGG AGG CTG TGT CCT CCT CCT   770
CTC CTC TGG TCC CAG GCA CCC TGG GGG AGG TGC CTG CTG CTG   812
GGT CAC CAG GTC CTC TCA AGA GTC CTC AGG GAG CCT CCG CCA   854
TCC CCA CTG CCA TCG ATT TCA CTC TAT GGA GGC AAT CCA TTA   896
AGG GCT CCA GCA ACC AAG AAG AGG AGG GGC CAA GCA CCT CCC   938
CTG ACC CAG AGT CTG TGT TCC GAG CAG CAC TCA GTA AGA AGG   980
TGG CTG ACT TGA                                           992
TTCATTTTCT GCTCCTCAAG TATTAAGTCA AGGAGCCGGT CACAAAGGCA   1042
GAAATGCTGG AGAGCGTCAT CAAAAATTAC AAGCGCTGCT TTCCTGAGAT   1092
CTTCGGCAAA GCCTCCGAGT CCTTGCAGCT GGTCTTTGGC ATTGACGTGA   1142
AGGAAGCGGA CCCCACCAGC AACACCTACA CCCTTGTCAC CTGCCTGGGA   1192
CTCCTATGAT GGCCTGGTGG TTTAATCAGA TCATGCCCAA GACGGGCCTC   1242
CTGATAATCG TCTTGGGCAT GATTGCAATG GAGGGCAAAT GCGTCCCTGA   1292
GGAGAAAATC TGGGAGGAGC TGGGTGTGAT GAAGGTGTAT GTTGGGAGGG   1342
AGCACAGTGT CTGTGGGGAG CCCAGGAAGC TGCTCACCCA AGATTTGGTG   1392
CAGGAAAACT ACCTGGAGTA CCGCAGGTGC CCAGCAGTGA TCCCATATGC   1442
TATGAGTTAC TGTGGGGTCC AAGGGCACTC GCTGCTTGAA AGTACTGGAG   1492
CACGTGGTCA GGGTCAATGC AAGAGTTCTC ATTTCCTACC CATCCCTGCA   1542
TGAAGCAGCT TTGAGAGAGG AGGAAGAGGG AGTCTGAGCA TGAGCTGCAG   1592
CCAGGGCCAC TGCGAGGGGG GCTGGGCCAG TGCACCTTCC AGGGCTCCGT   1642
CCAGTAGTTT CCCCTGCCTT AATGTGACAT GAGGCCCATT CTTCTCTCTT   1692
TGAAGAGAGC AGTCAACATT CTTAGTAGTG GGTTTCTGTT CTATTGGATG   1742
ACTTTGAGAT TTGTCTTTGT TTCCTTTTGG AATTGTTCAA ATGTTCCTTT   1792
TAATGGGTGG TTGAATGAAC TTCAGCATTC AAATTTATGA ATGACAGTAG   1842
TCACACATAG TGCTGTTTAT ATAGTTTAGG AGTAAGAGTC TTGTTTTTTA   1892
TTCAGATTGG GAAATCCATT CCATTTTGTG AATTGGGACA TAGTTACAGC   1942
AGTGGAATAA GTATTCATTT AGAAATGTGA ATGAGCAGTA AAACTGATGA   1992
GATAAAGAAA TTAAAAGATA TTTAATTCTT GCCTTATACT CAGTCTATTC   2042
GGTAAAATTT TTTTTTAAAA ATGTGCATAC CTGGATTTCC TTGGCTTCTT   2092
TGAGAATGTA AGACAAATTA AATCTGAATA AATCATTCTC CCTGTTCACT   2142
GGCTCATTTA TTCTCTATGC ACTGAGCATT TGCTCTGTGG AAGGCCCTGG   2192
GTTAATAGTG GAGATGCTAA GGTAAGCCAG ACTCACCCCT ACCCACAGGG   2242
TAGTAAAGTC TAGGAGCAGC AGTCATATAA TTAAGGTGGA GAGATGCCCT   2292
CTAAGATGTA GAG                                           2305
```

(2) INFORMATION FOR SEQUENCE ID NO: 18:

(i) SEQUENCE CHARACTERISTICS:

(A) LENGTH: 225 base pairs
(B) TYPE: nucleic acid
(D) TOPOLOGY: linear

(ii) MOLECULE TYPE: CDNA
(ix) FEATURE:

(A) NAME/KEY: MAGE-6 gene

(xi) SEQUENCE DESCRIPTION: SEQ ID NO: 18:

```
TAT TTC TTT CCT GTG ATC TTC AGC AAA GCT TCC GAT TCC TTG    42
CAG CTG GTC TTT GGC ATC GAG CTG ATG GAA GTG GAC CCC ATC    84
GGC CAC GTG TAC ATC TTT GCC ACC TGC CTG GGC CTC TCC TAC   126
GAT GGC CTG CTG GGT GAC AAT CAG ATC ATG CCC AGG ACA GGC   168
TTC CTG ATA ATC ATC CTG GCC ATA ATC GCA AGA GAG GGC GAC   210
TGT GCC CCT GAG GAG                                       225
```

(2) INFORMATION FOR SEQUENCE ID NO: 19:

(i) SEQUENCE CHARACTERISTICS:

(A) LENGTH: 1947 base pairs
(B) TYPE: nucleic acid
(D) TOPOLOGY: linear

(ii) MOLECULE TYPE: genomic DNA
(ix) FEATURE:

(A) NAME/KEY: MAGE-7 gene

(xi) SEQUENCE DESCRIPTION: SEQ ID NO: 19:

TGAATGGACA ACAAGGGCCC CACACTCCCC AGAACACAAG GGACTCCAGA 50
GAGCCCAGCC TCACCTTCCC TACTGTCAGT CCTGCAGCCT CAGCCTCTGC 100
TGGCCGGCTG TACCCTGAGG TGCCCTCTCA CTTCCTCCTT CAGGTTCTCA 150
GCGGACAGGC CGGCCAGGAG GTCAGAAGCC CCAGGAGGCC CCAGAGGAGC 200
ACCGAAGGAG AAGATCTGTA AGTAGGCCTT TGTTAGGGCC TCCAGGGCGT 250
GGTTCACAAA TGAGGCCCCT CACAAGCTCC TTCTCTCCCC AGATCTGTGG 300
GTTCCTCCCC ATCGCCCAGC TGCTGCCCGC ACTCCAGCCT GCTGCCCTGA 350
CCAGAGTCAT CATGTCTTCT GAGCAGAGGA GTCAGCACTG CAAGCCTGAG 400
GATGCCTTGA GGCCCAAGGA CAGGAGGCTC TGGGCCTGGT GGGTGCGCAG 450
GCTCCCGCCA CCGAGGAGCA CGAGGCTGCC TCCTCCTTCA CTCTGATTGA 500
AGGCACCCTG GAGGAGGTGC CTGCTGCTGG GTCCCCCAGT CCTCCCCTGA 550
GTCTCAGGGT TCCTCCTTTT CCCTGACCAT CAGCAACAAC ACTCTATGGA 600
GCCAATCCAG TGAGGGCACC AGCAGCCGGG AAGAGGAGGG GCCAACCACC 650
TAGACACACC CCGCTCACCT GGCGTCCTTG TTCCA 685
ATG GGA AGG TGG CTG AGT TGG TTC GCT TCC TGC TGC ACA AGT 727
ATC GAG TCA AGG AGC TGG TCA CAA AGG CAG AAA TGC TGG ACA 769
GTG TCA TCA AAA ATT ACA AGC ACT AGT TTC CTT GTG ATC TAT 811
GGC AAA GCC TCA GAG TGC ATG CAG GTG ATG TTT GGC ATT GAC 853
ATG AAG GAA GTG GAC CCC GCG GCC ACT CCT ACG TCC TTG TCA 895
CCT GCT TGG GCC TCT CCT ACA ATG GCC TGC TGG GTG ATG ATC 937
AGA GCA TGC CCG AGA CCG GCC TTC TGA 964
TTATGGTCTT GACCATGATC TTAATGGAGG GCCACTGTGC CCCTGAGGAG 1014
GCAATCTGGG AAGCGTTGAG TGTAATGGTG TATGATGGGA TGGAGCAGTT 1064
TCTTTGGGCA GCTGAGGAAG CTGCTCACCC AAGATTGGGT GCAGGAAAAC 1114
TACCTGCAAT ACCGCCAGGT GCCCAGCAGT GATCCCCCGT GCTACCAGTT 1164
CCTGTGGGGT CCAAGGGCCC TCATTGAAAC CAGCTATGTG AAAGTCCTGG 1214
AGTATGCAGC CAGGGTCAGT ACTAAAGAGA GCATTTCCTA CCCATCCCTG 1264
CATGAAGAGG CTTTGGGAGA GGAGGAAGAG GGAGTCTGAG CAGAAGTTGC 1314
AGCCAGGGCC AGTGGGGCAG ATTGGGGGAG GGCCTGGGCA GTGCACGTTC 1364
CACACATCCA CCACCTTCCC TGTCCTGTTA CATGAGGCCC ATTCTTCACT 1414
CTGTGTTTGA AGAGAGCAGT CAATGTTCTC AGTAGCGGGG AGTGTGTTGG 1464
GTGTGAGGGA ATACAAGGTG GACCATCTCT CAGTTCCTGT TCTCTTGGGC 1514
GATTTGGAGG TTTATCTTTG TTTCCTTTTG CAGTCGTTCA AATGTTCCTT 1564
TTAATGGATG GTGTAATGAA CTTCAACATT CATTTCATGT ATGACAGTAG 1614
GCAGACTTAC TGTTTTTTAT ATAGTTAAAA GTAAGTGCAT TGTTTTTTAT 1664
TTATGTAAGA AAATCTATGT TATTTCTTGA ATTGGGACAA CATAACATAG 1714
CAGAGGATTA AGTACCTTTT ATAATGTGAA AGAACAAAGC GGTAAAATGG 1764
GTGAGATAAA GAAATAAAGA AATTAAATTG GCTGGGCACG GTGGCTCACG 1814
CCTGTAATCC CAGCACTTTA GGAGGCAGAG GCACGGGGAT CACGAGGTCA 1864
GGAGATCGAG ACCATTCTGG CTAACACAGT GAAACACCAT CTCTATTAAA 1914
AATACAAAAC TTAGCCGGGC GTGGTGGCGG GTG 1947

(2) INFORMATION FOR SEQUENCE ID NO: 20:

(i) SEQUENCE CHARACTERISTICS:

(A) LENGTH: 1810 base pairs
(B) TYPE: nucleic acid
(D) TOPOLOGY: linear

(ii) MOLECULE TYPE: genomic DNA
(ix) FEATURE:

(A) NAME/KEY: MAGE-8 gene

(xi) SEQUENCE DESCRIPTION: SEQ ID NO: 20:

```
GAGCTCCAGG AACCAGGCTG TGAGGTCTTG GTCTGAGGCA GTATCTTCAA    50
TCACAGAGCA TAAGAGGCCC AGGCAGTAGT AGCAGTCAAG CTGAGGTGGT   100
```

```
GTTTCCCCTG TATGTATACC AGAGGCCCCT CTGGCATCAG AACAGCAGGA   150
ACCCCACAGT TCCTGGCCCT ACCAGCCCTT TTGTCAGTCC TGGAGCCTTG   200
GCCTTTGCCA GGAGGCTGCA CCCTGAGATG CCCTCTCAAT TTCTCCTTCA   250
GGTTCGCAGA GAACAGGCCA GCCAGGAGGT CAGGAGGCCC CAGAGAAGCA   300
CTGAAGAAGA CCTGTAAGTA GACCTTTGTT AGGGCATCCA GGGTGTAGTA   350
CCCAGCTGAG GCCTCTCACA CGCTTCCTCT CTCCCCAGGC CTGTGGGTCT   400
CAATTGCCCA GCTCCGGCCC ACACTCTCCT GCTGCCCTGA CCTGAGTCAT   450
C                                                         451
ATG CTT CTT GGG CAG AAG AGT CAG CGC TAC AAG GCT GAG GAA   493
GGC CTT CAG GCC CAA GGA GAG GCA CCA GGG CTT ATG GAT GTG   535
CAG ATT CCC ACA GCT GAG GAG CAG AAG GCT GCA TCC TCC TCC   577
TCT ACT CTG ATC ATG GGA ACC CTT GAG GAG GTG ACT GAT TCT   619
GGG TCA CCA AGT CCT CCC CAG AGT CCT GAG GGT GCC TCC TCT   661
TCC CTG ACT GTC ACC GAC AGC ACT CTG TGG AGC CAA TCC GAT   703
GAG GGT TCC AGC AGC AAT GAA GAG GAG GGG CCA AGC ACC TCC   745
CCG GAC CCA GCT CAC CTG GAG TCC CTG TTC CGG GAA GCA CTT   787
GAT GAG AAA GTG GCT GAG TTA GTT CGT TTC CTG CTC CGC AAA   829
TAT CAA ATT AAG GAG CCG GTC ACA AAG GCA GAA ATG CTT GAG   871
AGT GTC ATC AAA AAT TAC AAG AAC CAC TTT CCT GAT ATC TTC   913
AGC AAA GCC TCT GAG TGC ATG CAG GTG ATC TTT GGC ATT GAT   955
GTG AAG GAA GTG GAC CCT GCC GGC CAC TCC TAC ATC CTT GTC   997
ACC TGC CTG GGC CTC TCC TAT GAT GGC CTG CTG GGT GAT GAT  1039
CAG AGT ACG CCC AAG ACC GGC CTC CTG ATA ATC GTC CTG GGC  1081
ATG ATC TTA ATG GAG GGC AGC CGC GCC CCG GAG GAG GCA ATC  1123
TGG GAA GCA TTG AGT GTG ATG GGG GCT GTA TGA              1156
TGGGAGGGAG CACAGTGTCT ATTGGAAGCT CAGGAAGCTG CTCACCCAAG  1206
AGTGGGTGCA GGAGAACTAC CTGGAGTACC GCCAGGCGCC CGGCAGTGAT  1256
CCTGTGCGCT ACGAGTTCCT GTGGGGTCCA AGGGCCCTTG CTGAAACCAG  1306
CTATGTGAAA GTCCTGGAGC ATGTGGTCAG GGTCAATGCA AGAGTTCGCA  1356
TTTCCTACCC ATCCCTGCAT GAAGAGGCTT TGGGAGAGGA GAAAGGAGTT  1406
TGAGCAGGAG TTGCAGCTAG GGCCAGTGGG GCAGGTTGTG GGAGGGCCTG  1456
GGCCAGTGCA CGTTCCAGGG CCACATCCAC CACTTTCCCT GCTCTGTTAC  1506
ATGAGGCCCA TTCTTCACTC TGTGTTTGAA GAGAGCAGTC ACAGTTCTCA  1556
GTAGTGGGGA GCATGTTGGG TGTGAGGGAA CACAGTGTGG ACCATCTCTC  1606
AGTTCCTGTT CTATTGGGCG ATTTGGAGGT TTATCTTTGT TTCCTTTTGG  1656
AATTGTTCCA ATGTTCCTTC TAATGGATGG TGTAATGAAC TTCAACATTC  1706
ATTTTATGTA TGACAGTAGA CAGACTTACT GCTTTTTATA TAGTTTAGGA  1756
GTAAGAGTCT TGCTTTTCAT TTATACTGGG AAACCCATGT TATTTCTTGA  1806
ATTC                                                     1810
```

(2) INFORMATION FOR SEQUENCE ID NO: 21:

(i) SEQUENCE CHARACTERISTICS:

(A) LENGTH: 1412 base pairs
(B) TYPE: nucleic acid
(D) TOPOLOGY: linear

(ii) MOLECULE TYPE: genomic DNA
(ix) FEATURE:

(A) NAME/KEY: MAGE-9 gene

(xi) SEQUENCE DESCRIPTION: SEQ ID NO: 21:

```
TCTGAGACAG TGTCCTCAGG TCGCAGAGCA GAGGAGACCC AGGCAGTGTC         50
AGCAGTGAAG GTGAAGTGTT CACCCTGAAT GTGCACCAAG GGCCCCACCT        100
GCCCCAGCAC ACATGGGACC CCATAGCACC TGGCCCCATT CCCCCTACTG        150
TCACTCATAG AGCCTTGATC TCTGCAGGCT AGCTGCACGC TGAGTAGCCC        200
TCTCACTTCC TCCCTCAGGT TCTCGGGACA GGCTAACCAG GAGGACAGGA        250
GCCCCAAGAG GCCCCAGAGC AGCACTGACG AAGACCTGTA AGTCAGCCTT        300
TGTTAGAACC TCCAAGGTTC GGTTCTCAGC TGAAGTCTCT CACACACTCC        350
CTCTCTCCCC AGGCCTGTGG GTCTCCATCG CCCAGCTCCT GCCCACGCTC        400
CTGACTGCTG CCCTGACCAG AGTCATC                                 427
ATG TCT CTC GAG CAG AGG AGT CCG CAC TGC AAG CCT GAT GAA       469
GAC CTT GAA GCC CAA GGA GAG GAC TTG GGC CTG ATG GGT GCA       511
CAG GAA CCC ACA GGC GAG GAG GAG GAG ACT ACC TCC TCC TCT       553
GAC AGC AAG GAG GAG GAG GTG TCT GCT GCT GGG TCA TCA AGT       595
```

```
CCT CCC CAG AGT CCT CAG GGA GGC GCT TCC TCC TCC ATT TCC       637
GTC TAC TAC ACT TTA TGG AGC CAA TTC GAT GAG GGC TCC AGC       679
AGT CAA GAA GAG GAA GAG CCA AGC TCC TCG GTC GAC CCA GCT       721
CAG CTG GAG TTC ATG TTC CAA GAA GCA CTG AAA TTG AAG GTG       763
GCT GAG TTG GTT CAT TTC CTG CTC CAC AAA TAT CGA GTC AAG       805
GAG CCG GTC ACA AAG GCA GAA ATG CTG GAG AGC GTC ATC AAA       847
AAT TAC AAG CGC TAC TTT CCT GTG ATC TTC GGC AAA GCC TCC       889
GAG TTC ATG CAG GTG ATC TTT GGC ACT GAT GTG AAG GAG GTG       931
GAC CCC GCC GGC CAC TCC TAC ATC CTT GTC ACT GCT CTT GGC       973
CTC TCG TGC GAT AGC ATG CTG GGT GAT GGT CAT AGC ATG CCC      1015
AAG GCC GCC CTC CTG ATC ATT GTC CTG GGT GTG ATC CTA ACC      1057
AAA GAC AAC TGC GCC CCT GAA GAG GTT ATC TGG GAA GCG TTG      1099
AGT GTG ATG GGG GTG TAT GTT GGG AAG GAG CAC ATG TTC TAC      1141
GGG GAG CCC AGG AAG CTG CTC ACC CAA GAT TGG GTG CAG GAA      1183
AAC TAC CTG GAG TAC CGG CAG GTG CCC GGC AGT GAT CCT GCG      1225
CAC TAC GAG TTC CTG TGG GGT TCC AAG GCC CAC GCT GAA ACC      1267
AGC TAT GAG AAG GTC ATA AAT TAT TTG GTC ATG CTC AAT GCA      1309
AGA GAG CCC ATC TGC TAC CCA TCC CTT TAT GAA GAG GTT TTG      1351
GGA GAG GAG CAA GAG GGA GTC TGA                              1375
GCACCAGCCG CAGCCGGGGC CAAAGTTTGT GGGGTCA                     1412
```

(2) INFORMATION FOR SEQUENCE ID NO: 22:

(i) SEQUENCE CHARACTERISTICS:

(A) LENGTH: 920 base pairs
(B) TYPE: nucleic acid
(D) TOPOLOGY: linear

(ii) MOLECULE TYPE: genomic DHA
(ix) FEATURE:

(A) NAME/KEY: MAGE-10 gene

(xi) SEQUENCE DESCRIPTION: SEQ ID NO: 22:

```
ACCTGCTCCA GGACAAAGTG GACCCCACTG CATCAGCTCC ACCTACCCTA       50
CTGTCAGTCC TGGAGCCTTG GCCTCTGCCG GCTGCATCCT GAGGAGCCAT      100
CTCTCACTTC CTTCTTCAGG TTCTCAGGGG ACAGGGAGAG CAAGAGGTCA      150
AGAGCTGTGG GACACCACAG AGCAGCACTG AAGGAGAAGA CCTGTAAGTT      200
GGCCTTTGTT AGAACCTCCA GGGTGTGGTT CTCAGCTGTG GCCACTTACA      250
CCCTCCCTCT CTCCCCAGGC CTGTGGGTCC CCATCGCCCA AGTCCTGCCC      300
ACACTCCCAC CTGCTACCCT GATCAGAGTC ATC                        333
ATG CCT CGA GCT CCA AAG CGT CAG CGC TGC ATG CCT GAA GAA     375
GAT CTT CAA TCC CAA AGT GAG ACA CAG GGC CTC GAG GGT GCA     417
CAG GCT CCC CTG GCT GTG GAG GAG GAT GCT TCA TCA TCC ACT     459
TCC ACC AGC TCC TCT TTT CCA TCC TCT TTT CCC TCC TCC TCC     501
TCT TCC TCC TCC TCC TCC TGC TAT CCT CTA ATA CCA AGC ACC     543
CCA GAG GAG GTT TCT GCT GAT GAT GAG ACA CCA AAT CCT CCC     585
CAG AGT GCT CAG ATA GCC TGC TCC TCC CCC TCG GTC GTT GCT     627
TCC CTT CCA TTA GAT CAA TCT GAT GAG GGC TCC AGC AGC CAA     669
AAG GAG GAG AGT CCA AGC ACC CTA CAG GTC CTG CCA GAC AGT     711
GAG TCT TTA CCC AGA AGT GAG ATA GAT GAA AAG GTG ACT GAT     753
TTG GTG CAG TTT CTG CTC TTC AAG TAT CAA ATG AAG GAG CCG     795
ATC ACA AAG GCA GAA ATA CTG GAG AGT GTC ATA AAA AAT TAT     837
GAA GAC CAC TTC CCT TTG TTG TTT AGT GAA GCC TCC GAG TGC     879
ATG CTG CTG GTC TTT GGC ATT GAT GTA AAG GAA GTG GAT CC      920
```

(2) INFORMATION FOR SEQUENCE ID NO: 23:

(i) SEQUENCE CHARACTERISTICS:

(A) LENGTH: 1107 base pairs
(B) TYPE: nucleic acid
(D) TOPOLOGY: linear

(ii) MOLECULE TYPE: genomic DNA
(ix) FEATURE:

(A) NAME/KEY: MAGE-11 gene

(xi) SEQUENCE DESCRIPTION: SEQ ID NO: 23:

```
AGAGAACAGG CCAACCTGGA GGACAGGAGT CCCAGGAGAA CCCAGAGGAT          50
CACTGGAGGA GAACAAGTGT AAGTAGGCCT TTGTTAGATT CTCCATGGTT         100
CATATCTCAT CTGAGTCTGT TCTCACGCTC CCTCTCTCCC CAGGCTGTGG         150
GGCCCCATCA CCCAGATATT TCCCACAGTT CGGCCTGCTG ACCTAACCAG         200
AGTCATCATG CCTCTTGAGC AAAGAAGTCA GCACTGCAAG CCTGAGGAAG         250
CCTTCAGGCC CAAGAAGAAG ACCTGGGCCT GGTGGGTGCA CAGGCTCTCC         300
AAGCTGAGGA GCAGGAGGCT GCCTTCTTCT CCTCTACTCT GAATGTGGGC         350
ACTCTAGAGG AGTTGCCTGC TGCTGAGTCA CCAAGTCCTC CCCAGAGTCC         400
TCAGGAAGAG TCCTTCTCTC CCACTGCCAT GGATGCCATC TTTGGGAGCC         450
TATCTGATGA GGGCTCTGGC AGCCAAGAAA AGGAGGGGCC AAGTACCTCG         500
CCTGACCTGA TAGACCCTGA GTCCTTTTCC CAAGATATAC TACATGACAA         550
GATAATTGAT TTGGTTCATT TATTCTCCGC AAGTATCGAG TCAAGGGGCT         600
GATCACAAAG GCAGAA                                              616
ATG CTG GGG AGT GTC ATC AAA AAT TAT GAG GAC TAC TTT CCT        658
GAG ATA TTT AGG GAA GCC TCT GTA TGC ATG CAA CTG CTC TTT        700
GGC ATT GAT GTG AAG GAA GTG GAC CCC ACT AGC CAC TCC TAT        742
GTC CTT GTC ACC TCC CTC AAC CTC TCT TAT GAT GGC ATA CAG        784
TGT AAT GAG CAG AGC ATG CCC AAG TCT GGC CTC CTG ATA ATA        826
GTC CTG GGT GTA ATC TTC ATG GAG GGG AAC TGC ATC CCT GAA        868
GAG GTT ATG TGG GAA GTC CTG AGC ATT ATG GGG GTG TAT GCT        910
GGA AGG GAG CAC TTC CTC TTT GGG GAG CCC AAG AGG CTC CTT        952
ACC CAA AAT TGG GTG CAG GAA AAG TAC CTG GTG TAC CGG CAG        994
GTG CCC GGC ACT GAT CCT GCA TGC TAT GAG TTC CTG TGG GGT       1036
CCA AGG GCC CAC GCT GAG ACC AGC AAG ATG AAA GTT CTT GAG       1078
TAC ATA GCC AAT GCC AAT GGG AGG GAT CC                        1107
```

(2) INFORMATION FOR SEQUENCE ID NO: 24:

(i) SEQUENCE CHARACTERISTICS:

(A) LENGTH: 2150 base pairs
(B) TYPE: nucleic acid
(D) TOPOLOGY: linear

(ii) MOLECULE TYPE: genomic DNA
(ix) FEATURE:

(A) NAME/KEY: smage-I

(xi) SEQUENCE DESCRIPTION: SEQ ID NO: 24:

```
TCTGTCTGCA TATGCCTCCA CTTGTGTGTA GCAGTCTCAA ATGGATCTCT          50
CTCTACAGAC CTCTGTCTGT GTCTGGCACC CTAAGTGGCT TTGCATGGGC         100
ACAGGTTTCT GCCCCTGCAT GGAGCTTAAA TAGATCTTTC TCCACAGGCC         150
TATACCCCTG CATTGTAAGT TTAAGTGGCT TTATGTGGAT ACAGGTCTCT         200
GCCCTTGTAT GCAGGCCTAA GTTTTTCTGT CTGCTTAACC CCTCCAAGTG         250
AAGCTAGTGA AAGATCTAAC CCACTTTTGG AAGTCTGAAA CTAGACTTTT         300
ATGCAGTGGC CTAACAAGTT TTAATTTCTT CCACAGGGTT TGCAGAAAAG         350
AGCTTGATCC ACGAGTTCAG AAGTCCTGGT ATGTTCCTAG AAAG               394
ATG TTC TCC TGG AAA GCT TCA AAA GCC AGG TCT CCA TTA AGT        436
CCA AGG TAT TCT CTA CCT GGT AGT ACA GAG GTA CTT ACA GGT        478
TGT CAT TCT TAT CCT TCC AGA TTC CTG TCT GCC AGC TCT TTT        520
ACT TCA GCC CTG AGC ACA GTC AAC ATG CCT AGG GGT CAA AAG        565
AGT AAG ACC CGC TCC CGT GCA AAA CGA CAG CAG TCA CGC AGG        604
GAG GTT CCA GTA GTT CAG CCC ACT GCA GAG GAA GCA GGG TCT        646
TCT CCT GTT GAC CAG AGT GCT GGG TCC AGC TTC CCT GGT GGT        688
```

```
TCT GCT CCT CAG GGT GTG AAA ACC CCT GGA TCT TTT GGT GCA        730
GGT GTA TCC TGC ACA GGC TCT GGT ATA GGT GGT AGA AAT GCT        772
GCT GTC CTG CCT GAT ACA AAA AGT TCA GAT GGC ACC CAG GCA        814
GGG ACT TCC ATT CAG CAC ACA CTG AAA GAT CCT ATC ATG AGG        856
AAG GCT AGT GTG CTG ATA GAA TTC CTG CTA GAT AAA TTT AAG        898
ATG AAA GAA GCA GTT ACA AGG AGT GAA ATG CTG GCA GTA GTT        940
AAC AAG AAG TAT AAG GAG CAA TTC CCT GAG ATC CTC AGG AGA        982
ACT TCT GCA CGC CTA GAA TTA GTC TTT GGT CTT GAG TTG AAG       1024
GAA ATT GAT CCC AGC ACT CAT TCC TAT TTG CTG GTA GGC AAA       1066
CTG GGT CTT TCC ACT GAG GGA AGT TTG AGT AGT AAC TGG GGG       1108
TTG CCT AGG ACA GGT CTC CTA ATG TCT GTC CTA GGT GTG ATC       1150
TTC ATG AAG GGT AAC CGT GCC ACT GAG CAA GAG GTC TGG CAA       1192
TTT CTG CAT GGA GTG GGG GTA TAT GCT GGG AAG AAG CAC TTG       1234
ATC TTT GGC GAG CCT GAG GAG TTT ATA AGA GAT GTA GTG CGG       1276
GAA AAT TAC CTG GAG TAC CGC CAG GTA CCT GGC AGT GAT CCC       1314
CCA AGC TAT GAG TTC CTG TGG GGA CCC AGA GCC CAT GCT GAA       1360
ACA ACC AAG ATG AAA GTC CTG GAA GTT TTA GCT AAA GTC AAT       1402
GGC ACA GTC CCT AGT GCC TTC CCT AAT CTC TAC CAG TTG GCT       1444
CTT AGA GAT CAG GCA GGA GGG GTG CCA AGA AGG AGA GTT CAA       1486
GGC AAG GGT GTT CAT TCC AAG GCC CCA TCC CAA AAG TCC TCT       1528
AAC ATG TAG                                                   1537
TTGAGTCTGT TCTGTTGTGT TTGAAAAACA GTCAGGCTCC TAATCAGTAG        1587
AGAGTTCATA GCCTACCAGA ACCAACATGC ATCCATTCTT GGCCTGTTAT        1637
ACATTAGTAG AATGGAGGCT ATTTTTGTTA CTTTTCAAAT GTTTGTTTAA        1687
CTAAACAGTG CTTTTTGCCA TGCTTCTTGT TAACTGCATA AAGAGGTAAC        1737
TGTCACTTGT CAGATTAGGA CTTGTTTTGT TATTTGCAAC AAACTGGAAA        1787
ACATTATTTT GTTTTTACTA AAACATTGTG TAACATTGCA TTGGAGAAGG        1837
GATTGTCATG GCAATGTGAT ATCATACAGT GGTGAAACAA CAGTGAAGTG        1887
GGAAAGTTTA TATTGTTAAT TTTGAAAATT TTATGAGTGT GATTGCTGTA        1937
TACTTTTTTC TTTTTTGTAT AATGCTAAGT GAAATAAAGT TGGATTTGAT        1987
GACTTTACTC AAATTCATTA GAAAGTAAAT CGTAAAACTC TATTACTTTA        2037
TTATTTTCTT CAATTATGAA TTAAGCATTG GTTATCTGGA AGTTTCTCCA        2087
GTAGCACAGG ATCTAGTATG AAATGTATCT AGTATAGGCA CTGACAGTGA        2137
GTTATCAGAG TCT                                                2150
```

(2) INFORMATION FOR SEQUENCE ID NO: 25:

(i) SEQUENCE CHARACTERISTICS:

(A) LENGTH: 2099 base pairs
(B) TYPE: nucleic acid
(D) TOPOLOGY: linear

(ii) MOLECULE TYPE: genomic DNA
(ix) FEATURE:

(A) NAME/KEY: smage-II

(xi) SEQUENCE DESCRIPTION: SEQ ID NO: 25:

```
ACCTTATTGG GTCTGTCTGC ATATGCCTCC ACTTGTGTGT AGCAGTCTCA     50
AATGGATCTC TCTCTACAGA CCTCTGTCTG TGTCTGGCAC CCTAAGTGGC    100
TTTGCATGGG CACAGGTTTC TGCCCCTGCA TGGAGCTTAA ATAGATCTTT    150
CTCCACAGGC CTATACCCCT GCATTGTAAG TTTAAGTGGC TTTATGTGGA    200
TACAGGTCTC TGCCCTTGTA TGCAGGCCTA AGTTTTTCTG TCTGCTTAGC    250
CCCTCCAAGT GAAGCTAGTG AAAGATCTAA CCCACTTTTG GAAGTCTGAA    300
ACTAGACTTT TATGCAGTGG CCTAACAAGT TTTAATTTCT TCCACAGGGT    350
TTGCAGAAAA GAGCTTGATC CACGAGTTCG GAAGTCCTGG TATGTTCCTA    400
GAAAGATGTT CTCCTGGAAA GCTTCAAAAG CCAGGTCTCC ATTAAGTCCA    450
AGGTATTCTC TACCTGGTAG TACAGAGGTA CTTACAGGTT GTCATTCTTA    500
TCTTTCCAGA TTCCTGTCTG CCAGCTCTTT TACTTCAGCC CTGAGCACAG    550
TCAACATGCC TAGGGGTCAA AAGAGTAAGA CCCGCTCCCG TGCAAAACGA    600
CAGCAGTCAC GCAGGGAGGT TCCAGTAGTT CAGCCCACTG CAGAGGAAGC    650
AGGGTCTTCT CCTGTTGACC AGAGTGCTGG GTCCAGCTTC CCTGGTGGTT    700
CTGCTCCTCA GGGTGTGAAA ACCCCTGGAT CTTTTGGTGC AGGTGTATCC    750
TGCACAGGCT CTGGTATAGG TGGTAGAAAT GCTGCTGTCC TGCCTGATAC    800
AAAAAGTTCA GATGGCACCC AGGCAGGGAC TTCCATTCAG CACACACTGA    850
AAGATCCTAT CATGAGGAAG GCTAGTGTGC TGATAGAATT CCTGCTAGAT    900

AAGTTTAAGA TGAAAGAAGC AGTTACAAGG AGTGAAATGC TGGCAGTAGT    950
TAACAAGAAG TATAAGGAGC AATTCCCTGA GATCCTCAGG AGAACTTCTG   1000
CACGCCTAGA ATTAGTCTTT GGTCTTGAGT TGAAGGAAAT TGATCCCAGC   1050
ACTCATTCCT ATTTGCTGGT AGGCAAACTG GGTCTTTCCA CTGAGGGAAG   1100
TTTGAGTAGT AACTGGGGGT TGCCTAGGAC AGGTCTCCTA ATGTCTGTCC   1150
TAGGTGTGAT CTTCATGAAG GGTAACCGTG CCACTGAGCA AGAGGTCTGG   1200
CAATTTCTGC ATGGAGTGGG GGTATATGCT GGGAAGAAGC ACTTGATCTT   1250
TGGCGAGCCT GAGGAGTTTA TAAGAGATGT AGTGCGGGAA AATTACCTGG   1300
AGTACCGCCA GGTACCTGGC AGTGATCCCC CAAGCTATGA GTTCCTGTGG   1350
GGACCCAGAG CCCATGCTGA AACAACCAAG ATGAAAGTCC TGGAAGTTTT   1400
AGCTAAAGTC AATGGCACAG TCCCTAGTGC CTTCCCTAAT CTCTACCAGT   1450
TGGCTCTTAG AGATCAGGCA GGAGGGGTGC CAAGAAGGAG AGTTCAAGGC   1500
AAGGGTGTTC ATTCCAAGGC CCCATCCCAA AAGTCCTCTA ACATGTAGTT   1550
GAGTCTGTTC TGTTGTGTTT GAAAAACAGT CAGGCTCCTA ATCAGTAGAG   1600
AGTTCATAGC CTACCAGAAC CAACATGCAT CCATTCTTGG CCTGTTATAC   1650
ATTAGTAGAA TGGAGGCTAT TTTTGTTACT TTTCAAATGT TTGTTTAACT   1700
AAACAGTGCT TTTTGCCATG CTTCTTGTTA ACTGCATAAA GAGGTAACTG   1750
TCACTTGTCA GATTAGGACT TGTTTTGTTA TTTGCAACAA ACTGGAAAAC   1800
ATTATTTTGT TTTTACTAAA ACATTGTGTA ACATTGCATT GGAGAAGGGA   1850
TTGTCATGGC AATGTGATAT CATACAGTGG TGAAACAACA GTGAAGTGGG   1900
AAAGTTTATA TTGTTAGTTT TGAAAATTTT ATGAGTGTGA TTGCTGTATA   1950
CTTTTTTCTT TTTTGTATAA TGCTAAGTGA AATAAAGTTG GATTTGATGA   2000
CTTTACTCAA ATTCATTAGA AAGTAAATCA TAAAACTCTA TTACTTTATT   2050
ATTTTCTTCA ATTATTAATT AAGCATTGGT TATCTGGAAG TTTCTCCAG    2099
```

(2) INFORMATION FOR SEQUENCE ID NO: 26:

(i) SEQUENCE CHARACTERISTICS:

(A) LENGTH: 9 amino acids
(B) TYPE: amino acids
(D) TOPOLOGY: linear

(ii) MOLECULE TYPE: protein

(xi) SEQUENCE DESCRIPTION: SEQ ID NO: 26:

Glu Ala Asp Pro Thr Gly His Ser Tyr
5

(2) INFORMATION FOR SEQUENCE ID NO: 27:

(i) SEQUENCE CHARACTERISTICS:

(A) LENGTH: 19 base pairs
(B) TYPE: nucleic acid
(C) STRANDEDNESS: single
(D) TOPOLOGY: linear

(xi) SEQUENCE DESCRIPTION: SEQ ID NO: 27:

TGGAGGACCA GAGGCCCCC 19

(2) INFORMATION FOR SEQUENCE ID NO: 28:

(i) SEQUENCE CHARACTERISTICS:

(A) LENGTH: 22 base pairs
(B) TYPE: nucleic acid
(C) STRANDEDNESS: single
(D) TOPOLOGY: linear

(xi) SEQUENCE DESCRIPTION: SEQ ID NO: 28:

GGACGATTAT CAGGAGGCCT GC 22

(2) INFORMATION FOR SEQUENCE ID NO: 29:

(i) SEQUENCE CHARACTERISTICS:

(A) LENGTH: 18 base pairs
(B) TYPE: nucleic acid
(C) STRANDEDNESS: single
(D) TOPOLOGY: linear

(xi) SEQUENCE DESCRIPTION: SEQ ID NO: 29:

GAGCAGACAG GCCAACCG 18

(2) INFORMATION FOR SEQUENCE ID NO: 30:

(i) SEQUENCE CHARACTERISTICS:

(A) LENGTH: 18 base pairs
(B) TYPE: nucleic acid
(C) STRANDEDNESS: single
(D) TOPOLOGY: linear

(xi) SEQUENCE DESCRIPTION: SEQ ID NO: 30:

**AAGGACTCTG CGTCAGGC** **18**

(2) INFORMATION FOR SEQUENCE ID NO: 31:

(i) SEQUENCE CHARACTERISTICS:

(A) LENGTH: 23 base pairs
(B) TYPE: nucleic acid
(C) STRANDEDNESS: single
(D) TOPOLOGY: linear

(xi) SEQUENCE DESCRIPTION: SEQ ID NO: 31:

**CTAGAGGAGC ACCAAAGGAG AAG** **23**

(2) INFORMATION FOR SEQUENCE ID NO: 32:

(i) SEQUENCE CHARACTERISTICS:

(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO: 32:

**TGCTCGGAAC ACAGACTCTG G** **21**

(2) INFORMATION FOR SEQUENCE ID NO: 33:

(i) SEQUENCE CHARACTERISTICS:

(A) LENGTH: 19 base pairs
(B) TYPE: nucleic acid
(C) STRANDEDNESS: single
(D) TOPOLOGY: linear

(xi) SEQUENCE DESCRIPTION: SEQ ID NO: 33:

TGGAGGACCA GAGGCCCCC 19

(2) INFORMATION FOR SEQUENCE ID NO: 34:

(i) SEQUENCE CHARACTERISTICS:

(A) LENGTH: 24 baee pairs
(B) TYPE: nucleic acid
(C) STRANDEDNESS: single
(D) TOPOLOGY: linear

(xi) SEQUENCE DESCRIPTION: SEQ ID NO: 34:

CAGGATGATT ATCAGGAAGC CTGT 24

(2) INFORMATION FOR SEQUENCE ID NO: 35:

(i) SEQUENCE CHARACTERISTICS:

(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO: 35:

CAGAGGAGCA CCGAAGGAGA A 21

(2) INFORMATION FOR SEQUENCE ID NO: 36:

(i) SEQUENCE CHARACTERISTICS:

(A) LENGTH: 19 base pairs
(B) TYPE: nucleic acid
(C) STRANDEDNESS: single
(D) TOPOLOGY: linear

(xi) SEQUENCE DESCRIPTION: SEQ ID NO: 36:

CAGGTGAGCG GGGTGTGTC 19

(2) INFORMATION FOR SEQUENCE ID NO: 37:

(i) SEQUENCE CHARACTERISTICS:

(A) LENGTH: 22 base pairs
(B) TYPE: nucleic acid
(C) STRANDEDNESS: single
(D) TOPOLOGY: linear

(xi) SEQUENCE DESCRIPTION: SEQ ID NO: 37:

CCCCAGAGAA GCACTGAAGA AG 22

(2) INFORMATION FOR SEQUENCE ID NO: 38:

(i) SEQUENCE CHARACTERISTICS:

(A) LENGTH: 17 base pairs
(B) TYPE: nucleic acid
(C) STRANDEDNESS: single
(D) TOPOLOGY: linear

(xi) SEQUENCE DESCRIPTION: SEQ ID NO: 38:

GGTGAGCTGG GTCCGGG 17

(2) INFORMATION FOR SEQUENCE ID NO: 39:

(i) SEQUENCE CHARACTERISTICS:

(A) LENGTH: 19 base pairs
(B) TYPE: nucleic acid
(C) STRANDEDNESS: single
(D) TOPOLOGY: linear

(xi) SEQUENCE DESCRIPTION: SEQ ID NO: 39:

CCCCAGAGCA GCACTGACG 19

(2) INFORMATION FOR SEQUENCE ID NO: 40:

(i) SEQUENCE CHARACTERISTICS:

(A) LENGTH: 19 base pairs
(B) TYPE: nucleic acid
(C) STRANDEDNESS: single
(D) TOPOLOGY: linear

(xi) SEQUENCE DESCRIPTION: SEQ ID NO: 40:

CAGCTGAGCT GGGTCGACC 19

(2) INFORMATION FOR SEQUENCE ID NO: 41:

(i) SEQUENCE CHARACTERISTICS:

(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO: 41:

CACAGAGCAG CACTGAAGGA G 21

(2) INFORMATION FOR SEQUENCE ID NO: 42:

(i) SEQUENCE CHARACTERISTICS:

(A) LENGTH: 23 base pairs
(B) TYPE: nucleic acid
(C) STRANDEDNESS: single
(D) TOPOLOGY: linear

(xi) SEQUENCE DESCRIPTION: SEQ ID NO: 42:

CTGGGTAAAG ACTCACTGTC TGG 23

(2) INFORMATION FOR SEQUENCE ID NO: 43:

(i) SEQUENCE CHARACTERISTICS:

(A) LENGTH: 22 base pairs

(B) TYPE: nucleic acid
(C) STRANDEDNESS: single
(D) TOPOLOGY: linear

(xi) SEQUENCE DESCRIPTION: SEQ ID NO: 43:

GAGAACCCAG AGGATCACTG GA 22

(2) INFORMATION FOR SEQUENCE ID NO: 44:

(i) SEQUENCE CHARACTERISTICS:

(A) LENGTH: 23 base pairs
(B) TYPE: nucleic acid
(C) STRANDEDNESS: single
(D) TOPOLOGY: linear

(xi) SEQUENCE DESCRIPTION: SEQ ID NO: 44:

GGGAAAAGGA CTCAGGGTCT ATC 23

(2) INFORMATION FOR SEQUENCE ID NO: 45:

(i) SEQUENCE CHARACTERISTICS:

(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 45:

GGTGGAAGTG GTCCGCATCG 20

(2) INFORMATION FOR SEQUENCE ID NO: 46:

(i) SEQUENCE CHARACTERISTICS:

(A) LENGTH: 22 base pairs
(B) TYPE: nucleic acid
(C) STRANDEDNESS: single
(D) TOPOLOGY: linear

(xi) SEQUENCE DESCRIPTION: SEQ ID NO: 46:

GCCCTCCACT GATCTTTAGC AA 22

(2) INFORMATION FOR SEQ ID NO: 47:

(i) SEQUENCE CHARACTERISTICS:

(A) LENGTH: 22 base pairs
(B) TYPE nucleic acid
(C) STRANDEDNESS: single
(D) TOPOLOGY: linear

(xi) SEQUENCE DESCRIPTION: SEQ ID NO: 47:

CGGCCGAAGG AACCTGACCC AG 22

(2) INFORMATION FOR SEQ ID NO: 48:

(i) SEQUENCE CHARACTERISTICS:

(A) LENGTH: 23 base pairs
(B) TYPE: nucleic acid
(C) STRANDEDNESS: single
(D) TOPOLOGY: linear

(xi) SEQUENCE DESCRIPTION: SEQ ID NO: 48:

GCTGGAACCC TCACTGGGTT GCC 23

(2) INFORMATION FOR SEQ ID NO: 49:

(i) SEQUENCE CHARACTERISTICS:

(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 49:

AAGTAGGACC CGAGGCACTG 20

(2) INFORMATION FOR SEQ ID NO: 50:

(i) SEQUENCE CHARACTERISTICS:

(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 50:

GAAGAGGAAG AAGCGGTCTG 20

(2) INFORMATION FOR SEQ ID NO: 51:

(i) SEQUENCE CHARACTERISTICS:

(A) LENGTH: 21 base pairs
(B) TYPE: nucleic acid
(C) STRANDEDNESS: single
(D) TOPOLOGY: linear

(xi) SEQOENCE DESCRIPTION: SEQ ID NO: 51:

GGGACCAGGAGACACGGAATA 19

(2) INFORMATION FOR SEQ ID NO: 52:

(i) SEQUENCE CHARACTERISTICS:

(A) LENGTH: 17 base pairs
(B) TYPE: nucleic acid
(C) STRANDEDNESS: single
(D) TOPOLOGY: linear

(xi) SEQUENCE DESCRIPTION: SEQ ID NO: 52:

AGCCCGTCCACGCACCG 22

(2) INFORMATION FOR SEQ ID NO: 53:

(i) SEQUENCE CHARACTERISTICS:

(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 53:

ACTCAGCTCC TCCCAGATTT 20

(2) INFORMATION FOR SEQ ID NO: 54:

(i) SEQUENCE CHARACTERISTICS:

(A) LENGTH: 17 base pairs
(B) TYPE: nucleic acid
(C) STRANDEDNESS: single
(D) TOPOLOGY: linear

(xi) SEQUENCE DESCRIPTION: SEQ ID NO: 54:

GAAGAGGAGG GGCCAAG 17

(2) INFORMATION FOR SEQ ID NO: 55:

(i) SEQUENCE CHARACTERISTICS:

(A) LENGTH: 18 base pairs
(B) TYPE: nucleic acid
(C) STRANDEDNESS: single
(D) TOPOLOGY: linear

(xi) SEQUENCE DESCRIPTION: SEQ ID NO: 55:

TCTTGTATCC TGGAGTCC 18

(2) INFORMATION FOR SEQ ID NO: 56:

(i) SEQUENCE CHARACTERISTICS:

(A) LENGTH : 18 base pairs
(B) TYPE: nucleic acid
(C) STRANDEDNESS: single
(D) TOPOLOGY: linear

(xi) SEQUENCE DESCRIPTION: SEQ ID NO: 56:

TTGCCAAGAT CTCAGGAA 18

## Description

### FIELD OF THE INVENTION

This invention relates to general methods for diagnosing cancers via determining expression of at least one member of the MAGE family of tumor rejection antigen precursors. More particularly, cancers such as lung adenocarcinoma, neck, squamous cell, prostate, and bladder cancers can be diagnosed by determining expression of one or more members of this family of genes. Also a part of the invention are primers which can be used in these methods, such as amplification methods, of which the polymerase chain reaction ("PCR") is the most well known.

### BACKGROUND AND PRIOR ART

The study of the recognition or lack of recognition of cancer cells by a host organism has proceeded in many different directions. Understanding of the field presumes some understanding of both basic immunology and oncology.

Early research on mouse tumors revealed that these displayed molecules which led to rejection of tumor cells when transplanted into syngeneic animals. These molecules are "recognized" by T-cells in the recipient animal, and provoke a cytolytic T-cell response with lysis of the transplanted cells. This evidence was first obtained with tumors induced in vitro by chemical carcinogens, such as methylcholanthrene. The antigens expressed by the tumors and which elicited the T-cell response were found to be different for each tumor. See Prehn, et al., J. Natl. canc. Inst. 18: 769-778 (1957); Klein et al., Cancer Res. 20: 1561-1572 (1960); Gross, Cancer Res. 3: 326-333 (1943), Basombrio, Cancer Res. 30: 2458-2462 (1970) for general teachings on inducing tumors with chemical carcinogens and differences in cell surface antigens. This class of antigens has come to be known as "tumor specific transplantation antigens" or "TSTAS". Following the observation of the presentation of such antigens when induced by chemical carcinogens, similar results were obtained when tumors were induced in vitro via ultraviolet radiation. See Kripke, J. Natl. Canc. Inst. 53: 333-1336 (1974).

While T-cell mediated immune responses were observed for the types of tumor described supra, spontaneous tumors were thought to be generally non-immunogenic. These were therefore believed not to present antigens which provoked a response to the tumor in the tumor carrying subject. See Hewitt, et al., Brit. J. Cancer 33: 241-259 (1976).

The family of tum$^-$ antigen presenting cell lines are immunogenic variants obtained by mutagenesis of mouse tumor cells or cell lines, as described by Boon et al., J. Exp. Med. 152: 1184-1193 (1980). To elaborate, tum$^-$ antigens are obtained by mutating tumor cells which do not generate an immune response in syngeneic mice and will form tumors (i.e., "tum$^+$" cells). When these tum$^+$ cells are mutagenized, they are rejected by syngeneic mice, and fail to form tumors (thus "tum$^-$"). see Boon et al., Proc. Natl. Acad. Sci. USA 74: 272 (1977). Many tumor types have been shown to exhibit this phenomenon. See, e.g., Frost et al., Cancer Res. 43: 125 (1983).

It appear that tum$^-$ variants fail to form progressive tumors because they initiate an immune rejection process. The evidence in favor of this hypothesis includes the ability of "tum$^-$" variants of tumors, i.e., those which do not normally form tumors, to do so in mice with immune systems suppressed by sublethal irradiation, van Pel et al., Proc. Natl. Acad. Sci. USA 76: 5282-5285 (1979); and the observation that intraperitoneally injected tum$^-$ cells of mastocytoma P815 multiply exponentially for 12-15 days, and then are eliminated in only a few days in the midst of an influx of lymphocytes and macrophages (Uyttenhove et al., J. Exp. Med. 152: 1175-1183 (1980)). Further evidence includes the observation that mice acquire an immune memory which permits them to resist subsequent challenge to the same tum$^-$ variant, even when immunosuppressive amounts of radiation are administered with the following challenge of cells (Boon et al., Proc. Natl, Acad. Sci. USA 74: 272-275 (1977); Van Pel et al., supra; Uyttenhove et al., supra).

Later research found that when spontaneous tumors were subjected to mutagenesis, immunogenic variants were produced which did generate a response. Indeed, these variants were able to elicit an immune protective response against the original tumor. See Van Pel et al., J. Exp. Med. 157: 1992-2001 (1983). Thus, it has been shown that it is possible to elicit presentation of a so-called "tumor rejection antigen" in a tumor which is a target for a syngeneic rejection response. Similar results have been obtained when foreign genes have been transfected into spontaneous tumors. See Fearon et al., Cancer Res. 48: 2975-1980 (1988) in this regard.

A class of antigens has been recognized which are presented on the surface of tumor cells and are recognized by cytolytic T cells, leading to lysis. This class of antigens will be referred to as "tumor rejection antigens" or "TRAs" hereafter. TRAs may or may not elicit antibody responses. The extent to which these antigens have been studied, has been via cytolytic T cell characterization studies, in vitro i.e., the study of the identification of the antigen by a particular cytolytic T cell ("CTL" hereafter) subset. The subset proliferates upon recognition of the presented tumor rejection antigen, and the cells presenting the antigen are lysed. Characterization studies have identified CTL clones which specifically lyse cells expressing the antigens. Examples of this work may be found in Levy et al., Adv. Cancer Res. 24: 1-59 (1977); Boon et al., J. Exp. Med. 152: 1184-1193 (1980); Brunner et al., J. Immunol. 124: 1627-1634 (1980); Maryanski et al., Eur. J. Immunol. 124: 1627-1634 (1980); Maryanski et al., Eur. J. Immunol. 12: 406-412 (1982);

palladino et al., Canc. Res. 47: 5074-5079 (1987). This type of analysis is required for other types of antigens recognized by CTLs, including minor histocompatibility antigens, the male specific H-Y antigens, and the class of antigens referred to as "tum$^-$" antigens, and discussed herein.

A tumor exemplary of the subject matter described supra is known as P815. See DePlaen et al., Proc. Natl. Acad. Sci. USA 85: 2274-2278 (1988); Szikora et al., EMBO J 9: 1041-1050 (1990), and Sibille et al., J. Exp. Med. 172: 35-45 (1990).

The P815 tumor is a mastocytoma, induced in a DBA/2 mouse with methylcholanthrene and cultured as both an in vitro tumor and a cell line. The P815 line has generated many tum$^-$ variants following mutagenesis, including variants referred to as P91A (DePlaen, supra), 35B (Szikora, supra), and P198 (Sibille, supra). In contrast to tumor rejection antigens - and this is key distinction - the tum$^-$ antigens are only present after the tumor cells are mutagenized. Tumor rejection antigens are present on cells of a given tumor without mutagenesis. Hence, with reference to the literature, a cell line can be tum$^+$, such as the line referred to as "P1", and can be provoked to produce tum$^-$ variants. Since the tum$^-$ phenotype differs from that of the parent cell line, one expects a difference in the DNA of tum$^-$ cell lines as compared to their tum$^+$ parental lines, and this difference can be exploited to locate the gene of interest in tum$^-$ cells. As a result, it was found that genes of tum$^-$ variants such as P91A, 35B and P198 differ from their normal alleles by point mutations in the coding regions of the gene. See Szikora and Sibille, supra, and Lurquin et al. Cell 58: 293-303 (1989). This has proved not to be the case with the TRAs of this invention. These papers also demonstrated that peptides derived from the tum$^-$ antigen are presented by the $L^d$ molecule for recognition by CTLs. P91A is presented by $L^d$, P35 by $D^d$ and P198 by $K^d$.

PCT application US 5,925,729 filed on May 22, 1992 assigned to the same assignee as the subject application, teaches a family of human tumor rejection antigen precursor coding genes, referred to as the MAGE family, and their expression in various tumor types. Lung adenocarcinoma is not among these. Several of these genes are also discussed in van der Bruggen et al., Science 254: 1643 (1991). It is now clear that the various genes of the MAGE family are expressed in tumor cells, and can serve as markers for the diagnosis of such tumors, as well as for other purposes discussed therein. See also Traversari et al., Immunogenetics 35: 145 (1992); van der Bruggen et al., Science 254: 1643 (1991). The mechanism by which a protein is processed and presented on a cell surface has now been fairly well documented. A cursory review of the development of the field may be found in Barinaga, "Getting Some 'Backbone': How MHC Binds Peptides", Science 257: 880 (1992); also, see Fremont et al., Science 257: 919 (1992); Matsumura et al., Science 257: 927 (1992); Latron et al., Science 257: 964 (1992). These papers generally point to a requirement that the peptide which binds to an MHC/HLA molecule be nine amino acids long (a "nonapeptide"), and to the importance of the first and ninth residues of the nonapeptide.

Studies on the MAGE family of genes have now revealed that, in some cases a nonapeptide is presented on the surface of tumor cells, and that the presentation of the nonapeptide requires that the presenting molecule be HLA-Al. Complexes of the MAGE-1 tumor rejection antigen (the "TRA" or nonapeptide") leads to lysis of the cell presenting it by cytolytic T cells ("CTLs"). Additional research has correlated other nonapeptides derived from MAGE and genes to HLA-A1 and other MHC class I molecules.

Research presented in, e.g., WO 9 405 304, showed that, when comparing homologous regions of various MAGE genes to the region of the MAGE-1 gene coding for the relevant nonapeptide, there is a great deal of homology. Zakut et al. (cancer research vol. 53, no. 1, 1993, pages 5-8) describes an alternative to determine expression patterns of genes encoding MAGE 1-3. The manuscript describes the amplification by polymerase chain reaction (PCR) of a fragment common to each gene. The fragments although homologous with MAGE 1-3, do contain restriction sites which, following restriction digestion, results in a pattern of restriction fragments specific to each gene. Thus, it is possible, after PCR amplification and digestion, to identify which MAGE gene is expressed in a particular tumour tissue.

The nucleic acid sequences which code for the nonapeptides were also described therein. These nucleic acid molecules were described as also being useful as diagnostic probes for tumor presence.

The application also showed how it had been found that a cellular model could be used, wherein a non-human cell can be transfected with a nucleic acid sequence coding for a human HLA molecule. The resulting transfectant could then be used to test for nonapeptide specificity of the particular HLA molecule, or as the object of a second transfection with a MAGE gene. The co-transfectant could be used to determine whether the particular MAGE based TRA is presented by the particular HLA molecule.

Many of the references referred to supra present data on the expression pattern of various MAGE genes in different types of cell lines and tumor tissues. What is evident from these data is that there is no "unifying principle" which allows one to predict which MAGE gene will be expressed by a particular tumor type. Thus, while on one level one can say that MAGE genes are "markers" for tumors, on the level of specific tumor types, the correlation of marker and tumor type is not predictable, and must be determined empirically.

It has now been found that one can carry out cancer determination assays by assaying for expression of one or more members of the MAGE family of tumor rejection antigen precursors. How this is accomplished is shown in the examples which follow.

## BRIEF DESCRIPTION OF THE FIGURES

Figures 1A and 1B depict detection of transfectants expressing antigen P815A.

Figure 2 shows the sensitivity of clones P1.HTR, PO.HTR, genomic transfectant P1A.T2 and cosmid transfectant P1A.TC3.1 to lysis by various CTLs, as determined by chromium release assays.

Figure 3 is a restriction map of cosmid C1A.3.1.

Figure 4 shows Northern Blot analysis of expression of gene P1A.

Figure 5 sets forth the structure of gene P1A with its restriction sites.

Figure 6 shows the results obtained when cells were transfected with the gene for P1A, either isolated from P815 or normal cells and then tested with CTL lysis.

Figure 7 shows lytic studies using mast cell line L138. 8A.

Figure 8 is a map of subfragments of the 2.4 kb antigen E fragment which also express the antigen.

Figure 9 shows homology of sections of exon 3 from genes mage 1, 2 and 3.

Figure 10 shows the result of Northern blots for MAGE genes on various tissues.

Figure 11 presents the data of Figure 13 in table form.

Figure 12 shows Southern Blot experiments using the various human melanoma cell lines employed in this application.

Figure 13 is a generalized schematic of the expression of MAGE 1, 2 and 3 genes by tumor and normal tissues.

Figure 14 shows results from a chromium release assay using CTL clone 20/38 on various cell lines.

Figure 15 presents the result of assays undertaken to determine antigenic specificity of CTL clone 20/38.

Figure 16 shows the results obtained when a TNF release assay was carried out on various transfected cells.

Figure 17 shows results secured from qualitative PCR assays for MAGE-1 in lung adenocarcinomas.

Figure 18 presents data pertaining to quantitative measurement of MAGE-1 expression in lung adenocarcinomas.

Figure 19 shows reverse transcription/PCR amplification production of mRNA extracted from the bladder tumor of a patient referred to as "HM15". This is shown in all lanes marked "R". In lanes marked "D"', amplification products of the genomic DNA from the patient are shown.

Figure 20 displays the fraction of tumors expressing genes MAGE-1, 2, 3 and 4 among the superficial and invasive transitional cell carcinomas of the bladder.

## DETAILED DESCRIPTION OF PREFERRED EMBODIMENTS

Many different "MAGE" genes have been identified, as will be seen from the sequences which follow the application. The protocols described in the following examples were used to isolate these genes and cDNA sequences.

"MAGE" as used herein refers to a nucleic acid sequence isolate from human cells. The acronym "smage" is used to describe sequences of murine origin.

When "TRAP" or "TRAs" are discussed herein as being specific to a tumor type, this means that the molecule under consideration is associated with that type of tumor, although not necessarily to the exclusion of other tumor types.

## Example 1

In order to identify and isolate the gene coding for antigen P815A, gene transfection was used. This approach requires both a source of the gene, and a recipient cell line. Highly transfectable cell line P1.HTR was the starting material for the recipient, but it could not be used without further treatment, as it presents "antigen A", one of four recognized P815 tumor antigens. See Van Pel et al., Molecular Genetics 11: 467-475 (1985). Thus, screening experiments were carried out to isolate cell lines which did not express the antigen and which nonetheless possessed P1.HTR's desirable qualities.

To do this, P1.HTR was screened with CTLs which were specific for each of tumor antigens A, B, C and D. Such CTLs are described by Uyttenhove et al., J. Exp. Med. 157: 1040-1052 (1983).

To carry out the selection, $10^6$ cells of P1.HTR were mixed with 2-4x$10^6$ cells of the CTL clone in a round bottom tube in 2 ml of medium, and centrifuged for three minutes at 150xg. After four hours at 37°C, the cells were washed and resuspended in 10 ml of medium, following Maryanski et al., Eur. J. Immunol. 12: 406-412 (1982). Additional information on the CTL assay and screening protocol, in general may be found in Boon et al., J. Exp. Med. 152: 1184-1193 (1980), and Maryanski et al., Eur. J. Inmunol. 12: 406-412 (1982).

When these selections were carried out, a cell line variant was found which expressed neither antigen A or B. Additional selections with CTLs specific for antigen C then yielded a variant which also lacked antigen C. Please see figure 2 for a summary of the results of these screenings. The variant PO.HTR is negative for antigens A, B and C, and was therefore chosen for the transfection experiments.

The cell line PO.HTR has been deposited in accordance with the Budapest Treaty at the Institute Pasteur Collection Nationale De cultures De Microorganismes, 28, Rue de Docteur Roux, 75724 Paris France, and has accession number I-1117.

This methodology is adaptable to secure other cell lines which are variants of a cell type which normally presents at least one of the four recognized P815 tumor antigens, i.e., antigens A, B, C and D, where the variants present none of antigens A, B and C. P1.HTR is a mastocytoma cell line, so it will be seen that the protocol enables the isolation of biologically pure mastocytoma cell lines which express none of P815 antigens A, B and C, but which are highly transfectable. Other tumor types may also be screened in this fashion to secure desired, biologically pure cell lines. The resulting cell lines should be at least as transfectable with foreign DNA as is P1.HTR, and should be selected so as to not express a specific antigen.

**Example 2**

Previous work reported by DePlaen et al., Proc. Natl. Acad. Sci. USA 85: 2274-2278 (1988) had shown the efficacy of using cosmid library transfection to recover genes coding for tum$^-$ antigens.

Selective plasmid and genomic DNAs of P1.HTR were prepared, following Wölfel et al., Immunogenetics 26: 178-187 (1987). The transfection procedure followed Corsaro et al., Somatic Cell Molec. Genet 7: 603-616 (1981), with some modifications. Briefly, 60 µg of cellular DNA and 3 µg of DNA of plasmid pHMR272, described by Bernard et al., Exp. Cell. Biol. 158: 237-243 (1985) were mixed. This plasmid confers hygromycin resistance upon recipient cells, and therefore provides a convenient way to screen for transfectants. The mixed DNA was combined with 940 ul of 1 mM Tris-HCl (pH 7.5), 0.1 mM EDTA, and 310 ul 1M $CaCl_2$. The solution was added slowly, and under constant agitation to 1.25 ml of 50 mM Hepes, 280 mM NaCl, 1.5 mM $Na_2HPO_4$, adjusted to pH 7.1 with NaOH. Calcium phosphate - DNA precipitates were allowed to form for 30-45 minutes at room temperature. Following this, fifteen groups of PO.HTR cells ($5x10^6$ per group) were centrifuged for 10 minutes at 400xg. Supernatants were removed, and pellets were resuspended directly into the medium containing the DNA precipitates. This mixture was incubated for 20 minutes at 37°C, after which it was added to an 80 $cm^2$ tissue culture flask containing 22.5 ml DMEM, supplemented with 10% fetal calf serum. After 24 hours, medium was replaced. Forty-eight hours after transfection, cells were collected and counted. Transfected cells were selected in mass culture using culture medium supplemented with hygromycin B (350 ug/ml). This treatment selected cells for hygromycin resistance.

For each group, two flasks were prepared, each containing $8x10^6$ cells in 40 ml of medium. In order to estimate the number of transfectants, $1x10^6$ cells from each group were plated in 5 ml DMEM with 10% fetal calf serum (FCS), 0.4% bactoagar, and 300 ug/ml hygromycin B. The colonies were then counted 12 days later. Two independent determinations were carried out and the average taken. This was multiplied by 5 to estimate the number of transfectants in the corresponding group. Correction had to be made for the cloning efficiency of P815 cells, known to be about 0.3.

**Example 3**

Eight days after transfection as described in example 2, supra, antibiotic resistant transfectants were separated from dead cells, using density centrifugation with Ficoll-Paque. These cells were maintained in non-selective medium for 1 or 2 days. The cells were plated in 96 well microplates (round bottom), at 30 cells/microwell in 200 ul of culture medium. Anywhere from 100-400 microwells were prepared, depending on the number of transfectants prepared. Agar colony tests gave estimates of 500-3000. After 5 days, the wells contained about $6x10^4$ cells and replicate plates were prepared by transferring 1/10 of the wells to microplates which were then incubated at 30°C. One day later, master plates were centrifuged, medium removed, and 750 CTLs against P815 antigen A (CTL·P1:5) were added to each well together with $10^6$ irradiated syngeneic feeder spleen cells in CTL culture medium containing 40 U/ml recombinant human IL-2, and HAT medium to kill stimulator cells. Six days later, plates were examined visually to identify wells where CTLs had proliferated. Where plates showed proliferating microcultures, aliquots of 100 ul of the wells were transferred to another plate containing $^{51}$Cr labeled P1.HTR target cells ($2x10^3$ - $4x10^3$ per well), and chromium release was measured after 4 hours. Replicate microcultures corresponding to those showing high CTL activity were expanded and cloned by limited dilution in DMEM with 10% FCS. Five days later, about 200 clones were collected and screened with the CTL.P1:5 cell line, described supra, in a visual lysis assay. See figure 1A for these results.

In these experiments, three of the fifteen groups of transfectants yielded a few positive microcultures. These microcultures were tested for lytic activity against P1.HTR, as described supra. Most of the microcultures where proliferation was observed showed lytic activity. This activity was well above background, as shown in figure 1B. This figure summarizes data wherein two groups of cells (groups "5" and "14"), 400 and 300 microwells were seeded with 30 hygromycin resistant transfected cells. Amplification and duplication of the microcultures was followed by addition of anti-A CTL P1:5. Six days later, lytic activity against P1.HTR was tested. In the figure, each point represents lytic activity of a single microculture.

Duplicate microcultures corresponding to several positive veils were subcloned, and more than 1% of the subclones were found to be lysed by anti-A CTL. Thus, three independent transfectants expressing P815A were obtained from 33,000 hygromycin resistant transfectants. One of these lines, referred to hereafter as P1A.T2 was tested further.

The relevant antigen profile of P1A.T2 is shown in figure 2, this being obtained via anti-CTL assays of the type described supra.

**Example 4**

The CTL assays carried out for P1A.T2 demonstrated that it presented antigen A ("P815A"), and therefore had received the gene from P1.HTR. To that end, this cell line was used as a source for the gene for the antigen precursor in the following experiments.

Prior work had shown that genes coding for tum antigens could be recovered directly from transfectants obtained with a cosmid library. See DePlaen et al., Proc. Natl. Acad. Sci. USA 85: 2274-2278 (1988). This procedure was followed for recovery of the P815 gene.

Total genomic DNA of P1A.T2 was partially digested with restriction endonuclease Sau 3A1, and fractionated by NaCl density gradient ultracentrifugation to enrich for 35-50 kb DNA fragments, following Grosveld et al., Gene 10: 6715-6732 (1982). These fragments were ligated to cosmid arms of C2RB, described by Bates et al., Gene 26: 137-146 (1983). These cosmid arms had been obtained by cleavage with SmaI and treatment with calf intestinal phosphatase, followed by digestion with BamHI. Ligated DNA was packaged into lambda phage components, and titrated on E. coli ED 8767, following Grosveld et al., supra. Approximately $9x10^5$ ampicillin resistant colonies were obtained per microgram of DNA insert.

The cosmid groups were amplified by mixing 30,000 independent cosmids with 2 ml of ED 8767 in 10 mM $MgCl_2$, incubated 20 minutes at 37°C, diluted with 20 ml of Luria Bertani ("LB") medium, followed by incubation for one hour. This suspension was titrated and used to inoculate 1 liter of LB medium in the presence of ampicillin (50 ug/ml). At a bacterial concentration of $2x10^8$ cells/ml ($OD_{600}$=0.8), a 10 ml aliquot was frozen, and 200 ug/ml chloramphenicol was added to the culture for overnight incubation. Total cosmid DNA was isolated by alkaline lysis procedure, and purified on CsCl gradient.

In these experiments, a library of 650,000 cosmids was prepared. The amplification protocol involved the use of 21 groups of approximately 30,000 cosmids.

**Example 5**

Using the twenty-one groups of cosmids alluded to supra, (60 ug) and 4 ug of pHMR272, described supra, groups of $5x10^6$ PO.HTR cells were used as transfectant hosts. Transfection was carried out in the same manner as described in the preceding experiments. An average of 3000 transfectants per group were tested for antigen presentation, again using CTL assays as described. One group of cosmids repeatedly yielded positive transfectants, at a frequency of about 1/5,000 drug resistant transfectants. The transfectants, as with P1A.T2, also showed expression of both antigen A and B. The pattern of expression of transfectant P1A.TC3.1 is shown in figure 2,

**Example 6**

As indicated in Example 5, supra, three independent cosmid transfected cells presenting P815A antigen were isolated. The DNA of these transfectants was isolated and packaged directly with lambda phage extracts, following DePlaen et al., Proc. Natl. Acad. Sci. USA 85: 2274-2278 (1988). The resulting product was titrated on E. coli ED 8767 with ampicillin selection, as in Example 5. Similarly, amplification of the cosmids and transfection followed Example 5, again using PO.HTR.

High frequencies of transfection were observed, as described in Table 1, which follows:

Table 1.

| Transfer of the expression of antigen P815A by cosmids obtained by direct packaging | | |
|---|---|---|
| Transfectant obtained with the cosmid library | No. of cosmids obtained by direct packaging of 0.5 μg of DNA | No. of transfectants expressing P815A / no. of HmB' transfectants |
| TC3.1 | 32 | 87/192 |
| TC3.2 | 32000 | 49/384 |
| TC3.3 | 44 | 25/72 |

The cosmids were analyzed with restriction enzymes and it was found that directly packaged transfectant P1A.TC3.1 contained 32 cosmids, 7 of which were different. Each of these 7 cosmids was transfected into PO.HTR, in the manner described supra, and again, following the protocols described above, transfectants were studied for presentation of P815A. Four of the cosmid transfectants showed P815A presentation and, as with all experiments described herein, P815B was co-expressed.

Of the four cosmids showing presentation of the two antigens, cosmid C1A.3.1 was only 16.7 kilobases long, and was selected for further analysis as described infra.

The cosmid C1A.3.1 was subjected to restriction endonuclease analysis, yielding the map shown in Figure 3.

All EcoRI fragments were transfected, again using the above described protocols, and only the 7.4 kilobase fragment produced a transfectant that anti-A CTLs could lyse. Similar experiments were carried out on the PstI fragments, and only a 4.1 kb fragment fully contained within the 7.4 kb EcoRI fragment produced lysable transfectants.

This fragment (i.e., the 4.1 kb PstI fragment), was digested with SmaI, giving a 2.3 kb fragment which also yielded host cells presenting antigens A and B after transfection. Finally, a fragment 900 bases long, secured with SmaI/XbaI, also transferred expression of the precursors of these two antigens, i.e., the transfected host cell presented both antigen A and antigen B.

## Example 7

The 900 base fragment described above was used as a probe to detect the expression of the P815A gene in parent cell line P1.HTR. To accomplish this, total cellular RNA was first isolated using the guanidine-isothiocyanate procedure of Davis et al., Basic Methods In Molecular Biology (Elsevier Science Publishing Co, New York) (1986). The same reference was the source of the method used to isolate and purify polyA$^+$ mRNA using oligodT cellulose column chromatography.

Samples were then subjected to Northern Blot analysis. RNA samples were fractionated on 1% agarose gels containing 0.66 M formaldehyde. The gels were treated with 10xSSC (SSC: 0.15 M NaCl; 0.015 M sodium citrate, pH 7.0) for 30 minutes before overnight blotting on nitrocellulose membranes. These were baked for two hours at 80°C, after which the membranes were prehybridized for 15 minutes at 60°C in a solution containing 10% dextran sulfate, 1% SDS and 1M NaCl. Hybridization was then carried out using denatured probe (the 900 base fragment), together with 100 ug/ml salmon sperm DNA.

When this protocol was carried out using P1.HTR poly A$^+$ RNA, a band of 1.2 kb and two fainter bands were identified, as shown in Figure 4, lane 1 (6 ug of the RNA).

The same probe was used to screen a cDNA library, prepared from poly-A$^+$ RNA from the cell line. This yielded a clone with a 1kb insert, suggesting a missing 5' end. The Northern blots for the cDNA are not shown.

Hybridization experiments in each case were carried out overnight at 60°C. The blots were washed twice at room temperature with 2xSSC and twice at 60°C with 2xSSC supplemented with 1% SDS.

The foregoing experiments delineated the DNA expressing the P815A antigen precursor sufficiently to allow sequencing, using the well known Sanger dideoxy chain termination method. This was carried out on clones generated using a variety of restriction endonucleases and by specific priming with synthetic oligonucleotide primers. The results for exons of the gene are set forth in SEQUENCE ID NO: 4.

## Example 8

The Northern analysis described supra suggested that the 5' end of the cDNA was missing. To obtain this sequence, cDNA was prepared from P1.HTR RNA using a primer corresponding to positions 320-303. The sequence was then amplified using the polymerase chain reaction using a 3' primer corresponding to positions 286-266 and a 5' primer described by Frohman et al., Proc. Natl. Acad. Sci. USA 85: 8998-9002 (1988). A band of the expected size (270 bases) was found, which hybridized to the 900 bp SmaI/XbaI fragment described supra on a Southern blot. Following cloning into m13tg 130 and tg 131, the small, 270 bp fragment was sequenced. The sequence is shown in SEQUENCE ID NO: 1.

## Example 9

Following the procurement of the sequences described in Examples 7 and 8 and depicted in SEQ ID NO: 4, a 5.7 kb region of cosmid C1A.3.1 was sequenced. This fragment was known to contain the 900 base fragment which expressed P815A in transfectants. This experiment permitted delineation of introns and exons, since the cosmid is genomic in origin.

The delineated structure of the gene is shown in figure 5. Together with SEQ ID NO: 4, these data show that the gene for the antigen precursor, referred to as "P1A" hereafter, is approximately 5 kilobases long and contains 3 exons. An ORF for a protein of 224 amino acids starts in exon 1, ending in exon 2. The 900 base pair fragment which transfers expression of precursors for antigens A and B only contains exon 1. The promoter region contains a CAAT box, as indicated in SEQ ID NO: 1, and an enhancer sequence. This latter feature has been observed in promoters of most MHC class I genes, as observed by Geraghty et al., J. Exp. Med 171: 1-18 (1990); Kimura et al., Cell 44: 261-272 (1986).

A computer homology search was carried out, using program FASTA with K-triple parameters of 3 and 6, as suggested by Lipman et al., Science 227: 1435-1441 (1985), and using Genbank database release 65 (October 1990). No homology was found except for a stretch of 95 bases corresponding to part of an acid region coded by exon 1 (positions 524-618), which is similar to sequences coding for acidic regions in mouse nucleolar protein NO38/B23, as described by Bourbon et al., Mol. Biol. 200: 627-638 (1988), and Schmidt-Zachmann et al., Chromosoma 96: 417-426 (1988). Fifty six of 95 bases were identical. In order to test whether these homologies were the reason for cross hybridizing, experiments were carried out using a mouse spleen cDNA library screened with the 900 base fragment. cDNA clones corresponding closely to the sizes of the cross hybridizing bands were obtained. These were partially sequenced, and the 2.6 kb cDNA was found to correspond exactly to reported cDNA sequence of mouse nucleolin, while the 1.5 kb cDNA corresponded to mouse nucleolar protein NO38/B23.

Analysis of the nucleotide sequence of the gene, referred to as "P1A" hereafter, suggests that its coded product has a molecular mass of 25 kd. Analysis of the SEQUENCE ID NO: 4 shows a potential nuclear targeting signal at residues 5-9 (Dingwall et al., Ann. Rev. Cell Biol. 2: 367-390 (1986)), as well as a large acidic domain at positions 83-118. As indicated supra, this contains the region of homology between P1A and the two nucleolar proteins. A putative phosphorylation site can be found at position 125 (serine). Also, a second acidic domain is found close to the C-terminus as an uninterrupted stretch of 14 glutamate residues. A similar C-terminal structure has been found by Kessel et al. Proc. Natl. Acad. Sci. USA 84: 5306-5310 (1987), in a murine homeodomain protein having nuclear localization.

In studies comparing the sequence of gene P1A to the sequences for P91A, P35B and P198, no similarities were found, showing that P1A is indicative of a different class of genes and antigens.

## Example 10

With the P1A probe and sequence in hand, investigations were carried out to determine whether the gene present in normal tissue was identical to that expressed by the tumor. To do this, phage libraries were prepared, using lambda zapII 10 and genomic DNA of DBA/2 murine kidney cells. P1A was used as a probe. Hybridization conditions were as described supra, and a hybridizing clone was found. The clone contained exons one and two of the P1A gene, and corresponded to positions -0.7 to 3.8 of figure 5. Following localization of this sequence, PCR amplification was carried out to obtain the sequence corresponding to 3.8 to 4.5 of figure 5.

Sequence analysis was carried out, and no differences were found between the gene from normal kidneys and the P1A gene as obtained from the P815 tumor cells.

In further experiments, the gene as found in DBA/2 kidney cells was transfected into PO.HTR, as described supra. These experiments, presented pictorially in figure 6, showed that antigens A and B were expressed as efficiently by the kidney gene isolated from normal P815 cells as with the P1A gene isolated from normal kidney cells.

These experiments lead to the conclusion that the gene coding for the tumor rejection antigen precursor is a gene that does not result from a mutation; rather, it would appear that the gene is the same as one present in normal cells, but is not expressed therein. The ramifications of this finding are important, and are discussed infra.

In studies not elaborated upon herein, it was found that variants of the gene were available. Some cells were "$P1A^-B^+$", rather than the normal "P1A". The only difference between these is a point mutation in exon 1, with the 18th triplet coding for Ala in the variant instead of Val.

## Example 11

Additional experiments were carried out with other cell types. Following the protocols described for Northern blot hybridizations supra, RNA of normal liver and spleen cells was tested to determine if a transcript of the P1A gene could be found. The Northern blot data are presented in figure 4 and, as can be seen, there is no evidence of expression.

The murine P815 cell line from which P1A was isolated is a mastocytoma. Therefore, mast cell lines were studied to determine if they expressed the gene. Mast cell line MC/9, described by Nabel et al., Cell 23: 19-28 (1981), and short term cultures of bone marrow derived mast cells were tested in the manner described supra (Northern blotting) but no transcript was found. In contrast when a BALB/C derived IL-3 dependent cell line L138.8A (Hültner et al., J. Immunol. 142: 3440-3446 (1989)) was tested, a strong signal was found. The mast cell work is shown in figure 4.

It is known that both BALB/C and DBA/2 mice share $H-2^d$ haplotype, and thus it was possible to test sensitivity to lysis using the CTLs described supra. Figure 7 shows these results, which essentially prove that anti-A and anti-B CTLs lysed the cells strongly, whereas anti-C and anti-D lines did not.

Further tests were carried out on other murine tumor cell lines, i.e., teratocarcinoma cell line PCC4 (Boon et al., Proc. Natl. Acad. Sci. USA 74: 272-275 (1977), and leukemias LEC and WEHI-3B. Expression could not be detected in any of these samples.

**Example 12**

The actual presentation of the P1A antigen by MHC molecules was of interest. To test this, cosmid C1A.3.1 was transfected into fibroblast cell line DAP, which shows phenotype H-$2^k$. The cell lines were transfected with genes expressing one of the $K^d$, $D^d$, and $L^d$ antigen. Following transfection with both the cosmid and the MHC gene, lysis with CTLs was studied, again as described supra. These studies, summarized in Table 2, show that $L^d$ is required for presentation of the P1A antigens A and B.

Table 2

| H-2-restriction of antigens P815A and P815B | | |
|---|---|---|
| Recipient cell* | No. of clones lysed HmB' clones* | by the CTL/no. of |
| | CTL anti-A | CTL anti-B |
| DAP (H-$2^k$) | 0/208 | 0/194 |
| DAP + $K^d$ | 0/165 | 0/162 |
| DAP + $D^d$ | 0/157 | 0/129 |
| DAP + $L^d$ | 25/33 | 15/20 |

*Cosmid C1A.3.1 containing the entire P1A gene was transfected in DAP cells previously transfected with H-$2^d$ class I genes as indicated.
*Independent drug-resistant colonies were tested by lysis by anti-A or anti-B CTL in a visual assay.

The observation that one may associate presentation of a tumor rejection antigen with a particular MHC molecule was confirmed in experiments with human cells and HLA molecules, as elaborated upon infra.

**Example 13**

Using the sequence of the P1A gene as well as the amino acid sequence derivable therefrom, antigenic peptides which were $A^+ B^+$ (i.e., characteristic of cells which express both the A and B antigens), and those which are $A^- B^+$ were identified. The peptide is presented in SEQ ID NO: 26. This peptide when administered to samples of PO. HTR cells in the presence of CTL cell lines specific to cells presenting it, led to lysis of the PO.HTR cells, lending support to the view that peptides based on the product expressed by the gene can be used as vaccines.

**Example 14**

The human melanoma cell line referred to hereafter as MZ2-MEL is not a clonal cell line. It expresses four stable antigens recognized by autologous CTLs, known as antigens "D, E, F, and A". In addition, two other antigens "B" and "C" are expressed by some sublines of the tumor. CTL clones specific for these six antigens are described by Van den Eynde et al., Int. J. Canc. 44: 634-640 (1989). Among the recognized subclones of MZ2-MEL are MEL.43, MEL3.0 and MEL3.1. (Van den Eynde et al., supra). Cell line MEL3.1 expresses antigen E, as determined by CTL studies as described for P815 variants, supra, so it was chosen as a source for the nucleic acid sequence expressing the antigen precursor.

In isolating the pertinent nucleic acid molecule for a tumor rejection antigen precursor, the techniques developed supra, showed that a recipient cell is needed which fulfills two criteria: (i) the recipient cell must not express the TRAP of interest under normal conditions, and (ii) it must express the relevant class I HLA molecule. Also, the recipient cell must have a high transfection frequency, i.e., it must be a "good" recipient.

In order to secure such a cell line, the clonal subline MEL3.1 was subjected to repeated selection with anti-E CTL 82/30 as described by Van den Eynde, supra. The repeated cycles of selection led to isolation of subclone MZ2-MEL-2.2 isc $E^-$. This subclone is also $HPRT^-$, (i.e., sensitive to HAT medium: $10^{-4}$ M hypoxanthine, 3.8 x $10^{-7}$ aminopterine, 1.6 x $10^{-5}$ M 2-deoxythymidine). The subclone is referred to as "MEL-2.2" for simplicity hereafter.

**Example 15**

The genomic DNA of MEL3.0 was prepared following Wölfel et al., Immunogenetics 26: 178-187 (1987).

The plasmid pSVtkneoß, as described by Nicolas et al., Cold Spring Harb., Conf. Cell Prolif. 10: 469-485 (1983) confers geneticin resistance, so it can be used as a marker for cotransfection, as it was in this experiment.

Following a procedure similar but not identical to that of Corsaro et al., Somatic Cell Molec. Genet 7: 603-616 (1981), total genomic DNA and the plasmid were cotransfected. The genomic DNA (60 µg) and plasmid DNA (6 µg) were mixed in 940 µl of 1 mM Tris·HCl (pH 7.5), 0.1 mM EDTA, after which 310 µl of 1M $CaCl_2$ was added. This solution was slowly added, under constant agitation, to 1.25 ml of 2xHBS (50 mM HEPES, 280 mM NaCl 1.5 mM $Na_2HPO_4$, adjusted to pH 7.1 with NaOH). The calcium phosphate DNA precipitates were allowed to form for 30-45 minutes at room temperature, after which they were applied to 80 $cm^2$ tissue culture flasks which had been seeded 24 hours previously with $3x10^6$ MEL2.2 cells, in 22.5 ml of melanoma culture medium (Dulbecco's Modified Eagle's Medium) supplemented with 10% fetal calf serum. After 24 hours, the medium was replaced. Forty eight hours after transfection, the cells were harvested and seeded at $4x10^6$ cells per 80 $cm^2$ flask in melanoma culture medium supplemented with 2 mg/ml of geneticin. The geneticin serves as a selection marker.

## Example 16

Thirteen days after transfection, geneticin-resistant colonies were counted, harvested, and cultured in non-selective medium for 2 or 3 days. Transfected cells were then plated in 96-well microplates at 200 cells/well in 200 ul of culture medium with 20% fetal calf serum (FCS) in order to obtain approximately 30 growing colonies per well. The number of microcultures was aimed at achieving redundancy, i.e., such that every independent transfectant should be represented at least four times.

After 10 days, wells contained approximately $6x10^4$ cells. These cells were detached, and 1/3 of each microculture was transferred to a duplicate plate. After 6 hours, i.e., after readherence, medium was removed and 1500 anti-E CTL (CTL 82/30), were added to each well in 100 µl of CTL culture medium with 35 U/ml of IL-2. One day later, the supernatant (50 µl) was harvested and examined for TNF concentration, for reasons set forth in the following example.

## Example 17

The size of the mammalian genome is $6x10^6$ kb. As the average amount of DNA integrated in each drug-resistant transfectant was expected to be about 200 kb, a minimum of 30,000 transfectants would need to be examined to ascertain whether antigen E had been transfected. Prior work with murine cells had shown that when a CTL stimulation assay was used, groups containing only 3% of cells expressing the antigen of interested could be identified. This should reduce the number of assays by a factor of 30. While an anti-E CTL assay, as described supra, in mixed $E^+/E^-$ cells was helpful, it was not sufficient in that consistent results could not be obtained.

As a result, an alternative test was devised. Stimulation of CTLs was studied by release of tumor necrosis factor ("TNF") using well known methodologies which need not be repeated here. As described in Example 15, 1500 CTL 82/30 cells had been added per well of transfectants. These CTLs were collected 6 days after stimulation. As indicated supra, after 1/3 of the cells in each well had been removed and the remaining 2/3 ($4x10^4$) had readhered, the CTLs and IL-2 were added thereto. The 50 µl of supernatant was removed 24 hours later and transferred to a microplate containing $3x10^4$ W13 (WEHI-164 clone 13; Espevik et al., J. Immunol. Meth. 95: 99-105 (1986)) cells in 50 µl of W13 culture medium (RPMI-1640, supplemented with L-arginine (116 mg/l), L-asparagine (36 mg/l), L-glutamine (216 mg/l), and 10% FCS supplemented with 2 µg of actinomycin D at 37°C in an 8% $CO_2$ atmosphere. The cell line W13 is a mouse fibrosarcoma cell line sensitive to TNF. Dilutions of recombinant TNF-β in RPMI 1640 were added to target cell controls.

The W13 cultures were evaluated after 20 hours of incubation, and dead cell percentage was measured using an adaptation of the colorimetric assay of Hansen et al., J. Immunol. Meth. 119: 203-210 (1989). This involved adding 50 ml of (3-(4,5-dimethylthiazol-2-yl)-2,5-diphenyl tetrazolium bromide at 2.5 mg/ml in PBS, followed by two hours of incubation at 37°C. Dark blue formazan crystals were dissolved by adding 100 µl of lysis solution (1 volume N,N dimethyl formamide mixed at 37°C with two volumes of water containing 30% (w/v) sodium dodecyl sulphate, at pH 4.7 from 1.6% acetic acid and 2.5% 1N HCl). Plates were incubated at 37°C overnight, and ODs were taken at 570 nm using 650 nm as control. Dead cell percentage was determined via the formula:

$$100 \times 1 - \frac{100\text{-}(OD_{570}\text{ sample well})}{OD_{570}\text{ well + medium}}$$

following Espevik et al., J. Immunol. Meth. 95: 99-105 (1986). The results showed that even when the ratio of $E^+/E^-$ cells was as low as 1/45, significant production of TNF was observed, thus showing active CTLs. This led to the decision to test the drug resistant transfectants in groups of 30.

**Example 18**

Cells were tested for TNF production as discussed in Example 17, supra. A total of 100 groups of $E^-$ cells ($4x10^6$ cells/group) were tested following transfection, and $7x10^4$ independent geneticin resistant transfectants were obtained, for an average of 700 per group. Only one group of transfected cells led to a microculture which caused anti-E antigen CTL clone 82/30 to produce TNF. Of 300 clones tested, 8 were positive. These clones were then tested for lysis by anti-E CTL, using the standard $^{51}Cr$ release assay, and were found to be lysed as efficiently as the original $E^+$ cell line. The transfectant E.T1, discussed herein, had the same lysis pattern as did MEL2.2 for CTLs against antigens B,C,D and F.

The fact that only one transfectant presented the antigen out of 70,000 geneticin resistance transfectants may at first seem very low, but it is not. The work described supra for P815 showed an average frequency of 1/13,000. Human DNA recipient MEL2.2 appears to integrate 5 times less DNA than P1.HTR.

**Example 19**

Once transfectant E.T1 was found, analysis had to address several questions including whether an $E^+$ contaminant of the cell population was the cause. The analysis of antigen presentation, described supra, shows that E. T1 is $B^-$ and $C^-$, just like the recipient cell MEL2.2. It was also found to be $HPRT^-$, using standard selection procedures. All $E^+$ cells used in the work described herein, however, were $HPRT^+$.

It was also possible that an $E^+$ revertant of MEL2.2 was the source for E.T1. To test this, the observation by Perucho et al., Cell 22: 309-317 (1980), that cotransfected sequences usually integrate together at a single location of recipient genome was employed. If antigen E in a transfectant results from cotransfec-tion with pSVtkneoß, then sequences should be linked and deletion of the antigen might also delete the neighboring pSVtkneoß sequences. Wölfel et al., supra, has shown this to be true. If a normally E cell is transfected with pSVtkneoß, then sequences should be linked and deletion of the antigen might also delete the neighboring pSVtkneoß sequences. If a normally $E^+$ cell transfected with pSVtkneoß is E.T1, however, "co-deletion" should not take place. To test this, the transfectant E. T1 was subjected to immunoselection with 82/30, as described supra. Two antigen loss variants were obtained, which resisted lysis by this CTL. Neither of these had lost geneticin resistance; however, Southern blot analysis showed loss of several $neo^r$ sequences in the variants, showing close linkage between the E gene and $neo^r$ gene in E.T1, leading to the conclusion that E.T1 was a transfectant.

**Example 20**

The $E^+$ subclone MZ2-MEL 43 was used as a source of DNA for preparation of a cosmid library. This library of nearly 700,000 cosmids was transfected into MZ2-MEL 2.2 cells, following the cosmid transfection protocols described supra.

By packaging the DNA of cosmid transfectants directly into lambda phage components, it is sometimes possible to retrieve cosmids that contain the sequences of interest. This procedure was unsuccessful here, so rescue of the transfected sequence was accomplished by ligating DNA of the transfectant to appropriate restriction fragments of cosmid vector pTL6. This was tried with two transfectants and was successful with one of them. One cosmid, referred to as B3, was recovered from this experiment, and subjected to restriction endonuclease digestion via XmaI, or by BamHI digestion of a large, 12 kb XmaI transfected fragment. The fragments were cloned into vector pTZ 18R, and then transfected into MEL2.2. Again, TNF production was the measure by which successful transfection was determined. The experiments led to the determination of a gene sequence capable of transfecting antigen E on the 12 kb XmaI fragment, and then on the 2.4 kb fragment of BamHI digestion of the 12 kb segment.

The 2.4 kb fragment hybridizes with a 2.4 kb fragment from MZ2-MEL and with a T cell clone of patient MZ-2, as determined by Southern Blots (BamHI digested DMA). The band is absent from $E^-$ antigen loss variants of MZ2-MEL, as seen in SEQ ID NO: 7.

**Example 21**

After the 2.4 kb genomic segment had been identified, studies were carried out to determine if an "$E^+$" subline expressed any homologous DNA. Cell line MZ2-MEL 3.0 was used as a source, and a cDNA library was prepared from its mRNA, using art known techniques. The 2.4 kb segment was used as a probe, and an mRNA of about 1.8 kb was identified as homologous, using Northern blot analysis. When cDNA was screened, clones were obtained showing almost complete identity to parts of the 2.4 kb fragment. Two exons were thus identified. An additional exon was located upstream of these, via sequencing segments of cosmid B3 located in front of the 2.4 kb BamHI fragment. The gene extends over about 4.5 kb, as shown in Figure 8. The starting point of the transcribed region was confirmed using PCR for the 5' end of the cDNA. The three exons comprise 65, 73, and 1551 base pairs. An ATG is located at position 66 of exon 3, followed by a 927 base pair reading frame.

**Example 22**

To determine if smaller segments of the 2.4 kb fragment could transfer the expression of antigen E, smaller pieces corresponding to the larger gene were prepared, using art recognized techniques, and transferred into $E^-$ cells. Figure 8 shows the boundaries of the three segments.

Transfer of antigen expression in this manner indicates that the gene codes for the antigen precursor, rather than coding for a protein which activates the antigen.

**Example 23**

The probing of cDNA described supra revealed, surprisingly, two different but closely related cDNAs. These cDNAs, when tested, did not transfer expression of antigen E, but they do show substantial homology to the first cDNA segment. The three segments, appear to indicate a newly recognized family of genes, referred to as "MAGE" for "melanoma antigen". In Figure 9, MAGE-1" directs expression of the antigen from MZ2 cells. Portions of the third exon of each gene are presented in Figure 9. The second and third sequences are more closely related to each other than the first (18.1 and 18.9% difference compared to the first; 12% with each other). Out of 9 cDNA clones obtained, three of each type were obtained, suggesting equal expression. "MAGE" as used hereafter refers to a family of molecules, and the nucleic acids coding for them. These nucleic acids share a certain degree of homology and are expressed in tumor cells including several types of human tumor cells as well as in human tumors. The family is referred to as "MAGE" because the first members were identified in human melanoma cells. As the experiments which follow indicate, however, the members of the MAGE family are not at all restricted to melanoma tumors; rather, MAGE refers to a family of tumor rejection antigen precursors and the nucleic acid sequences coding therefore. The antigens resulting therefrom are referred to herein as "MAGE TRAs" or "melanoma antigen tumor rejection antigens"

**Example 24**

Experiments with mouse tumors have demonstrated that new antigens recognized by T cells can result from point mutations that modify active genes in a region that codes for the new antigenic peptide. New antigens can also arise from the activation of genes that are not expressed in most normal cells. To clarify this issue for antigen MZ2-E, the MAGE-1 gene present in the melanoma cells was compared to that present in normal cells of patient MZ2. Amplification by polymerase chain reaction (PCR) of DNA of phytohemagglutinin-activated blood lymphocytes using primers surrounding a 1300 bp stretch covering the first half of the 2.4 kb fragment was carried out. As expected, a PCR product was obtained whereas none was obtained with the DNA of the $E^-$ variant. The sequence of this PCR product proved identical to the corresponding sequence of the gene carried by the $E^+$ melanoma cells. Moreover, it was found that antigen MZ2-E was expressed by cells transfected with the cloned PCR product. This result suggests that the activation of a gene normally silent is responsible for the appearance of tumor rejection antigen MZ2-E.

**Example 25**

In order to evaluate the expression of gene MAGE-1 by various normal and tumor cells, Northern blots were hybridized with a probe covering most of the third exon. In contrast with the result observed with human tumor cell line MZ2-MEL 3.0, no band was observed with RNA isolated from a CTL clone of patient MZ2 and phytohemagglutinin-activated blood lymphocytes of the same patient. Also negative were several normal tissues of other individuals (Figure 10 and Figure 11). Fourteen melanoma cell lines of other patients were tested. Eleven were positive with bands of varying intensities. In addition to these cultured cell lines, four samples of melanoma tumor tissue were analyzed. Two samples, including a metastasis of patient MZ2, proved positive, excluding the possibility that expression of the gene represented a tissue culture artefact. A few tumors of other histological types, including lung tumors were tested. Most of these tumors were positive (Figures 10 and 11). These results indicated that the MAGE gene family is expressed by many melanomas and also by other tumors. However, they provided no clear indication as to which of genes MAGE-1, 2 or 3 were expressed by these cells, because the DNA probes corresponding to the three genes cross-hybridized to a considerable extent. To render this analysis more specific, PCR amplification and hybridization with highly specific oligonucleotide probes were used. cDNAs were obtained and amplified by PCR using oligonucleotide primers corresponding to sequences of exon 3 that were identical for the three MAGE genes discussed herein. The PCR products were then tested for their ability to hybridize to three other oligonucleotides that showed complete specificity for one of the three genes (Figure 9). Control experiments carried out by diluting RNA of melanoma MZ2-MEL 3.0 in RNA from negative cells indicated that under the conditions used herein the intensity of the signal decreased proportionally to the dilution and that positive signals could still be detected at a dilution of 1/300. The normal cells (lymphocytes) that were tested by PCR were confirmed to be negative for the expression of the three MAGE genes, suggesting therefore a level of expression of less than 1/300$^{th}$ that of the MZ2 melanoma cell line (Figure 11). For the panel of melanoma cell lines, the results clearly showed that some melanomas expressed MAGE genes mage 1, 2 and 3 whereas other expressed only mage-2 and 3 (Figures 11 and 10). Some of the other tumors also expressed all three genes whereas others expressed only MAGE-2 and 3 or only MAGE-3. It is impossible to exclude formally that some positive PCR results do not reflect the expression of one of the three characterized MAGE genes but that of yet another closely related gene that would share the sequence of the priming and hybridizing oligonucleotides. It can be concluded that the MAGE gene family is expressed by a large array of different tumors and that these genes are silent in the normal cells tested to this point.

**Example 26**

The availability of a sequence that transfects at high efficiency and efficiently expresses a TRAP made it possible to search for the associated major histocompatibility complex (MHC) class I molecule. The class I specificities of patient MZ2 are HLA-A1, A29, B37, B44 and C6. Four other melanomas of patients that had A1 in common with MZ2 were cotransfected with the 2.4 kb fragment and pSVtkneoß. Three of them yielded neo$^r$ transfectants that stimulated TNF release by anti-E CTL clone 82/30, which is CD8+ (Figure 11). No E- transfectant was obtained with four other melanomas, some of which shared A29, B44 or C6 with MZ2. This suggests that the presenting molecule for antigen MZ2-E is HLA-A1. In confirmation, it was found that, out of 6 melanoma cell lines derived from tumors of HLA-A1 patients, two stimulated TNF release by anti-E CTL clone 82/30 of patient MZ2. One of these tumor cell lines, MI13443-MEL, also showed high sensitivity to lysis by these anti-E CTL, These two melanomas were those that expressed MAGE-1 gene (Figure 11). Eight melanomas of patients with HLA haplotypes that did not include A1 were examined for their sensitivity to lysis and for their ability to stimulate TNF release by the CTL. None was found to be positive. The ability of some human anti-tumor CTL to lyse allogeneic tumors sharing an appropriate HLA specificity with the original tumor has been reported previously (Darrow, et al., J. Immunol. 142: 3329 (1989)). It is quite possible that antigenic peptides encoded by genes MAGE 2 and 3 can also be presented to autologous CTL by HLA-A1 or other class I molecules, especially in view of the similar results found with murine tumors, as elaborated upon supra.

**Example 27**

As indicated supra, melanoma MZ2 expressed antigens F, D and A', in addition to antigen E. Following the isolation of the nucleic acid sequence coding for antigen E, similar experiments were carried out to isolate the nucleic acid sequence coding for antigen F.

To do this, cultures of cell line MZ2-MEL2.2, an $E^-$ cell line described supra, were treated with anti-F CTL clone 76/6, in the same manner described for treatment with anti-E CTL clones. This resulted in the isolation of an F antigen loss variant, which was then subjected to several rounds of selection. The resulting cell line, "MZ2-MEL2.2.5" was completely resistant to lysis by anti-F CTLs, yet proved to be lysed by anti-D CTLs.

Again, following the protocols set forth for isolation of antigen -E precursor DNA, the $F^-$ variant was transfected with genomic DNA from $F^+$ cell line MZ2-MEL3.0. The experiments yielded 90,000 drug resistant transfectants. These were tested for MZ2-F expression by using pools of 30 cells in the TNF detection assay elaborated upon supra. One pool stimulated TNF release by anti-F CTLs, and was cloned. Five of 145 clones were found to stimulate anti-F CTLs. Lysis assays, also following protocols described supra, confirmed (i) expression of the gene coding for antigen F, and (ii) presentation of antigen F itself.

**Example 28**

Following identification of $F^+$ cell lines, the DNA therefrom was used to transfect cells. To do this, a cosmid library of $F^+$ cell line MZ2-MEL.43 was prepared, again using the protocols described supra. The library was divided into 14 groups of about 50,000 cosmids, and DNA from each group was transfected into MZ2-MEL2.2.5. Transfectants were then tested for their ability to stimulate TNF release from anti-F CTL clone 76/6. Of 14 groups of cosmids, one produced two independent transfectants expressing antigen F; a yield of two positives out of 17,500 geniticin resistant transfectants.

**Example 29**

The existence of a gene family was suggested by the pattern observed on the Southern blot (Figure 12). To do this, the 2.4 kb BamHI fragment, which transferred the expression of antigen MZ2-E, was labelled with 32p and used as a probe on a Southern Blot of BamHI digested DNA of E + cloned subclone MZ2-MEL2.2. Hybridization conditions included 50 µl/$cm^2$ of 3.5xSSC, 1xDenhardt's solution; 25 mM sodium phosphate buffer (pH 7.0), 0.5% SDS, 2mM EDTA, where the 2.4 kb probes had been labelled with [$\alpha^{32}$p]dCTP (2-3000 Ci/mole), at 3x$10^6$ cpm/ml. Hybridization was carried out for 18 hours at 65°C. After this, the membranes were washed at 65°C four times for one hour each in 2xSSC, 0.1% SDS, and finally for 30 minutes in 0.1xSSC, 0.1% SDS. To identify hybridization, membranes were autoradiographed using Kodak X-AR film and Kodak X-Omatic fine intensifying screens.

In the following examples, whenever "hybridization" is referred to, the stringency conditions used were similar to those described supra. "Stringent conditions" as used herein thus refers to the foregoing conditions; subject to routine, art recognized modification.

**Example 30**

The cDNA coding for MAGE 4 was identified from a sample of the human sarcoma cell line LB23-SAR. This cell line was found to not express MAGE 1, 2 or 3, but the mRNA of the cell line did hybridize to the 2.4 kb sequence for MAGE 1. To study this further, a cDNA library was prepared from total LB23-SAR mRNA, and was then hybridized to the 2.4 kb fragment. A cDNA sequence was identified as hybridizing to this probe, and is identified hereafter as MAGE 4.

**Example 31**

Experiments were carried out using PHA-activated lymphocytes from patient "MZ2", the source of the "MZ" cells discussed supra. An oligonucleotide probe which showed homology to MAGE 1 but not MAGE 2 or 3 was hybridized with a cosmid library derived from the PHA activated cells. The size of the hybridizing BamHI cosmid fragment, however, was 4.5 kb, thus indicating that the material was not mage 1; however, on the basis of homology to mage 1-4, the fragment can be referred to as "MAGE 5". The sequence of MAGE 5 is presented in SEQ ID NO: 16.

**Example 32**

Melanoma cell line LB-33-MEL was tested. Total mRNA from the cell line was used to prepare cDNA, which was then amplified with oligos CHO9: (ACTCAGCTCCTCCCAGATTT) (SEQ ID NO: 53), and CHO10: (GAAGAGGAGGGGCCAAG) (SEQ ID NO: 54). These oligos correspond to regions of exon 3 that are common to previously described mage 1, 2 and 3.

To do this, 1 µg of RNA was diluted to a total volume of 20 µl, using 2 µl of 10x PCR buffer, 2 µl of each of 10 mM dNTP, 1.2 µ l of 25 mM $MgCl_2$, 1 µl of an 80 mM solution of CHO9, described supra, 20 units of RNAsin, and 200 units of M-MLV reverse transcriptase. This was followed by incubation for 40 minutes at 42°C. PCR amplification followed, using 8 µl of 10x PCR buffer, 4.8 µl of 25 mM $MgCl_2$, 1 µl of CHO10, 2.5 units of Thermus acquaticus ("Taq") polymerase, and water to a total volume of 100 µl. Amplification was then carried out for 30 cycles (1 minute 94°C; 2 minutes at 52°C, 3 minutes at 72°C). Ten µl of each reaction were then size fractionated on agarose gel, followed by nitrocellulose blotting. The product was found to hybridize with oligonucleotide probe CHO18 (TCTTGTATCCTGGAGTCC) (SEQ ID NO: 55). This probe identified mage 1 but not mage 2 or 3. However, the product did not hybridize to probe SEQ 4 (TTGCCAAGATCTCAGGAA) (SEQ ID NO: 56). This probe also binds mage 1 but not 2 and 3. This indicated that the PCR product contained a sequence that differed from MAGE 1, 2 and 3. Sequencing of this fragment also indicated differences with respect to MAGE 4 and 5. These results indicate a sequence differing from previously identified MAGE 1, 2, 3, 4 and 5, and is named MAGE 6.

**Example 33**

In additional experiments using cosmid libraries from PHA-activated lymphocytes of MZ2, the 2.4 kb MAGE 1 fragment was used as a probe and isolated a complementary fragment. This clone, however, did not bind to oligonucleotides specific for MAGE 1, 2, 3 or 4. The sequence obtained shows some homology to exon 3 of mage 1, and differs from MAGES 1-6. It is referred to as mage 7 hereafter. Additional screenings yielded MAGE 8-11. All MAGE sequences identified are presented as SEQ ID's.

**Example 34**

The usefulness of the TRAPs, as well as TRAs derived therefrom, was exemplified by the following.

Exon 3 of MAGE 1 was shown to transfer expression of antigen E. As a result, it was decided to test whether synthetic peptides derived from this exon 3 could be used to confer sensitivity to anti-E CTL.

To do this, and using standard protocols, cells normally insensitive to anti-E/CTLs were incubated with the synthetic peptides derived from Exon 3.1. Using the CTL lytic assays described supra on P815A, and a peptide concentration of 3 mM, the peptide Glu-Ala-Asp-Pro-Thr-Gly-His-Ser-Tyr (SEQ ID NO: 26) was shown to be best. The assay showed lysis of 30%, indicating conferring of sensitivity to the anti-E CTL.

## Example 35

Nucleic acid sequences referred to as "smage" were isolated from murine cells. Using the protocols described supra, a cosmid library was prepared from the DNA of normal DBA/2 kidney cells, using cosmid vector C2RB. As a probe, the 2.4 kb BamHI fragment of MAGE-1 was used. The DNA was blotted to nylon filters, and these were washed in 2xSSC at 65°C to identify the smage material.

## Example 36

Further tissue samples were tested for the presence of MAGE genes, using the protocols discussed supra. Some of these results follow.

There was no expression of the MAGE genes in brain or kidney tumor tissue. Colon tumor tissue showed expression of MAGE 1, 2, 3 and 4, although not all tumors tested showed expression of all MAGE genes. This is also true for pancreatic tumor (MAGE 1); non-small cell lung (MAGE 1, 2, 3 and 4), prostate (MAGE 1), sarcomas (MAGE 1, 2, 3 and 4), breast (MAGE 1, 2 and 3), and larynx (MAGE 1 and 4).

## Example 37

A cytolytic CTL clone "20/38" was obtained from peripheral blood lymphocytes of melanoma patient MZ2. This clone is described by Van den Eynde et al., Int. J. Cancer 44: 634-640 (1989), the disclosure of which is incorporated by reference. The CTL clone was isolated following Herin et al., Int. J. Cancer 39: 390-396 (1987), which is incorporated by reference. The assay is described herein, however. Autologous melanoma cells were grown in vitro, and then resuspended at $10^7$ cells/ml in DMEM, supplemented with 10% HEPES and 30 mM FCS, and incubated for 45 minutes at 37°C with 20 μCi/ml of $Na(^{51}Cr)O_4$. Labelled cells were washed three times with DMEM, supplemented with 10 mM HEPES. These were then resuspended in DMEM supplemented with 10 mM HEPES and 10% FCS, after which 100 μl aliquots containing $10^3$ cells, were distributed into 96 well microplates. Samples of the CTL clone were added in 100 ul of the same medium, and assays were carried out in duplicate. Plates were centrifuged for four minutes at 100g, and incubated for four hours at 37°C in a 5.5% $CO_2$ atmosphere.

Plates were centrifuged again, and 100 ul aliquots of supernatant were collected and counted. Percentage of $^{51}$Cr release was calculated as follows:

$$\%\ ^{51}\text{Cr release} = \frac{(\text{ER-SR})}{(\text{MR-SR})} \times 100$$

where ER is observed, experimental $^{51}$Cr release, SR is spontaneous release measured by incubating $10^3$ labeled cells in 200 ul of medium alone, and MR is maximum release, obtained by adding 100 ul 0.3% Triton X-100 to target cells.

Those mononuclear blood samples which showed high CTL activity were expanded and cloned via limiting dilution, and were screened again, using the same methodology.

The same method was used to test target K562 cells. When EBV-B cells were used, the only change was the replacement of DMEM medium by Hank's medium, supplemented with 5% FCS.

These experiments led to isolation of CTL clone 20/38.

Figure 14 presents the results of these assays. Specifically, it will be seen that the CTL clone lysed autologous melanoma cell line MZ2-MEL.3.0, but did not lyse EBV-B cell lines, fibroblasts, K562 or non-autologous melanoma cell line SK-MEL-29.

## Example 38

Once the CTL clone was recognized as being specific for the autologous cell line, it was tested for antigenic specificity. To do this, antigen loss variants derived from melanoma cell line MEL-MZ2 were tested in the same type of chromium release assay described above. These target lines were MZ2-MEL 3.0, which is $D^+$, $E^+$, $F^+$, $A^+$, MZ2-MEL. 61, which is $D^-$, MZ2-MEL 2.2, which is $E^-$, and MZ2-MEL.4, which is $F^-$. In addition to CTL clone 20/38, clones which are known to be anti-A (CTL 28/336), anti-F (CTL 76/6), and anti-E (CTL 22/13) were tested.

These results are set forth in figure 15. It will be seen that CTL clone 20/38 lysed all the cell lines leading to chromium release except D- cell line MZ2-MEL.61, thus indicating that the CTL clone is anti-D. This result was confirmed, in experiments not included herein, by experiments where TNF release by the CTL clone was observed only in the presence of melanoma lines presenting antigen D.

## Example 39

Once antigen D was identified as the target molecule, studies were carried out to determine the HLA type which presented it. The experiments described in example 38 showed that antigen D was presented by MZ2-MEL, and this cell line's HLA specificity is known (i.e., A1, A29, B37, B44, Cw6, C.cl.10). It was also known, however, that a variant of MZ2-MEL which had lost HLA molecules A29, B44 and C.cl.10 still expressed antigen D, so these could be eliminated from consideration. Studies were not carried out on lines expressing B37, as none could be found.

In all, 13 allogeneic lines were tested, which expressed either HLA-A1 (10 of 13), or Cw6 (3 of 13). The cell lines were tested for their ability to stimulate release of TNF by CTL clone 20/38, using the method of Traversari et al., Immunogenetics 35: 145-152 (1992).

This assay measures TNF release via testing toxicity of supernatants on WEHI 164-13 cells.

In the assays, cell samples (3000, 10,000 or 30,000 cells) from the allogeneic lines were cultured in the presence of 1500 cells of the CTL clone, and 25 u/ml of IL-2. Twenty-four hours later, the supernatant from the culture was tested against the WEHI cells for toxicity. The results are presented in Table 3, which follows.

Eight cell lines were found to stimulate TNF release from the CTL clone 20/38. All of these lines were HLA-A1. None of the Cw6 presenting lines did so.

The cell lines were also assayed to determine MAGE expression. All eight of the lines which stimulated TNF release expressed MAGE-3, whereas the two HLA-A1 lines which were negative did not.

Table 3

| Melanoma | TNF pg/ml of | | | | | Expression Mage-3 | Expression of HLA-A-1 |
|---|---|---|---|---|---|---|---|
| | Number of Cells | | Exp 1 | | Exp 2 | | |
| | | | +CIL 20/38 | | +CIL 20/38 | | |
| M22-MEL. 61.2 | 50000 | | 1 | | 4 | +++ | + |
| M22-MEL-ET1 | 50000 | | >120 | | >120 | +++ | + |
| | 1666 | | 66 | | >120 | | |
| LY-1-MEL | 30000 | 1 | >120 | 1 | >120 | +++ | + |
| | 10000 | 1 | >120 | 1 | >120 | | |
| | 3000 | <1 | 114 | 2 | >120 | | |
| MI-10221 | 30000 | <1 | >120 | | | +++ | + |
| | 10000 | <1 | 71 | | | | |
| | 3000 | <1 | 74 | | | | |
| LY-2-MEL | 30000 | 1 | 57 | | | +++ | + |
| | 10000 | 1 | 86 | | | | |
| | 3000 | 1 | 91 | | | | |
| LY-4-MEL | 30000 | 1 | >120 | | | +++ | + |
| | 10000 | 1 | >120 | | | | |
| | 3000 | 1 | >120 | | | | |
| SK23-MEL | 30000 | 1 | 112 | | | +++ | + |

Table 3 (continued)

| Melanoma | TNF pg/ml of | | | | | Expression Mage-3 | Expression of HLA-A-1 |
|---|---|---|---|---|---|---|---|
| | Number of Cells | | Exp 1 | | Exp 2 | | |
| | | | +CIL 20/38 | | +CIL 20/38 | | |
| | 10000 | 1 | 116 | | | | |
| | 3000 | 1 | 105 | | | | |
| MI-665/2-MEL | 30000 | 1 | 3 | 2 | 4 | - | + |
| | 10000 | 1 | 2 | 2 | 5 | | |
| | 3000 | 1 | 5,2 | 1 | 5 | | |
| LE34-MEL | 30000 | 1 | >120 | 1 | 2 | +++ | + |
| | 10000 | 1 | >120 | | | | |
| | 3000 | 1 | >120 | | | | |
| LB45-MEL | 30000 | 1 | 11 | 1 | 30 | - | + |
| | 10000 | 1 | 6 | 1 | 12 | | |
| | 3000 | 1 | 2 | <1 | 7 | | |
| NA-6-MEL | 30000 | 1 | 77 | 5 | 98 | +++ | + |
| | 10000 | 1 | 104 | 5 | >120 | | |
| | 3000 | 1 | 110 | 4 | >120 | | |
| MI-13443-MEL | 30000 | 1 | >120 | | | +++ | + |
| | 10000 | 1 | >120 | | | | |
| | 3000 | 1 | >120 | | | | |
| LE5-MIL | 30000 | 1 | 8 | 4 | 9 | + | - |
| | 10000 | <1 | 5 | 4 | 11 | | |
| | 3000 | <1 | 5 | 1 | 5 | | |
| SK64-MEL | 30000 | 1 | 4 | 2 | 5 | ? | - |
| | 10000 | 1 | 2 | 1 | 5 | | |
| | 3000 | 1 | 1 | 1 | 4 | | |
| LE33-MEL | 30000 | | | 1 | 3,5 | +++ | - |
| | 10000 | | | 1 | 4 | | |
| | 3000 | | | 1 | 3 | | |
| LB73-MEL | 50000 | | 16 | | | - | - |

1500 CTL 20/38 and 25μ/ml IL2 were mixed with the indicated number of cells of the different allogeneic melanomas. 24 hours later, the amount of TNF present in the supernatent was assayed by testing its cytotoxicity for WEHI-164-13 cells.

**Example 40**

In view of the results set forth in example 39, experiments were carried out to determine if antigen D was in fact a tumor rejection antigen derived from MAGE-3. To do this, recipient COS-7 cells were transfected with 100ng of the gene for HLA-A1 cloned into pcDNA I/Amp, and 100 ng of one of (a) cDNA for MAGE-1 cloned into pcDNA I/Amp, (b) cDNA for MAGE-2 cloned into pcDSRα, or (c) cDNA for MAGE-3 cloned into pcDSRα. The transfecting sequences were ligated into the plasmids in accordance with manufacturer's instructions. Samples of COS-7 cells were seeded, at 15,000 cells/well into tissue culture flat bottom microwells, in Dulbeco's modified Eagles Medium ("DMEM") supplemented with 10% fetal calf serum. The cells were incubated overnight at 37°C, medium was removed and then replaced by 30 μl/well of DMEM medium containing 10% Nu serum, 400 μg/ml DEAE-dextran, 100 μM chloroquine, and the plasmids described above. Following four hours of incubation at 37°C, the medium was removed, and replaced by 50 μl of PBS containing 10% DMSO. This medium was removed after two minutes and replaced by 200 μl of DMEM supplemented with 10% of FCS.

Following this change in medium, COS cells were incubated for 24 hours at 37°C. Medium was then discarded, and 1500 cells of CTL clone 20/38 were added, in 100 μl of Iscove's medium containing 10% pooled human serum, supplemented with 25 u/ml of IL-2. Supernatant was removed after 24 hours, and TNF content was determined in an assay on WEHI cells, as described by Traversari et al., Immunogenetics 35: 145-152 (1992).

These results are shown in figure 16.

It will be seen that the CTL clone was strongly stimulated by COS-7 cells transfected with HLA-A1 and MAGE-3, but not by the cells transfected with the other Mage genes. This leads to the conclusion that antigen D is a tumor rejection antigen derived from the tumor rejection antigen precursor coded by gene MAGE-3, and that this TRA is presented by HLA-A1 molecules.

## Example 41

It is well known that different alleles of genes may produce different proteins. This principle should extend to the MAGE family of genes as well, and is an important consideration in view of diagnostic and therapeutic ramifications. Thus, polymorphism in the MAGE family was studied.

To address the issue of polymorphism, blood lymphocytes of ten individuals were collected, and genomic DNA extracted. This DNA was subjected to Southern blotting in accordance with James et al., Canc. Res. 48: 5546-5551 (1988). Briefly, the labelled 2.4 kb genomic DNA fragment of MAGE-1, containing the last two exons of MAGE-1, described supra, was hybridized with the filter carrying the digested DNA, at 42°C for at least 16 hours, in 50% formamide, 5% dextran sulfate, 6xSSC, 1% SDS and 0.1 mg/ml heterologous DNA. The hybridization filters were washed, consecutively, in 2xSSC, 0.1% SDS (room temperature, 15 minutes), and twice in 0.1xSSC, 0.1% SDS at 67°C for 30 minutes, each wash. Autoradiography was carried out at -70°C for 7-10 days, using standard film.

A pattern of 13 hybridizing bands was observed, which was conserved over all individuals. One individual did show an additional band, but also showed the 13 band pattern.

## Example 42

It was of interest to determine which chromosome or chromosomes bear the MAGE genes. To ascertain this, a panel of hamster/human somatic cell hybrids was used. The hybrids were obtained either from the Human Genetic Mutant Cell Repository ("GM" prefix), or from Johns Hopkins University ("$A_3$" prefix). Each hybrid was cytogenetically studied to determine human chromosome content.

Total genomic DNA of the hybrids was probed in the same manner described in Example 41, supra (the conditions of stringency used prevented cross hybridization with hamster DNA).

Table 4, which follows, summarizes the result of the probe work. Analysis of the data led to the conclusion that the pattern of hybridization was only concordant with location of MAGE-1 on the X chromosome.

**TABLE 4** - Segregation of MAGE-1 with human chromosomes in human-hamster hybrid cell DNA

| Hybrid | MAGE-1 | Human chromosome 1 | 2 | 3 | 4 | 5 | 6 | 7 | 8 | 9 | 10 | 11 | 12 | 13 | 14 | 15 | 16 | 17 | 18 | 19 | 20 | 21 | 22 | X | Y |
|---|---|---|---|---|---|---|---|---|---|---|---|---|---|---|---|---|---|---|---|---|---|---|---|---|---|
| GM06317 | - | - | - | - | - | - | - | - | - | - | - | - | - | - | - | - | - | - | - | - | - | - | - | - | + |
| GM06318B | + | - | - | - | - | - | - | - | - | - | - | - | - | - | - | - | - | - | - | - | - | - | - | + | - |
| GM07300 | + | - | - | - | - | - | + | - | + | - | - | + | - | - | - | - | - | - | - | - | - | - | - | + | - |
| GM07301 | + | - | - | - | - | - | - | - | - | - | - | - | + | - | - | - | - | - | - | - | - | - | - | + | - |
| GM08854 | - | - | - | - | - | - | - | - | - | - | - | - | - | - | - | - | - | - | - | - | - | + | - | - | - |
| GM09142 | - | - | - | + | - | + | - | - | - | - | - | - | - | - | - | - | - | - | - | - | - | 1 | - | 2 | - |
| GM10095 | + | - | - | - | - | - | - | - | - | 3 | - | - | - | - | - | - | - | - | - | - | - | - | - | 4 | - |
| GM10115 | - | - | - | - | + | - | - | - | - | - | - | - | - | - | - | - | - | - | - | - | - | - | - | - | - |
| GM10156B | - | - | - | - | - | - | - | - | + | - | - | - | - | - | - | - | - | - | - | - | - | - | - | - | - |
| GM10253 | - | - | - | + | - | - | - | - | - | - | - | - | - | - | - | - | - | - | - | - | - | - | - | - | - |
| GM10322 | - | - | - | - | - | - | - | - | - | - | - | - | - | - | - | - | - | - | - | - | - | + | + | - | - |
| GM10478 | - | - | - | - | + | - | - | - | - | - | - | - | - | - | - | - | - | - | - | - | + | - | - | - | - |
| GM10479 | - | - | - | - | - | - | - | - | - | - | - | - | - | - | + | - | - | - | - | - | - | - | - | - | - |
| GM10498 | - | - | - | - | - | - | - | - | - | - | - | - | - | - | - | - | - | + | - | - | - | - | - | - | - |
| GM10567 | - | - | - | - | - | - | - | - | - | - | - | - | - | - | - | - | + | - | - | - | - | - | - | - | - |
| GM10611 | - | - | - | - | - | - | - | - | - | + | - | - | - | - | - | - | - | - | - | - | - | - | - | - | - |
| GM10612 | - | - | - | - | - | - | - | - | - | - | - | - | - | - | - | - | - | - | - | + | - | - | - | - | - |
| GM10629 | - | - | - | - | - | - | + | - | - | - | - | - | - | - | - | - | - | - | - | - | - | - | - | - | - |
| GM10791 | - | - | - | - | - | - | - | + | - | - | - | - | - | - | - | - | - | - | - | - | - | - | - | - | - |
| GM10880 | - | + | - | - | - | - | - | - | - | - | - | - | - | + | + | - | - | - | - | - | - | - | - | - | - |
| GM10888 | - | - | - | - | - | - | - | - | - | - | - | - | - | - | - | - | - | - | - | - | - | - | + | - | - |
| A3ADA ID12 | - | + | - | + | + | + | - | - | + | - | - | - | + | + | + | - | + | - | - | - | + | + | - | - | + |
| A3ADA6F5 | + | + | + | - | - | + | + | + | + | - | - | - | + | + | + | + | - | + | + | + | - | + | + | + | + |
| A3ADA13 | + | + | - | + | + | - | - | + | + | - | - | + | + | + | + | + | ± | - | - | + | - | + | - | + | + |
| A3ADA14 | - | - | - | + | + | + | - | - | + | - | + | + | + | + | + | ± | - | - | - | - | + | + | + | - | + |
| A3G1 | + | - | - | + | + | - | 6 | - | ± | + | + | - | - | - | - | ± | - | + | - | ± | + | - | ± | + | + |
| A3HR20 | - | - | - | + | + | + | + | - | - | + | - | + | - | - | + | - | - | + | - | - | - | - | ± | - | - |
| P8Me4 | + | - | 5 | + | - | - | + | + | + | - | - | + | + | + | + | + | - | + | - | - | ± | + | - | ± | + |
| Number of concordant hybrids | (+/+) | 2 | 1 | 3 | 2 | 1 | 3 | 3 | 4 | 2 | 1 | 3 | 4 | 3 | 3 | 3 | 0 | 3 | 1 | 2 | 1 | 3 | 1 | 8 | 4 |
| | (-/-) | 17 | 19 | 14 | 14 | 15 | 18 | 18 | 16 | 17 | 18 | 17 | 17 | 16 | 14 | 18 | 17 | 17 | 19 | 18 | 16 | 14 | 15 | 18 | 17 |
| Number of discordant hybrids | (+/-) | 6 | 6 | 5 | 6 | 7 | 3 | 5 | 3 | 6 | 7 | 5 | 4 | 5 | 5 | 4 | 7 | 5 | 7 | 5 | 6 | 5 | 6 | 0 | 4 |
| | (-/+) | 2 | 0 | 5 | 5 | 4 | 2 | 1 | 3 | 2 | 1 | 2 | 2 | 3 | 5 | 0 | 2 | 2 | 0 | 1 | 3 | 5 | 3 | 0 | 2 |
| Percent discordancy | | 22 | 23 | 38 | 37 | 41 | 24 | 22 | 23 | 30 | 30 | 26 | 22 | 30 | 37 | 16 | 33 | 26 | 27 | 23 | 35 | 37 | 36 | 0 | 26 |

\+ = chromosome present; - = chromosome absent;
± = very faint bands, indicating that only a small percentage of the cells contained the chromosome (not included in calculation of percent discordancy
1,2 - GM09142 contains only part of chromosomes X and 21, der 21 1(X;21)(p21;p12)
3,4 - GM10095 contains only part of chromosomes X and 9, der 9 1(X;9)(q13;q34)
5 - PgMe4 contains a deleted chromosome 2 and is missing 2p23-p24
6 - A3G1 contains only the q arm of chromosome 6

## Example 43

In this experiment, a study was carried out to determine if all twelve known MAGE genes were located on the X chromosome. This was accomplished via the use of polymerase chain reaction ("PCR") technology.

RNA purification and cDNA synthesis were first carried out, in accordance with Weynants et al., Int. J. Cancer 56: 826-829 (1994). Next, 1/20 of the cDNA produced from 2 ug of total RNA was supplemented with 5 ul of PCR buffer (500 mM KCl, 100 mM Tris pH 8.3), 1 ul each of 10 mM dNTPs, 25 pmoles of each primer (see below), 3 ul of 25 mM $MgCl_2$, and 1.25 units of Taq polymerase, with water added to final volume of 50 ul.

The primers were as follows:

MAGE-3: 5'-TGGAGGACCAGAGGCCCCC, 5'-GGACGATTATCAGGAGGCCTGC (725 bp) (SEQ ID NOS: 27 AND 28)

MAGE-4: 5'-GAGCAGACAGGCCAACCG, 5'-AAGGACTCTGCGTCAGGC (446 bp) (SEQ ID NOS; 29 AND 30)

MAGE-5: 5'CTAGAGGAGCACCAAAGGAGAAG, 5'-TGCTCGGAACACAGACTCTGG (413 bp) (SEQ ID NOS: 31 AND 32)

MAGE-6: 5'-TGGAGGACCAGAGGCCCCC, 5'-CAGGATGATTATCAGGAAGCCTGT (727 bp) (SEQ ID NOS: 33 AND 34)

MAGE-7: 5'-CAGAGGAGCACCGAAGGAGAA, 5'-CAGGTGAGCGGGGTGTGTC (405 bp) (SEQ ID NOS: 35 AND 36)

MAGE-8: 5'-CCCCAGAGAAGCACTGAAGAAG, 5'-GGTGAGCTGGGTCCGGG (399 bp) (SEQ ID NOS: 37 AND 38)

MAGE-9: 5'-CCCCAGAGCAGCACTGACG, 5'-CAGCTGAGCTGGGTCGACC (391 bp) (SEQ ID NOS: 39 AND 40)

MAGE-10: 5'-CACAGAGCAGCACTGAAGGAG, 5'-CTGGGTAAAGACTCACTGTCTGG (485 bp) (SEQ ID NOS: 41 AND 42)

MAGE-11: 5'-GAGAACCCAGAGGATCACTGGA, 5'-GGGAAAAGGACTCAGGGTCTATC (422 bp) (SEQ ID NOS: 43 AND 44)

MAGE-12: 5'-GGTGGAAGTGGTCCGCATCG, 5'-GCCCTCCACTGATCTTTAGCAA (392 bp) (SEQ ID NOS: 45 AND 46)

Amplification was carried out for 30 cycles (MAGE-3, 4, 6, 12) or 32 cycles (MAGE-5, 7-11), where a cycle was one minute at 94°C followed by two minutes at 65°C for MAGE-5, 7-12, or two minutes at 68°C (MAGE-4), or two minutes at 71°C (MAGE-3 and MAGE-6); followed by three minutes at 72°C (MAGE-3, 5-12), or two minutes at 72°C (MAGE-4). The analysis was carried out on hybrid cell line GM 10868, which contains human chromosome 12, and GM 07301, which contains chromosome 12 and the X-chromosome. All assays were negative with the human GM 10868 line, and all were positive with the GM 07301 cell line, which indicated that all 12 genes are found on the X-chromosome.

**Example 44**

The sizes of mRNAs for the different MAGE genes are similar, and thus Northern blot analysis cannot be used to determine expression of the various MAGE genes in different tissues, both normal and tumor. PCR analysis, along the lines of the study in example 43, supra, however, was believed to be useful.

To this end, a series of various tumors and normal tissues were tested for expression of MAGE genes.

Total RNA of the cells tested was extracted, and was then oligo dT primed, following art known techniques. The resulting material was then subjected to PCR, following the protocols of example 43, supra. For MAGE-1 and MAGE-2, the protocols of Brasseur et al., Int. J. Cancer. 52: 839-841 (1992), and DeSmet et al., Immunogenetics 39: 121-120 (1994).

Table 5, which follows, elaborates these results, with a representative but by no means exhaustive listing of tissues tested. Each of MAGE 1-4, 6 and 12 showed significant expression in a number of tumors of varied tissue types. MAGE-5 and 8-11 were expressed very weakly in all tissues tested, whereas MAGE-7 RNA was not detectable at all. With respect to normal tissues, including tissues taken from a >20 week fetus, all were negative for MAGE RNA but for testis and placenta. Testis expressed all MAGE genes but MAGE-7, while placenta expressed MAGE-3, 4, and 8-11.

TABLE 5.

| Expression of tissues Mage-1, 2, 3, 4, 6 and -12 by tumors and normal | | | | | | |
|---|---|---|---|---|---|---|
| | MAGE 1 | MAGE 2 | MAGE 3 | MAGE 4 | MAGE 6 | MAGE 12 |
| COLON CARCINOMAS | | | | | | |
| MZ-CO-2¶ | ++ | ++ | + | - | - | + |
| SK-CO-11¶ | - | ++ | +++ | - | + | ++ |
| LB150** | - | - | - | + | - | - |
| HSR 320 ¶ | - | +++ | +++ | + | ++ | +++ |
| LEUKEMIAS | | | | | | |
| K562 ¶ | - | ++ | +++ | - | ++ | +++ |
| MELANOMAS | | | | | | |
| MI10221 ¶ | - | +++ | +++ | +++ | +++ | +++ |
| MZ2-MEL 3.0 ¶ | +++ | +++ | +++ | - | +++ | + |
| LB265** | - | ++ | - | - | - | + |
| LG7** | - | ++ | - | - | - | - |
| LG11** | ++ | ++ | ++ | - | - | +++ |
| L3271 ** | - | ++ | +++ | - | ++ | +++ |
| LUNG CANCERS | | | | | | |
| LB178(NSCLC) ** | ++ | - | - | +++ | - | - |
| LB175 (NSOLC). | - | ++ | +++ | +++ | - | +++ |
| LB11 (SCLC) ¶ | ++ | +++ | +++ | - | - | +++ |
| LB12 (SCLC) ¶ | - | +++ | +++ | - | - | +++ |
| SARCOMAS | | | | | | |
| LB23 ¶ | - | - | - | ++ | - | - |
| LB408** | - | - | - | ++ | - | - |
| LB258** | + | ++ | + | - | - | ++ |
| BREAST CARCINOMAS | | | | | | |
| LB280** | ++ | - | ++ | - | - | + |
| LB284** | ++ | ++ | ++ | + | - | ++ |
| Stomach | | - | - | - | - | - |
| Lung | - | - | - | - | - | - |
| Breast | - | - | - | - | - | - |
| Colon | - | - | - | - | - | - |
| Skin | - | - | - | - | - | - |
| Uterus | - | - | - | - | - | - |
| Testis | ++ | ++ | ++ | ++ | ++ | ++ |

TABLE 5. (continued)

| Expression of tissues Mage-1, 2, 3, 4, 6 and -12 by tumors and normal | | | | | | |
|---|---|---|---|---|---|---|
| | MAGE 1 | MAGE 2 | MAGE 3 | MAGE 4 | MAGE 6 | MAGE 12 |
| COLON CARCINOMAS | | | | | | |
| Thymocytes | - | - | - | - | - | - |
| EBV-lymphocytes | - | - | - | - | - | - |
| Foetal liver | - | - | - | - | - | - |
| Foetal brain | - | - | - | - | - | - |
| Placenta LB694 | - | - | + | +++ | - | - |
| RNA from tumor cell lines (¶), tumor samples (**) and normal tissues were tested by RT-PCR for the expression of MAGE genes. PCR primers were chosen as indicated in methods. For MAGE-12, PCR amplification of RNA in the absence of reverse transcription indicated that in our conditions the contamination by genomic DNA was negligible. The level of expression evaluated by band intensity of PCR products fractionated in agarose gels is represented by +++, ++, +. Absence of product is indicated by -. | | | | | | |

**Example 45**

The expression of the MAGE-1, 2 and 3 genes in various tumors and normal tissues was evaluated, using both reverse transcription and polymerase chain reaction ("PCR") amplification. To perform these assays, the total RNA of the cells of interest was extracted via the well known guanidine-isothiocyanate procedure of Davis et al., Basic Methods in Molecular Biology, 1986 (New York, Elsevier, pp. 130), which is incorporated by reference in its entirety. cDNA was then synthesized, by taking 2 ug of the RNA, diluting it with water, and then adding the following materials: 4 ul of 5X reverse transcriptase buffer, 1 ul each of each dNTP (10 mM), 1 ul of a 40 µM solution of oligo dT(15), 20 units of RNAsin, 2 ul of 0.1 M dithiothreitol, and 200 units of MoMLV reverse transcriptase. All materials were mixed in a 20 ul reaction volume, and incubated at 42°C for 60 minutes and diluted to 100 ul with water.

Presence or absence of each of MAGE-1, -2, and -3 cDNA was detected via PCR amplification, in separate reactions, using oligonucleotide primers located in different exons of the MAGE gene of interest. For MAGE-1, the primers were:

5'-CGGCCGAAGGAACCTGACCCAG-3'
(SEQ ID NO: 47)

5'-GCTGGAACCCTCACTGGGTTGCC-3'
(SEQ ID NO: 48)

These are described by Brasseur et al., Int. J. Cancer 52: 839-841 (1992).
For MAGE-2, the primers were:

5'-AAGTAGGACCCGAGGCACTG-3'
(SEQ ID NO: 49)

5'-GAAGAGGAAGAAGCGGTCTG-3'
(SEQ ID NO: 50)

(DeSmet et al., Immunogenetics 39: 121-129 (1994)).
For MAGE-3, the primers were:

5'-TGGAGGACCAGAGGCCCCC-3' (SEQ ID NO: 27)

5'-GGACGATTATCAGGAGGCCTGC-3' (SEQ ID NO: 28)

For each PCR reaction, 5 ul of cDNA were supplemented with 5 ul of 10XPCR buffer, 1 ul of each dNTP (10 mM), 1 ul each of 40 µM primer solutions, 1.25 units of Taq polymerase, and water, to a total volume of 50 ul. Each mixture was heated for five minutes at 94°C. Amplification was then carried out for 30 cycles (MAGE-1: 1 minute at 94°C, 3 minutes at 72°C; MAGE-2: 1 minute at 94°C, 2 minutes at 67°C; MAGE-3: 1 minute at 94°C, 4 minutes at 72°C). Cycling was concluded, in each case, with a final extension at 72°C for 15 minutes. A 10 ul sample of each reaction was run on a 1% agarose gel, and visualized by ethidium bromide fluorescence. To ensure that RNA was not degraded, a PCR assay with primers specific for β-actin was carried out, following the listed protocols, except that only 20 cycles were carried out with the annealing step at 65°C. Date are summarized in the Table which follows:

Table 6

| **Expression of gene MAGE-1,2 and 3 in lung tumors** | | | |
|---|---|---|---|
| | Proportion of positive samples | | |
| | MAGE-1 | MAGE-2 | MAGE-3 |
| **Non-small cell lung cancer** | 16/46 | 16/46 | **14/46** |
| squamous cell carcinoma | 8/26 | 6/26 | **7/26** |
| adenocarcinoma | 8/18 | 9/18 | 7/18 |
| large cell carcinoma | 0/2 | 1/2 | 0/2 |
| **Small cell cancer** | 1/3 | 2/3 | 2/3 |
| **Normal lung samples** | 0/8 | 0/8 | 0/8 |

## Example 46

The previous example showed how to identify expression of various MAGE genes. This example explains quantitation of the expression.

First, cDNA was synthesized in the same way described in example 45, except that the oligo dT consisted solely of dT15, and the reaction mixture was preincubated at room temperature for 10 minutes to optimize annealing. Also, following the incubation, the transcriptase activity was terminated by heating the mixture at 95°C for 15 minutes. PCR amplification was carried out, by combining 5 ul of 10x PCR buffer, 0.5 ul of a 2.5 mM dNTP mix, 0.2 µCi of $\alpha^{32}$P-dCTP, 0.5 ul of each primer (40 µM solution), 1.25 units of Taq polymerase, and water, to a total of 50 ul. The mixtures were chilled on ice, and then 5 ul of chilled cDNA solution (100 ng total RNA) were added thereto. The mixture was heated to 94°C for five minutes, and 24 cycles of amplification were carried out (one minute at 94°C, three minutes at 72°C per cycle). Cycling concluded with a final extension at 72°C, for 15 minutes. A 15 ul sample of PCR product was run on an agarose gel which was then fixed in 10% trichloroacetic acid for 30 minutes, dried, and then exposed to a phospho-screen for 90 minutes before scanning by Phosphor-Imager to measure incorporated $^{32}$P. This was compared to the incorporations from various dilutions of RNA of reference melanoma cell line MZ2-MEL-3.0.

Quantitative measurements of β-actin messenger and "GAPDH" (i.e., glyceraldehyde 3-phosphate dehydrogenase) was carried out on each cDNA sample, under similar conditions. The one difference was that only 18 amplification cycles were carried out. A separate PCR reaction was set up with primers for β-actin and GAPDH, with only β-actin used for normalization. Results were expressed via formula:

$$100 \times \frac{\left[\frac{\text{MAGE-1-S}}{\text{Actin-S}}\right]}{\left[\frac{\text{MAGE-1-MEL}}{\text{Actin-MEL}}\right]}$$

where:

S = product from tumor sample
MEL = product from MZ2-MEL 3.0

The results obtained were comparable to those obtained previously with melanoma tumors. Level of expression varied, from 1 to 160% of the amount expressed by the reference cell line. Figure 18 presents some of these results (i.e., normalized results, relative to levels of β-actin expression). Values are percent of the level of MAGE-1 expression measured with RNA of the reference line MZ2-MEL-3.0. Values are for MAGE-1 positive tumors of Table 7). Table 7, which follows, summarizes patterns of expression for various tumors.

Table 7.

| Pattern of expression of genes MAGE-1,2 and 3 by MAGE-positive lung tumor samples | | | |
|---|---|---|---|
| | MAGE-1° | MAGE-2 | MAGE-3 |
| Squamous cell carcinoma | | | |
| LB 175 | ++ | ++ | +++ |
| LB 178 | ++ | - | - |
| LB 182 (A1)$^{-}$ | - | + | - |
| LB 195 | + | ++ | +++ |
| LB 206 | +++ | + | ++ |
| LB 321 | + | - | - |
| LB 323 | +++ | + | +++ |
| LB 424 | + | - | + |
| LB 425 | - | - | + |
| LB 498 (A1) | +++ | - | - |
| LB 557 | - | +++ | +++ |
| Adenocarcinoma | | | |
| LB 117 (A1) | + | ++ | ++ |
| LB 212 | ++ | + | - |
| LB 264 (A1) | +++ | ++ | +++ |
| LB 292 | - | ++ | +++ |
| LB 306 | ++ | + | ++ |
| LB 322 | - | + | + |
| LB 474 (A1) | + | ++ | - |
| LB 497 | ++ | +++ | +++ |
| LB 510 | +++ | - | - |
| LB 558 (A1) | + | + | + |
| Large cell carcinoma | | | |
| LB 259 | - | + | - |

Table 7. (continued)

| Pattern of expression of genes MAGE-1,2 and 3 by MAGE-positive lung tumor samples | | | |
|---|---|---|---|
| | MAGE-1° | MAGE-2 | MAGE-3 |
| Small cell lung cancer | | | |
| LB 444 | - | ++ | +++ |
| LB 648 (A1) | + | ++ | +++ |

**Example 47**

The expression of the MAGE-3 gene in various tumors and normal tissues was evaluated, using both reverse transcription and polymerase chain reaction ("PCR") amplification. To perform these assays, the total RNA of the cells of interest was extracted via the well known guanidine-isothiocyanate procedure of Davis et al., Basic Methods in Molecular Biology, 1986 (New York, Elsevier, pp. 130). cDNA was then synthesized, by taking 2 ug of the RNA, diluting it with water, and then adding the following materials: 4 ul of 5X reverse transcriptase buffer, 1 ul each of each dNTP (10 mM), 2 ul of a 20 μM solution of oligo dT, 20 units of RNAsin, 2 ul of 0.1 M dithiothreitol, and 200 units of MoMLV reverse transcriptase. All materials were mixed in a 20 ul reaction volume, and incubated at 42°C for 60 minutes. For the amplification reaction, 1/20 of the cDNA reaction product was supplemented with 5 ul of PCR buffer, 0.5 ul of each of the dNTPs (10 mM), 1 ul each of 20 μM solutions of primer (see infra), and 1.25 units of Taq polymerase. Water was added to a final volume of 50 uls. The primers used for MAGE-3 were:

5'-TGGAGGACCAGAGGCCCCC-3'
(SEQ ID NO: 27)

5'-GGACGATTATCAGGAGGCCTGC-3'
(SEQ ID NO: 28)

These correspond to a sense sequence in exon 2 of the gene (SEQ ID NO: 27), and an antisense sequence in exon 3 (SEQ ID NO: 28).

PCR was performed for 30 cycles (one minute at 94°C, four minutes at 72°C). PCR products were size fractionated on a 1% agarose gel, and then analyzed. The results are presented in the table which follows. These data confirm some results obtained previously, but also show the expression of MAGE-3 in head and neck squamous cell carcinomas, a result not suggested by previous work.

Table 8. Expression of gene MAGE-3 by tumoral, normal and fetal tissues.

**TUMORS**

| HISTOLOGICAL TYPE | Number of MAGE-3 positive tumors* | |
|---|---|---|
| | *cell lines* | *tumors samples* |
| Melanomas | 50/62 (81%) | 72/105 (69%) |
| Head and neck squamous cell carcinomas | - | 20/36 (56%) |
| Lung carcinomas | | |
| *NSCLC ‡* | 1/2 | 14/46 (30%) |
| *SCLC* | 18/22 (82%) | 2/3 |
| Colorectal carcinomas | 5/16 | 5/31 (16%) |
| Mammary carcinomas | 2/6 | 16/132 (12%) |
| Bladder tumors | - | 2/6 |
| Sarcomas | 1/4 | 3/10 |
| Prostatic carcinomas | - | 3/20 |
| Renal carcinomas | 0/5 | 0/38 |
| Leukemias | 2/6 | 0/20 |
| Lymphomas | 0/6 | 0/5 |

**NORMAL TISSUES**

| HISTOLOGICAL TYPE | MAGE-3 expression* |
|---|---|
| *ADULT TISSUES* | |
| Brain | - |
| Colon | - |
| Stomach | - |
| Liver | - |
| Ovary | - |
| Skin | - |
| Lung | - |
| Kidney | - |
| Breast | - |
| Testis | ++ |
| *FETAL TISSUES* | |
| Brain | - |
| Liver | - |
| Spleen | - |

*Expression of gene MAGE-3 was tested by RT-PCR amplification on total RNA, with the primers described in methods. These primers distinguish MAGE-3 from the 11 other MAGE genes that have been identified.

‡ NSCLC are non-small cell lung carcinomas, SCLC are small cell lung carcinomas.

**Example 48**

Bladder tumor specimens were collected at surgery. They were divided into two portions, one of which was used for routine histopathological evaluation. The other portion was frozen in liquid nitrogen immediately after transurethral resection, or radical cystectomy. These frozen samples were stored at -80°C until used for RNA extraction. Normal bladder tissue was obtained by biopsies of cadavers from donors in an organ transplant program.

Total RNA was extracted from the samples by the classic guanidine-isothiocyanate/cesium chloride method of Davis et al, Basic Methods in Molecular Biology, pp. 130-135, Elsevier, New York (1986). Synthesis of cDNA was then carried out extension with oligo(dT) using 2 ug of RNA in a 20 ul reaction volume following DeSmet et al., Immunogenetics 39:121-129(1994).

Following incubation at 42°C for one hour, the cDNA reaction mixture was diluted to 100 ul with water. Separate polymerase chain reaction amplification were then carried out to determine whether any of MAGE-1, 2, 3 or 4 cDNA were present. The amplifications were carried out using oligonucleotide primers located in different exons of the MAGE genes. PCR amplification was also carried out using primers for HLA-A1.

The primers used were the following:

5'-TGGAGGACCAGAGGCCCCC-3 (sense, exon 2) (SEQ ID NO: 27) and

5'-GGACGATTATCAGGAGGCCTGC-3' (antisense, exon 3) (SEQ ID NO: 28) for MAGE-3

5'-CGGCCGAAGGAACCTGACCCAG-3' (sense, exon 1) (SEQ ID NO: 47) and

5'GCTGGAACCCTCACTGGGTTGCC-3' (anti-sense, exon 3) (SEQ ID NO: 48) for MAGE-1

5'-AAGTAGGACCCGAGGCACTG-3' (sense, exon 2) (SEQ ID NO: 49) and

5'-GAAGAGGAAGAAGCGGTCTG-3' (anti-sense, exon 3) (SEQ ID NO: 50) for MAGE-2

5'-GAGCAGACAGGCCAACCG-3' (sense, exon 2) (SEQ ID NO: 29) and

5'-AAGGACTCTGCGTCAGGC-3' (anti-sense, exon 3) (SEQ ID NO: 30) for MAGE-4

5'-GGGACCAGGAGACACGGAATA-3' (sense, exon 2) (SEQ ID NO: 51) and

5'-AGCCCGTCCACGCACCG-3' (anti-sense, exon 3) (SEQ ID NO: 52) for HLA-A1

SEQ ID NOS: 27 and 28 are described by Weynants et al., Int. J. Cancer 56: 826-829 (1994). SEQ ID NOS: 47 and 48 are described in Brasseur et al., Int. J. Cancer 52: 839-841 (1992). SEQ ID NOS: 49 and 50 are disclosed in DeSmet et al., Immunogenetics 39: 121-129 (1994). SEQ ID NOS: 29 and 30 are disclosed in WO. 9 523 874. SEQ ID NOS: 51 and 52 are found in Gaugler et al., J. Exp. Med. 179: 921-930 (1994), as well as the above-identified parent application. All of these references are incorporated by reference.

The amplification protocol was as follows. Each PCR reaction used 5 ul of cDNA, supplemented with 5 ul of 10x PCR buffer, 1 ul each of 10 mM dNTP, 0.5 ul each of 80 uM solutions of primers, 1.25 units of Taq DNA polymerase, and water to achieve a total volume of 50 ul. The mixtures were heated to 94°C for 5 minutes, followed by amplification in a thermal cycler, for 30 cycles. For MAGE-1, 1 cycle was one minute at 94°C followed by three minutes at 72°C. For MAGE-2, one cycle was 94°C for one minute, followed by two minutes at 67°C and two minutes at 72°C. For MAGE-3, one cycle was one minute at 94°C; followed by four minutes at 72°C. For MAGE-4, one cycle was one minute at 94°C, two minutes at 68°C, and two minutes at 72°C. The cycle for HLA-A1 was the same as that for MAGE-4. A 10 ul sample of each reaction was run on a 1% agarose gel, and then visualized by ethidium bromide fluorescence. In order to provide a control for RNA integrity, a 20 cycle PCR assay, using primers specific for β actin, was carried out in each case, following Weynants et al., supra.

The protocols described were developed with certain goals in mind. Primers were selected so as to be in different exons, thus preventing false positives due to DNA contamination of the RNA preparations. Under the conditions used, DNA generates either no PCR product, or longer products which are readily distinguishable from amplified cDNA. This is shown by figure 19. In figure 19, a bladder tumor sample from a patient, referred to as "HM15" is shown in each "R" lane. Lanes marked "D" show products obtained from amplification of the patients' genomic DNA. The PCR products were run on a 2.5% low melting agarose gel, but the assays were identical to the protocol of this example in all other ways. Size markers are on the left hand side. There was no band in the MAGE-1 reaction, because of the large intron between the two primers.

Table 10, which follows, shows the results obtained for a number of tumors (nomenclature is explained below). Of 57 samples of primary transitional cell carcinoma, 21% expressed MAGE-1, 30% expressed MAGE-2, 35% expressed MAGE-3, and 33% expressed MAGE-4. Ta tumors and low grade T1 tumors expressed none of these, or expressed only a single gene, at low levels. Higher stage tumors, in contrast, frequently expressed high levels of several genes. It was also found that the fraction of invasive tumors which expressed MAGE genes was 2-5 times higher than the fraction observed with superficial tumors, as is depicted in figure 20 (this figure is based upon data from Table 10). Tumors expressing at least one of the four MAGE genes accounted for 61% of the 28 invasive tumors studied. Among the 29 superficial tumors, the proportion was only 28%. Results paralleled other results reported previously for melanoma, in that all but one of the tumors expressing MAGE-1 also expressed MAGE-3.

None of the six biopsies of normal bladder examined expressed any of the MAGE genes discussed herein.

In some instances, several tumor samples were obtained from the same patient. The analysis of these patients is set out in Table 9. Patient HM61 had a primary tumor and an invaded lymph node. They displayed a very similar pattern of expression of MAGE-1, 2, and 3, with MAGE-1 predominating. Normal mucosa adjacent to the tumor was completely negative for MAGE-2 and MAGE-3, with a very low level of MAGE-1 expression, which was probably due to the presence of a few tumor cells. In patient "HM25", the initial tumor, and an early recurrence, both expressed MAGE-1, 2, 3 and 4. A recurrence which occurred two years after the first displayed a very different pattern, expressing only MAGE-2 and MAGE-3. A similar discordance between primary tumor and recurrence was observed with patient "HM20". Patients HM30 and LB526 showed differences in the pattern of MAGE-expression in different samples of the same primary tumor.

In the tables which follow, "Ta" stands for a superficial lesion, limited to bladder mucosa (also known as "stage Ta"). "Stage T1", or "T1" is used for superficial lesions limited to subepithelial connective tissue. "Stages T2-T4", or "T2-T4" refer to tumors which have invaded bladder muscle. The nomenclature "G1", "G2" and "G3" refers to the degree of differentiation, or histopathological grade. "G1" superficial tumor is well differentiated, while a "G3" tumor is poorly differentiated. See Mostofi et al., "Histological Typing of Urinary Bladder Tumors. WHO International Histological Classification o Tumors" (1973).

TABLE 9 - EXPRESSION OF GENES MAGE-1, 2, 3 AND 4 IN MULTIPLE SAMPLES FROM BLADDER CARCINOMA PATIENTS

| Patients | Samples | Tumor stage and grade | MAGE-1 | MAGE-2 | MAGE-3 | MAGE-4 |
|---|---|---|---|---|---|---|
| HM 61 | Primary tumor | T2 G3 | ++ | + | + | - |
| | Metastatic iliac lymph node | | +++ | + | + | - |
| | Mucosa adjacent to the tumor | | + | - | - | - |
| HM 25 | Primary tumor | T2 G2 | + | ++ | + | ++ |
| | Tumor recurrence after 1 month | T2 G2 | ++ | ++ | ++ | ++ |
| | Tumor recurrence after 2 years | T1 G2 | - | +++ | +++ | - |
| HM 20 | Primary tumor | T1 G1 | + | - | - | - |
| | Tumor recurrence after 2 months | T1 G1 | - | - | - | - |
| HM 30 | Primary tumor, 1st sample | T2 G2 | - | - | ++ | - |
| | Primary tumor, 2nd sample | T2 G2 | - | - | - | - |
| LB 526 | Primary tumor, radical cystectomy | T3 G2 | + | + | ++ | + |
| | Primary tumor, 9-day pre-operative biopsy | T3 G2 | + | + | + | - |

¶Table 10.

| EXPRESSION OF GENES MAGE-1, 2, 3 AND 4 IN BLADDER TRANSITIONAL-CELL CARCINOMA SAMPLES | | | | | | |
|---|---|---|---|---|---|---|
| Tumor Stage and Grade ‡ | | Patients | MAGE-1 | MAGE-2 | MAGE-3 | MAGE-4 ¶ |
| Superficial tumors (n=29) | | | | | | |
| Ta *(n=7)* | G1 | HM 7 | - | - | + | - |
| | | HM 32 (Al)* | - | - | - | |
| | | HM 33 (Al) | - | - | - | - |
| | | HM 49 | - | - | - | - |
| | G2 | LB 523 | - | - | - | - |
| | | LB 817 | - | - | - | - |
| | | LB 818 | - | - | - | - |
| T1 *(n=22)* | G1 | HM 2 | - | - | - | - |
| | | HM 6 (Al) | - | - | - | - |
| | | HM 17 | - | - | - | - |
| | | HM 20 | + | - | - | - |
| | | HM 22 | - | - | - | - |
| | | HM 34 | - | | | |
| | | HM 35 | - | - | - | + |
| | G2 | HM 4 | - | + | + | + |
| | | HM 5 | - | - | - | - |
| | | HM 9 | - | - | - | - |
| | | HM 27 | - | - | - | +++ |
| | | HM 37 | - | - | - | - |
| | | HM 38 (Al) | - | - | - | - |
| | | HM 39 | - | - | - | + |
| | | HM 40 (Al) | - | - | - | - |
| | | HM 41 | - | - | - | - |
| | C3 | HM 14 | ++ | +++ | +++ | ++ |
| | | HM 23 | - | - | - | - |
| | | HM 26 | - | +++ | +++ | +++ |
| | | HM 42 (Al) | - | - | - | - |
| | | HM 53 | - | - | - | - |
| | | LB 767 (Al) | - | - | - | - |
| Invasive turnors (n=28) | | | | | | |
| T2 *(n=15)* | G2 | HM 8 | - | - | - | - |
| | | HM 13 (Al) | - | - | - | - |
| | | HM 24 (Al) | + | +++ | +++ | ++ |
| | | HM 25 | + | ++ | + | ++ |
| | | HM 30 | - | - | ++ | - |
| | | LB 796 | - | - | - | - |
| | G3 | HM 3 (Al) | - | - | - | - |
| | | HM 10 | - | + | ++ | ++ |
| | | HM 12 | - | + | + | - |
| | | HM 15 | +++ | +++ | +++ | +++ |
| | | HM 61 (Al) | ++ | + | + | - |
| | | LB 524 (Al) | - | - | +++ | + |
| | | LB 824 | - | ++ | +++ | + |
| | | LB 825 | + | ++ | + | + |
| | | LB 831 | + | ++ | +++ | ++ |

Table 10. (continued)

| EXPRESSION OF GENES MAGE-1, 2, 3 AND 4 IN BLADDER TRANSITIONAL-CELL CARCINOMA SAMPLES | | | | | | |
|---|---|---|---|---|---|---|
| Invasive turnors (n=28) | | | | | | |
| T3 *(n=11)* | G2 | HM 44 | - | - | - | - |
| | | HM 45 | - | +++ | +++ | - |
| | | HM 46 (Al) | - | - | - | - |
| | | LB 526 | + | + | ++ | + |
| | G3 | HM 11 | ++ | + | ++ | + |
| | | HM 18 | - | - | - | - |
| | | HM 21 | - | - | - | - |
| | | HM 47 | +++ | +++ | +++ | +++ |
| | | HM 48 | - | - | - | - |
| | | HM 50 | +++ | +++ | +++ | + |
| | | HM 52 (Al) | - | - | - | - |
| T4 *(n=2)* | G3 | HM 1 | - | - | - | + |
| | | HM 51 | - | - | - | - |

The foregoing examples show that expression of MAGE tumor rejection antigen precursors is correlated to various cancers. One aspect of the invention, then, is a method for determining these cancers by assaying a sample for expression of at least one MAGE tumor rejection antigen precursor. As MAGE genes are nearly without exception expressed only tumor cells, there can be no question but that expression of a MAGE gene or genes is indicative of cancer. The fact that the cancer is a particular type, such as lung adenocarcinoma, is easily ascertainable, as adenocarcinoma cells have distinct morphologies which are identifiable by the skilled artisan. Similarly, the fact that the tumor of interest is a lung adenocarcinoma as compared to a tumor from a different body part is self evident; one does not find lung adenocarcinoma in, e.g., large intestine tissue. Analogous statements can be made for bladder and other cancers.

The assay for the MAGE genes can take many forms. Most preferably the assay is done via determining gene expression, such as by determining mRNA transcription products. For example, amplification protocols, including but not being limited to polymerase chain reaction (PCR), and ligase chain reaction (LCR), are preferred. The assay can also be carried out using nucleic acid molecule probes, which are labelled or unlabelled, and which specifically hybridize to sequences characteristic of the MAGE gene of interest. Labelling nucleotide probes is well known to the art, labels including radioactive, fluorescent, chromophoric, magnetic, and other identifiable materials. Antibodies, haptens such as biotin, (strept) avidin, digoxin, digoxigenin, and so forth, can all be used. Non-labelled probes can also be used. In such a case, the probes will form a double stranded molecule with their target. Any remaining single stranded material can be enzymatically digested, and when something remains, it is a sign of MAGE expression. For the case of polymerase chain reaction or other methodologies where a primer or primers are required, the molecules represented by SEQ ID NO: 47 and SEQ ID NO: 48 are especially preferred for MAGE-1, SEQ ID NO: 49 and 50 for MAGE-2, SEQ ID NOS: 27 and 28 for MAGE-3 and SEQ ID NOS: 29 and 30 for MAGE-4. Similarly, these molecules are preferred as probes.

Quantitation of MAGE expression is shown herein as well. This is an important feature of the invention because in a given tumor sample (as compared to tumor cell lines) there will always be an undetermined proportion of normal cells.

One may also assay for the expression product of the MAGE gene, e.g., the tumor rejection antigen precursor protein, via assays such as immunoassays. See, e.g., U.S. Patent Application Serial No. 08/190,411 filed February 1, 1994, and Chen, et al., Proc. Natl. Acad. Sci. USA 91(3): 1004-1008 (1994) teaching MAGE-1 specific mAbs.

The terms and expressions which have been employed are used as terms of description and not of limitation, and there is no intention in the use of such terms and expressions of excluding any equivalents of the features shown and described or portions thereof, it being recognized that various modifications are possible within the scope of the invention.

## Claims

1. A pair of nucleic acid molecules for use in the amplification of at least part of a MAGE tumor rejection antigen precursor **characterised in that** the members are selected from the group consisting of SEQ ID NO's: 27-46.

2. A kit useful in determining expression of a MAGE tumor rejection antigen precursor, comprising at least one of the following pairs:

   SEQ ID NOS: 27 and 28
   SEQ ID NOS: 29 and 30
   SEQ ID NOS: 31 and 32
   SEQ ID NOS: 33 and 34
   SEQ ID NOS: 35 and 36
   SEQ ID NOS: 37 and 38
   SEQ ID NOS: 39 and 40
   SEQ ID NOS: 41 and 42
   SEQ ID NOS: 43 and 44
   SE ID NOS: 45 and 46.

3. Method for determining expression of a cancer MAGE tumor rejection antigen precursor in a cell comprising:

   i) isolating nucleic acid from a cell to be analysed;
   ii) contacting said nucleic acid with at least one pair of nucleic acid molecules of claim 1 or 2; and
   iii) incubating said nucleic acid/nucleic acid pair under conditions conducive to the PCR amplification of at least one MAGE tumour rejection antigen precursor so as to determine the expression of said MAGE precursor.

4. The method of claim 3, wherein said expression of said tumor rejection antigen precursor is a determination of the presence, regression or spread of cancer.

5. The method of claim 4, wherein said cancer is melanoma.

6. The method of claim 4, comprising contacting said sample with SEQ ID NOS: 27 and 28.

7. The method of claim 4, wherein said cancer is a head squamous cell carcinoma, a neck squamous cell carcinoma, a prostate carcinoma and a bladder tumour, the method comprising contacting said sample with SEQ ID NO's: 27 and 28 or SEQ ID NO's: 29 and 30.

8. The method of claim 4, wherein said cancer is a bladder tumour, said method comprising contacting said sample with SEQ ID NO's: 27 and 28, followed by amplification.

9. The method of claim 4, wherein said MAGE tumor rejection antigen precursor is MAGE-3 or MAGE-4.

## Patentansprüche

1. Ein Paar Nukleinsäuremoleküle zur Verwendung bei der Amplifikation von mindestens einem Teil eines MAGE tumorabstoßenden Antigenvorläufers, **dadurch gekennzeichnet, daß** die Teile aus der Gruppe, die aus SEQ ID NR.: 27-46 bestehen, ausgewählt sind.

2. Ein Kit, das zur Bestimmung der Expression eines tumorabstoßenden MAGE-Antigenvorläufers nützlich ist, bestehend aus mindestens einem der folgenden Paare:

   SEQ ID NR.: 27 und 28
   SEQ ID NR.: 29 und 30
   SEQ ID NR.: 31 und 32
   SEQ ID NR.: 33 und 34
   SEQ ID NR.: 35 und 36
   SEQ ID NR.: 37 und 38

SEQ ID NR.: 39 und 40
SEQ ID NR.: 41 und 42
SEQ ID NR.: 43 und 44
SEQ ID NR.: 45 und 46.

**3.** Verfahren zur Bestimmung der Expression eines tumorabstoßenden Krebs-MAGE-Antigenvorläufers in einer Zelle, bestehend aus:

i) dem Isolieren der Nukleinsäure von einer zu analysierenden Zelle;

ii) dem In-Kontakt-Bringen dieser Nukleinsäure mit mindestens einem Paar Nukleinsäuremoleküle aus Anspruch 1 oder 2; und

iii) dem Inkubieren der Nukleinsäure/des Nukleinsäurepaars unter Bedingungen, die der PCR-Amplifikation von mindestens einem MAGE tumorabstoßenden Antigenvorläufer förderlich sind, um die Expression dieses MAGE-Vorläufers zu bestimmen.

**4.** Verfahren gemäß Anspruch 3, wobei die Expression des tumorabstoßenden Antigenvorläufers eine Bestimmung des Vorhandenseins, der Rückbildung oder der Ausbreitung von Krebs ist.

**5.** Verfahren gemäß Anspruch 4, wobei der Krebs ein Melanom ist.

**6.** Verfahren gemäß Anspruch 4, das das In-Kontakt-Bringen der Probe mit SEQ ID NR.: 27 und 28 umfaßt.

**7.** Verfahren gemäß Anspruch 4, wobei der Krebs ein squamöses Zellkarzinom am Kopf, ein squamöses Zellkarzinom am Hals, ein Prostatakarzinom und ein Blasentumor ist, wobei das Verfahren das In-Kontakt-Bringen der Probe mit SEQ ID NR.: 27 und 28 oder SEQ ID NR.: 29 und 30 umfaßt.

**8.** Verfahren gemäß Anspruch 4, wobei der Krebs ein Blasentumor ist, wobei das Verfahren das In-Kontakt-Bringen der Probe mit SEQ ID NR.: 27 und 28 und anschließende Amplifikation umfaßt.

**9.** Verfahren gemäß Anspruch 4, wobei der MAGE tumorabstoßende Antigenvorläufer MAGE-3 oder MAGE-4 ist.

## Revendications

**1.** Une paire de molécules d'acide nucléique destinées à être utilisées dans l'amplification d'au moins une partie d'un précurseur d'antigène tumoral de rejet MAGE **caractérisées en ce que** les éléments sont sélectionnés dans le groupe constitué des SEQ ID NOS : 27 à 46.

**2.** Un kit utile pour déterminer l'expression d'un précurseur d'antigène tumoral de rejet MAGE, comprenant au moins une des paires suivantes :

SEQ ID NOS: 27 et 28
SEQ ID NOS : 29 et 30
SEQ ID NOS : 31 et 32
SEQ ID NOS : 33 et 34
SEQ ID NOS : 35 et 36
SEQ ID NOS : 37 et 38
SEQ ID NOS : 39 et 40
SEQ ID NOS 41 et 42
SEQ ID NOS 43 et 44
SEQ ID NOS: 45 et 46.

**3.** Procédé pour déterminer l'expression d'un précurseur d'antigène tumoral de rejet MAGE d'un cancer dans une cellule comprenant les étapes consistant :

i) à isoler l'acide nucléique d'une cellule devant être analysée ;

ii) à mettre en contact ledit acide nucléique avec au moins une paire de molécules d'acide nucléique de la revendication 1 ou de la revendication 2 ; et

iii) à incuber ledit acide nucléique/ladite paire d'acides nucléiques dans des conditions propices à l'amplification par PCR d'au moins un précurseur d'antigène tumoral de rejet MAGE afin de déterminer l'expression dudit précurseur de MAGE.

**4.** Le procédé de la revendication 3, dans lequel ladite expression dudit précurseur d'antigène tumoral de rejet est une détermination de la présence, la régression ou la propagation du cancer.

**5.** Le procédé de la revendication 4, dans lequel ledit cancer est un mélanome.

**6.** Le procédé de la revendication 4, comprenant l'étape consistant à mettre en contact ledit échantillon avec les SEQ ID NOS : 27 et 28.

**7.** Le procédé de la revendication 4, dans lequel ledit cancer est un carcinome cellulaire squameux de la tête, un carcinome cellulaire squameux du cou, un carcinome de la prostate et une tumeur de la vessie, le procédé comprenant l'étape consistant à mettre ledit échantillon en contact avec les SEQ ID NOS : 27 et 28 ou les SEQ ID NOS : 29 et 30.

**8.** Le procédé de la revendication 4, dans lequel ledit cancer est une tumeur de la vessie, ledit procédé comprenant l'étape consistant à mettre en contact ledit échantillon avec les SEQ ID NOS : 27 et 28, suivie par l'amplification.

**9.** Le procédé de la revendication 4, dans lequel ledit précurseur d'antigène tumoral de rejet MAGE est MAGE-3 ou MAGE-4.

# FIG. 1A

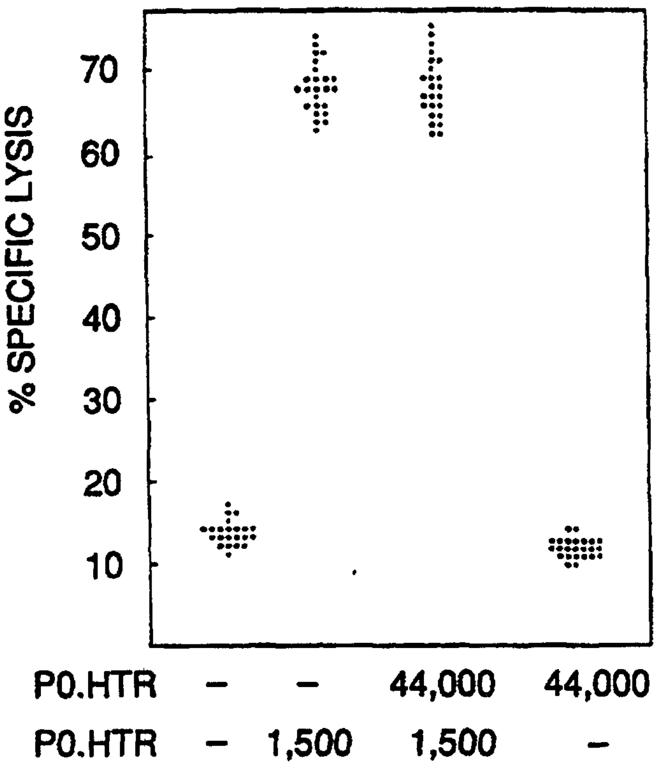
% SPECIFIC LYSIS
70
60
50
40
30
20
10
P0.HTR – – 44,000 44,000
P0.HTR – 1,500 1,500 –

FIG. 1B

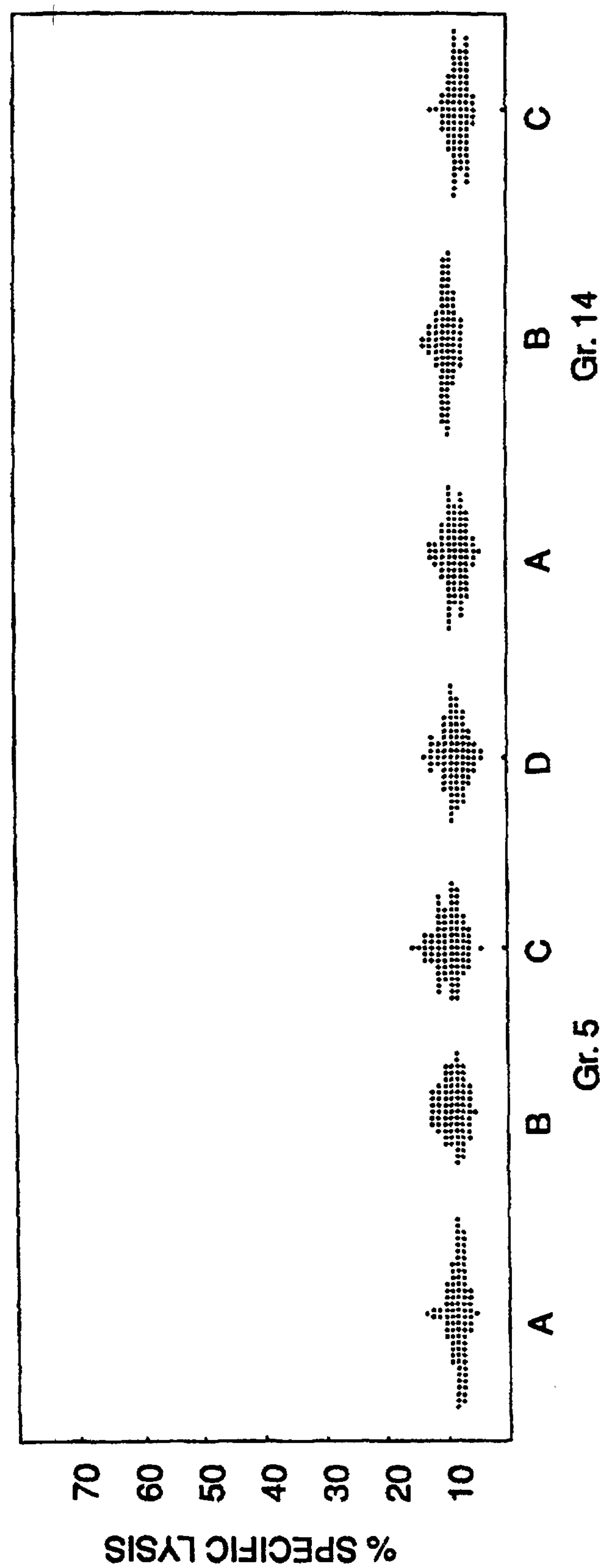

FIG. 2

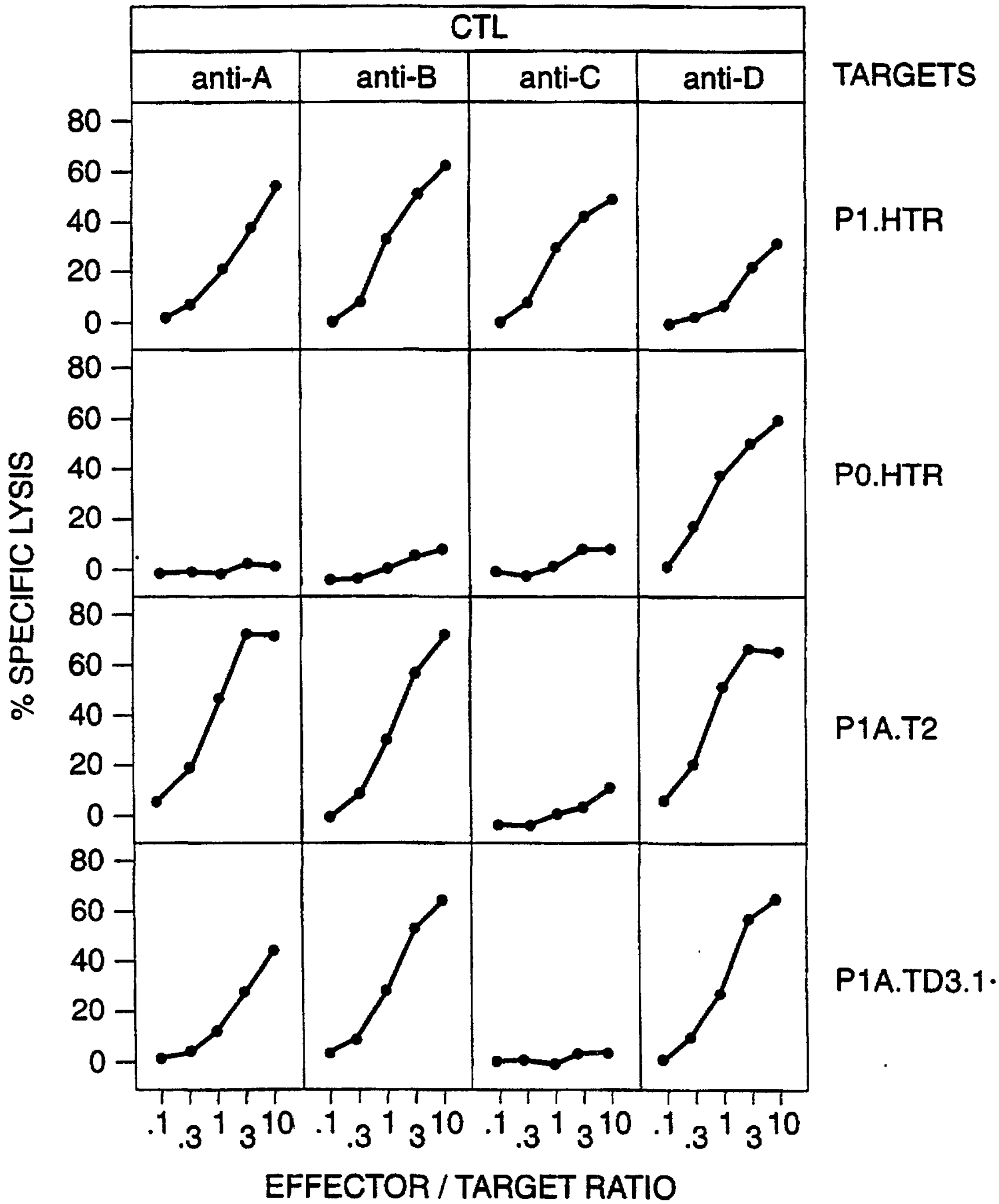
CTL
anti-A
anti-B
anti-C
anti-D
TARGETS
P1.HTR
P0.HTR
P1A.T2
P1A.TD3.1
% SPECIFIC LYSIS
80
60
40
20
0
.1 .3 1 3 10
EFFECTOR / TARGET RATIO

# FIG. 3

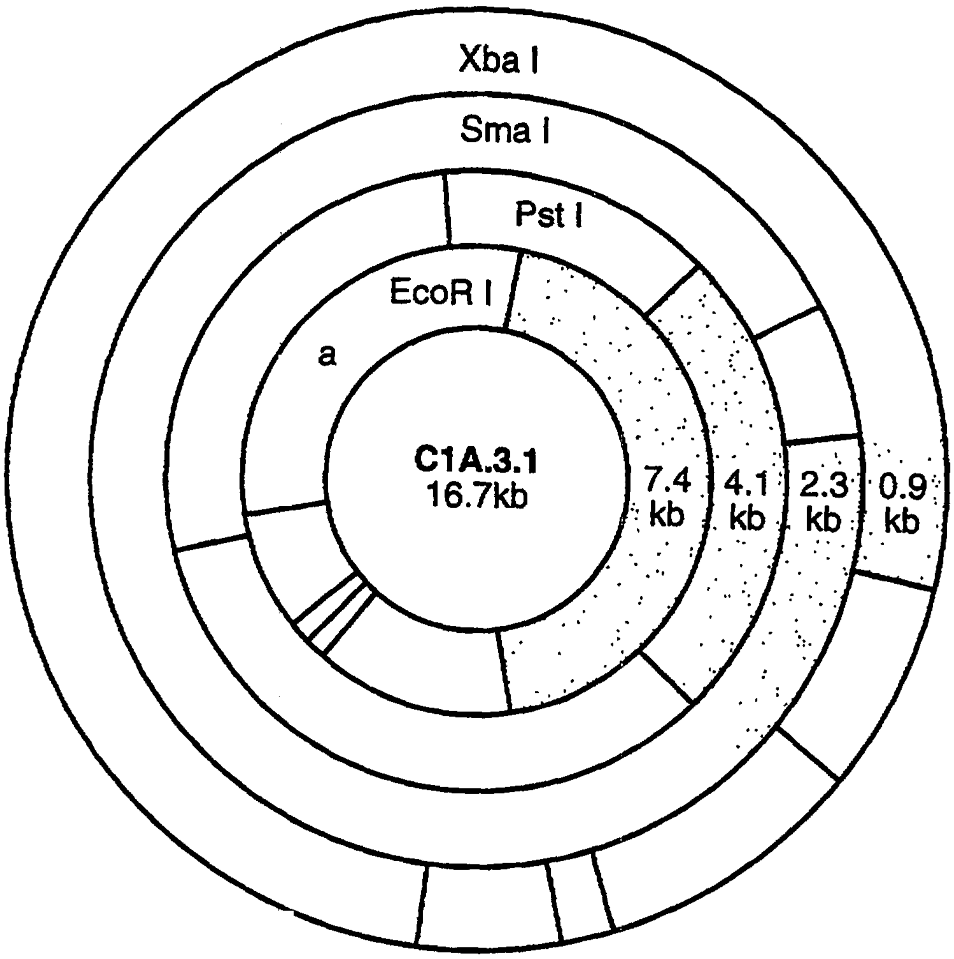

FIG. 4

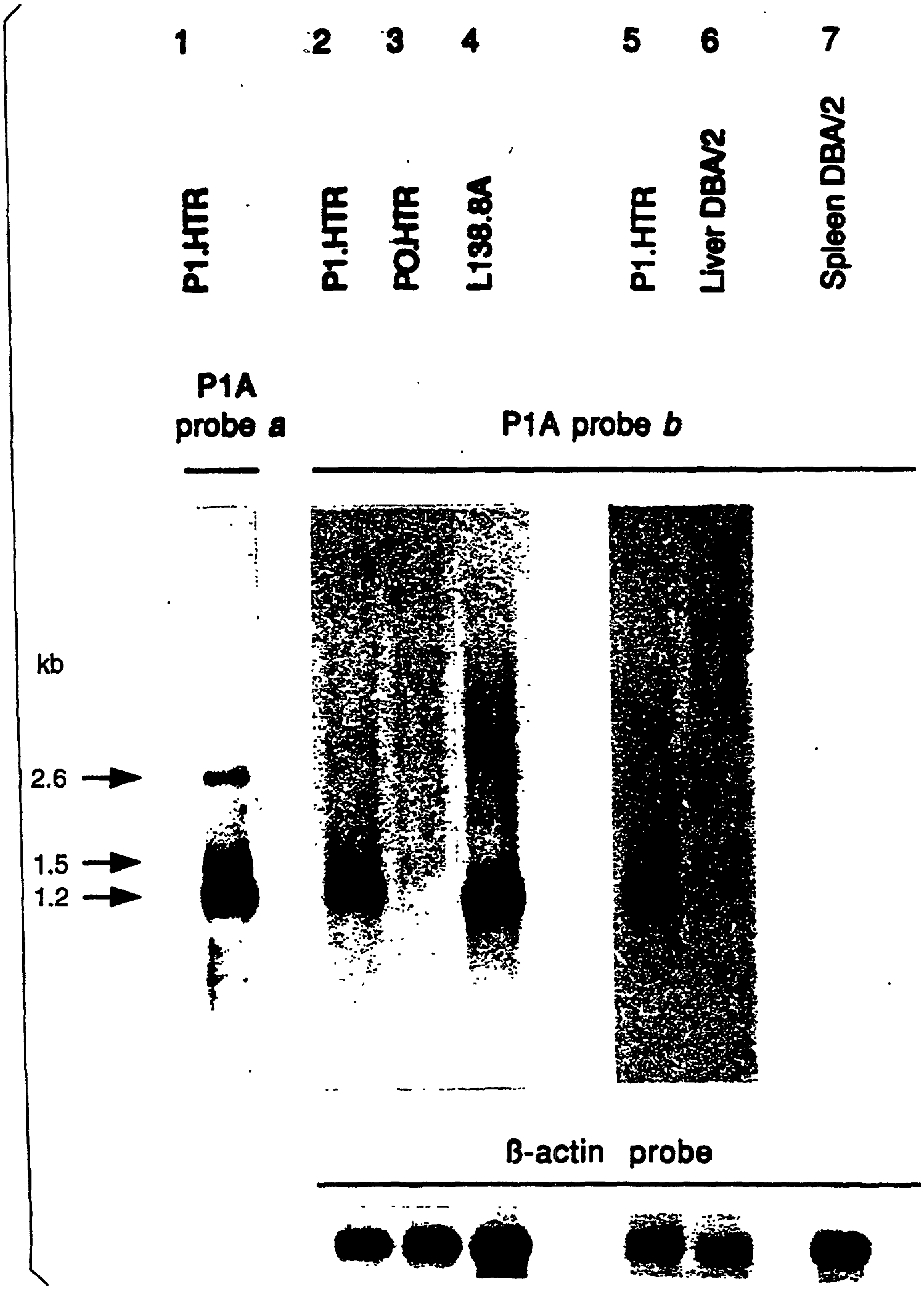
1
2
3
4
5
6
7
P1.HTR
P1.HTR
PO.HTR
L138.8A
P1.HTR
Liver DBA/2
Spleen DBA/2
P1A probe a
P1A probe b
kb
2.6
1.5
1.2
ß-actin probe

FIG. 5

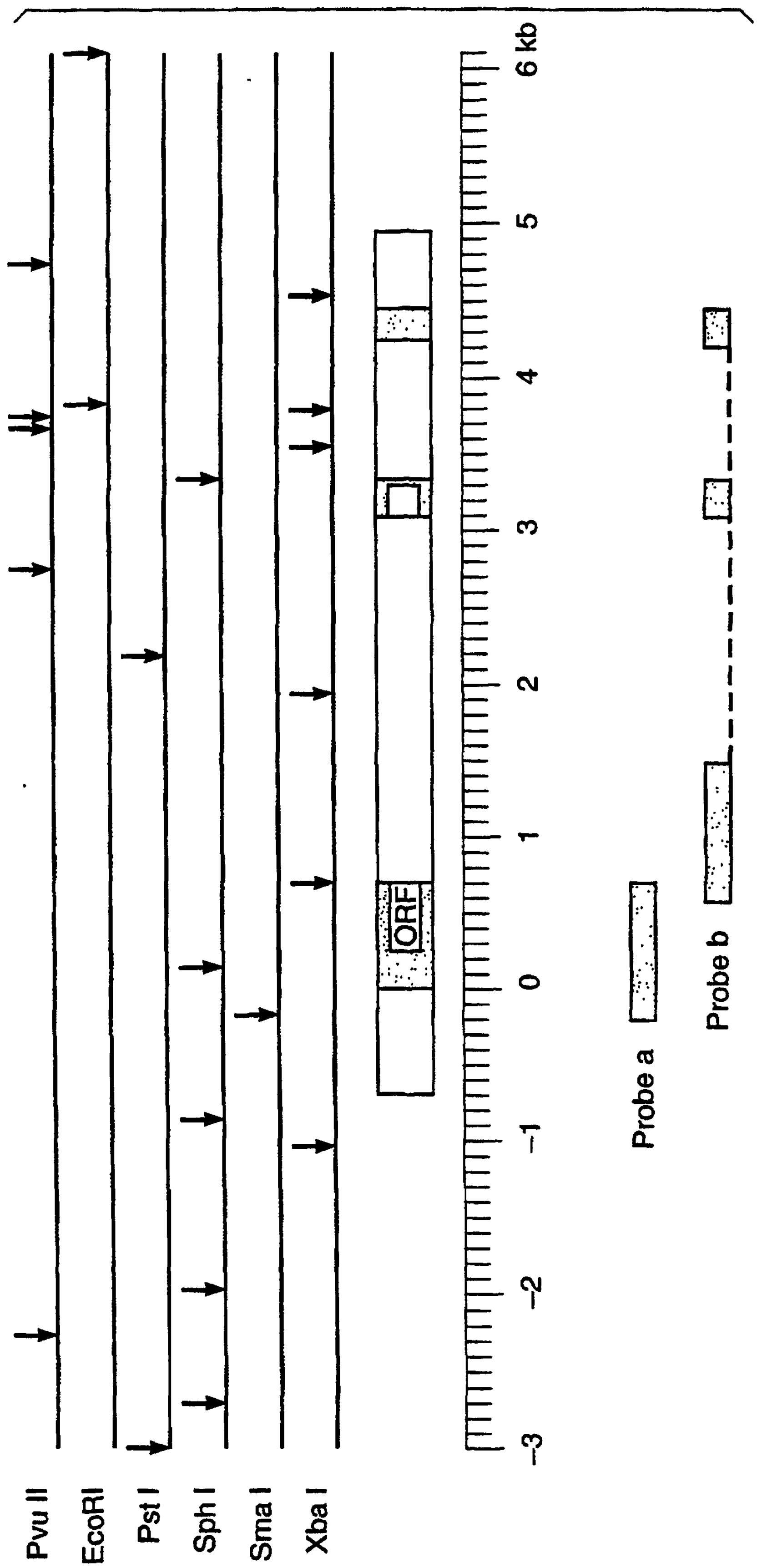
Pvu II
EcoRI
Pst I
Sph I
Sma I
Xba I
ORF
Probe a
Probe b
−3
−2
−1
0
1
2
3
4
5
6 kb

# FIG. 6

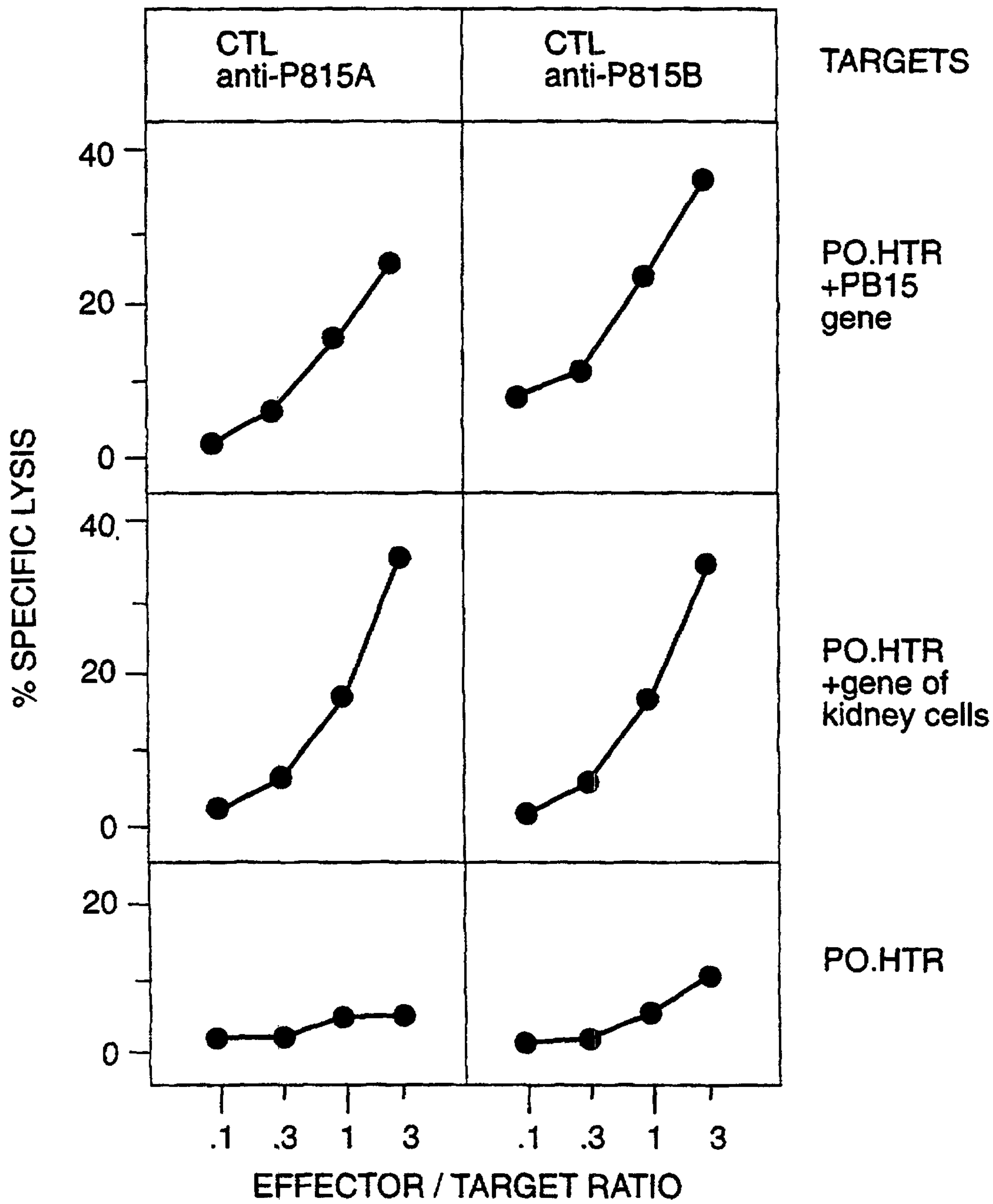
CTL
anti-P815A
CTL
anti-P815B
TARGETS
PO.HTR
+PB15
gene
PO.HTR
+gene of
kidney cells
PO.HTR
% SPECIFIC LYSIS
40
20
0
.1
.3
1
3
EFFECTOR / TARGET RATIO

# FIG. 7

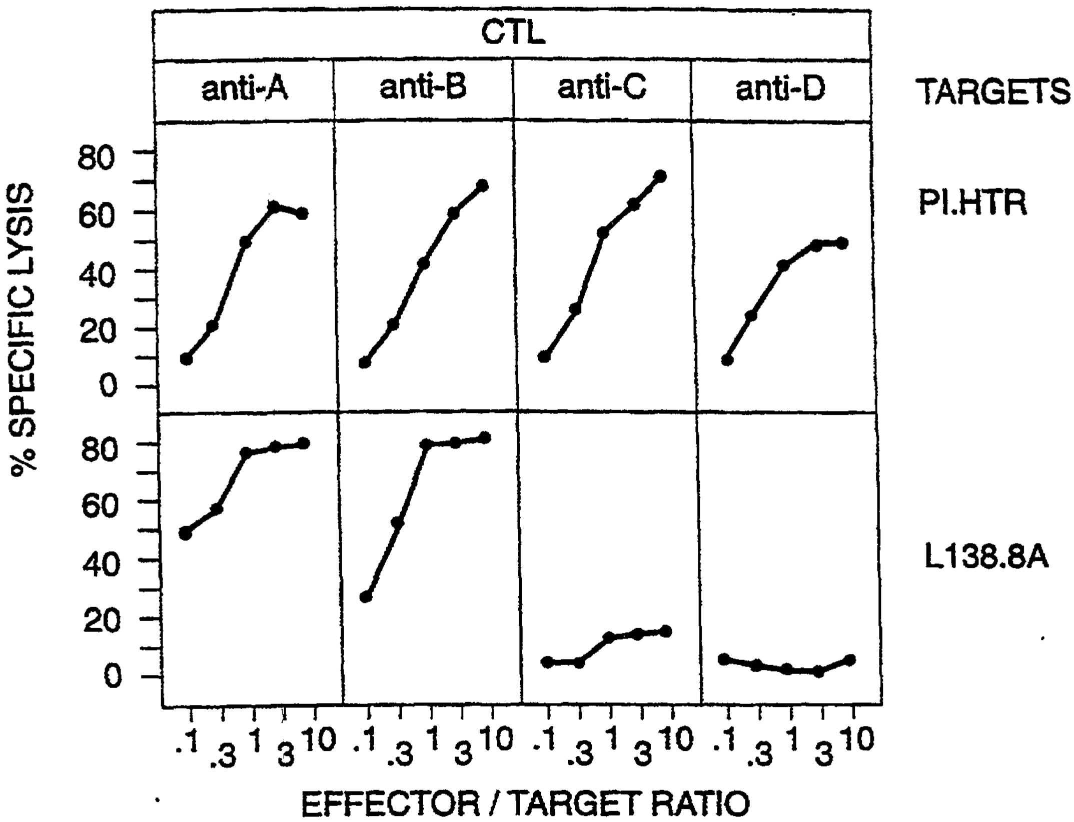
CTL
anti-A
anti-B
anti-C
anti-D
TARGETS
PI.HTR
L138.8A
% SPECIFIC LYSIS
80
60
40
20
0
.1 .3 1 3 10
EFFECTOR / TARGET RATIO

# FIG. 8

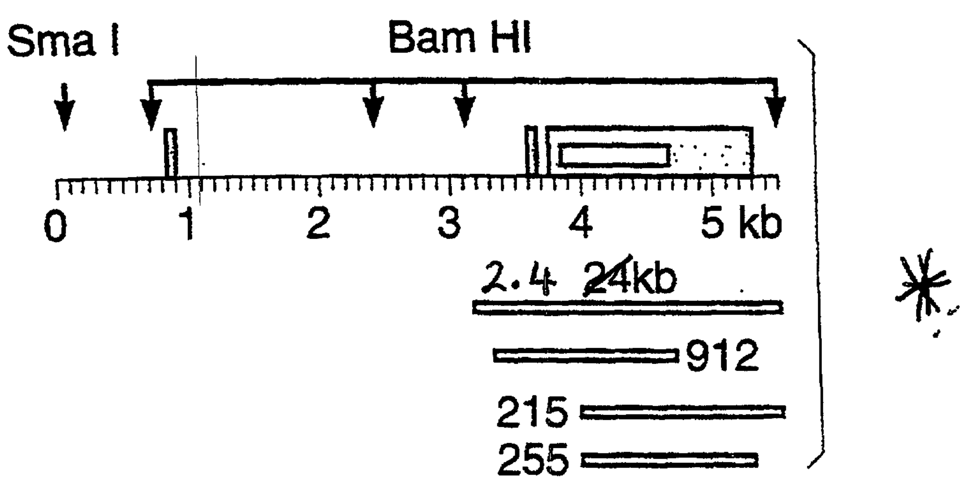
Sma I
Bam HI
0
1
2
3
4
5 kb
2.4 24kb
912
215
255

# FIG. 9

```
MAGE-3 III CCTCCCCAGAGTCCTCAGGGAGCCTCCagCcTcCCCACTACCATgAACTaCcCTCtctgGAGcCAAtCCtaTGAGGacTCCAGCAaCCaaGAAGAGGAGG
                                                                       <-------------------- CHO-3
MAGE-2 II  CCTCCCCAcAGTCCTCAGGGAGCCTCCagCTTctCgACTACCATCAACTaCACTCtttgGAGaCAAtCCgaTGAGGGcTCCAGCAaCCaaGAAGAGGAGG
                                                                       <-------------------- CHO-2
MAGE-1 I   CCTCCCCAGAGTCCTCAGGGAGCCTCCGCCTTTCCCACTACCATCAACTTCACTCGACAGAGGCAACCCAGTGAGGGTTCCAGCAGCCGTGAAGAGGAGG
       225  CHO-8 --------->

III GGCCAAGCACCTtcccTgaCC-TGGAGTCCgaGTTCCaAGCAGcAcTCAgTAgGAAGGTGGCcGAgTTGGTTcaTTTTCTGCTCCTCAAgTATCGAGCCA
II  GGCCAAGaAtgTtTcccgaCCtTGGAGTCCGAGTTCCaAGCAGcAATCAgTAgGAAGaTGGtTGAgTTGGTTcaTTTTCTGCTCCTCAAgTATCGAGCCA
I   GGCCAAGCACCTCTTGTATCC-TGGAGTCCTTGTTCCGAGCAGTAATCACTAAGAAGGTGGCTGATTTGGTTGGTTTTCTGCTCCTCAAATATCGAGCCA
325

III GGGAGCCgGTCACAAAGGCAGAAATGCTGGgGAGTGTCgTCggAAATTggcAGtAtTtcTTTCCTGtGATCTTCaGCAAAGCtTCcagtTCCTTGCAGCT
II  GGGAGCCgGTCACAAAGGCAGAAATGCTGGAGAGTGTCcTCAgAAATTgCcAGgACTtcTTTCCcGtGATCTTCaGCAAAGCCTCcGAGTaCTTGCAGCT
I   GGGAGCCAGTCACAAAGGCAGAAATGCTGGAGAGTGTCATCAAAAATTACAAGCACTGTTTTCCTGAGATCTTCGGCAAAGCCTCTGAGTCCTTGCAGCT
425                                                                <-------------------- SEQ-4

III GGTCTTTGGCATcGAgcTGAtGGAAGtgGACCCCAtCGGCCACTtgTAcaTCtTTGcCACCTGCCTgGGcCTCTCCTAcGATGGCCTGCTGGGTGAcAAT
II  GGTCTTTGGCATcGAgGTGgtGGAAGtgGtCCCCAtCaGCCACTtgTAcaTCCTTGTCACCTGCCTgGGcCTCTCCTAcGATGGCCTGCTGGGcGAcAAT
I   GGTCTTTGGCATTGACGTGAAGGAAGCAGACCCCACCGGCCACTCCTATGTCCTTGTCACCTGCCTAGGTCTCTCCTATGATGGCCTGCTGGGTGATAAT
525

III CAGATCATGCCCAAGgCAGGCcTCCTGATAATcGTCCTGGcCATaATcGCAAgaGAGGGCGaCtgTGCcCCTGAGGAGaAAATCTGGGAGGAGCTGAGTG
II  CAGgTCATGCCCAAGACAGGCcTCCTGATAATcGTC-TGGcCATaATcGCAATaGAGGGCGaCtgTGCcCCTGAGGAGaAAATCTGGGAGGAGCTGAGTa
I   CAGATCATGCCCAAGACAGGCTTCCTGATAATTGTCCTGGTCATGATTGCAATGGAGGGCGGCCATGCTCCTGAGGAGGAAATCTGGGAGGAGCTGAGTG
625                                                                          <-------------------- CHO-9
```

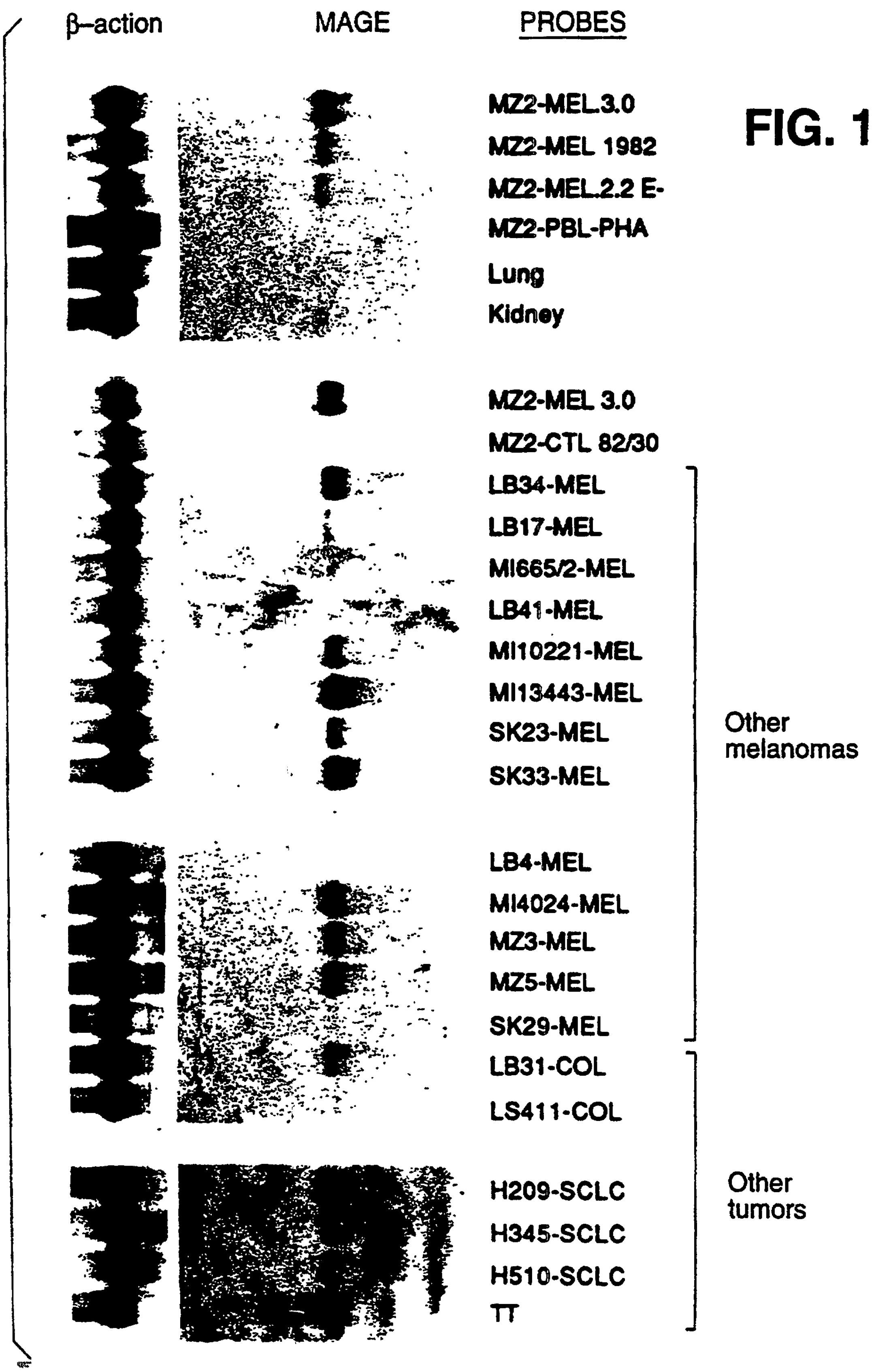
β–action
MAGE
PROBES
MZ2-MEL3.0
MZ2-MEL 1982
MZ2-MEL2.2 E-
MZ2-PBL-PHA
Lung
Kidney
MZ2-MEL 3.0
MZ2-CTL 82/30
LB34-MEL
LB17-MEL
MI665/2-MEL
LB41-MEL
MI10221-MEL
MI13443-MEL
SK23-MEL
SK33-MEL
LB4-MEL
MI4024-MEL
MZ3-MEL
MZ5-MEL
SK29-MEL
LB31-COL
LS411-COL
H209-SCLC
H345-SCLC
H510-SCLC
TT
Other melanomas
Other tumors

FIG. 10

# FIG. 11A

| | | EXPRSSION OF MAGE GENE FAMILY | | | | RECOGNITION BY ANI-E CTL | | |
|---|---|---|---|---|---|---|---|---|
| | | Northern blot probed with cross-reactive MAGE-1 probe* | cDNA-PCR product probed with oligonucleotide specific for: | | | tested by: | | Expression of antigen MZ2-E after transfection** |
| | | | MAGE-1 | MAGE-2 | MAGE-3† | TNF release‡ | Lysis§ | |
| Cells of patient MZ2 | melanoma cell line MZ2-MEL.3.0 | + | ++++ | ++++ | ++++ | + | + | |
| | tumor sample MZ2 (1982) | + | +++ | +++ | +++ | | | |
| | antigen-loss variant MZ2-MEL.2.2 | + | − | +++ | +++ | − | − | |
| | CTL clone MZ2-CTL.82/30 | − | − | − | − | | | |
| | PHA-activated blood lymphocytes | − | − | − | − | | | |
| Normal tissues | Liver | − | − | − | − | | | |
| | Muscle | − | − | − | − | | | |
| | Skin | − | − | − | − | | | |
| | Lung | − | − | − | − | | | |
| | Brain | − | − | − | − | | | |
| | Kidney | − | − | − | − | | | |
| Melanoma cell lines of HLA-A1 patients | LB34-MEL | + | ++ | ++++ | ++++ | + | +− | |
| | MI665/2-MEL | − | − | − | − | − | − | + |
| | MI10221-MEL | + | − | ++ | +++ | − | − | + |
| | MI13443-MEL | + | +++ | ++++ | ++++ | + | + | |
| | SK33-MEL | + | − | ++++ | ++++ | − | − | − |
| | SK23-MEL | + | − | ++++ | ++++ | − | − | + |

* Data obtained in the conditions of figure 5.
† Data obtained as described in figure 6.
‡ TNF release by CTL 82/30 after stimulation with the tumor cells as described in (11).
§ Lysis of 51 Cr labelled target by CTL 82/30 in the conditions of figure 1.
**Cells transfected with the 2.4 kb fragment of gene MAGE-1 were tested for their ability fo stimulate TNF release by CTL 82/30

# FIG. 11B

| | | EXPRSSION OF MAGE GENE FAMILY | | | | RECOGNITION BY ANI-E CTL | | |
|---|---|---|---|---|---|---|---|---|
| | | Northern blot probed with cross-reactive MAGE-1 probe* | cDNA-PCR product probed with oligonucleotide specific for: | | | tested by: | | Expression of antigen MZ2-E after transfection** |
| | | | MAGE-1 | MAGE-2 | MAGE-3† | TNF release‡ | Lysis§ | |
| Melanoma cell lines of other patients | LB17-MEL | + | + | ++++ | ++++ | − | − | − |
| | LB33-MEL | + | − | +++ | +++ | − | − | − |
| | LB4-MEL | − | − | − | − | − | − | |
| | LB41-MEL | − | − | − | − | − | − | |
| | MI4024-MEL | + | +++ | ++++ | ++++ | − | − | |
| | SK29-MEL | − | − | − | − | − | − | |
| | MZ3-MEL | + | + | ++++ | ++++ | − | − | |
| | MZ5-MEL | + | − | ++++ | ++++ | − | − | |
| Melanoma tumor sample | BB5-MEL | + | +++ | ++ | +++ | | | |
| Other tumor cell lines | small cell lung cancer H209 | + | − | ++++ | ++++ | | | |
| | small cell lung cancer H345 | + | − | ++++ | ++++ | | | |
| | small cell lung cancer H510 | + | − | ++++ | ++++ | | | |
| | small cell lung cancer LB11 | + | + | ++++ | ++++ | | | |
| | bronchial squamous cell carcinoma LB37 | + | − | − | +++ | | | |
| | thyroid medullary carcinoma TT | + | ++++ | +++ | ++++ | | | |
| | colon carcinoma LB31 | + | − | +++ | ++++ | | | |
| | colon carcinoma LS411 | − | − | − | − | − | | |
| Other tumor samples | chronic myeloid leukemia LLC5 | − | − | − | − | | | |
| | acute myeloid leukemia TA | − | − | − | − | | | |

* Data obtained in the conditions of figure 5.
† Data obtained as described in figure 6.
‡ TNF release by CTL 82/30 after stimulation with the tumor cells as described in (11).
§ Lysis of 51 Cr labelled target by CTL 82/30 in the conditions of figure 1.
**Cells transfected with the 2.4 kb fragment of gene MAGE-1 were tested for their ability fo stimulate TNF release by CTL 82/30

FIG. 12

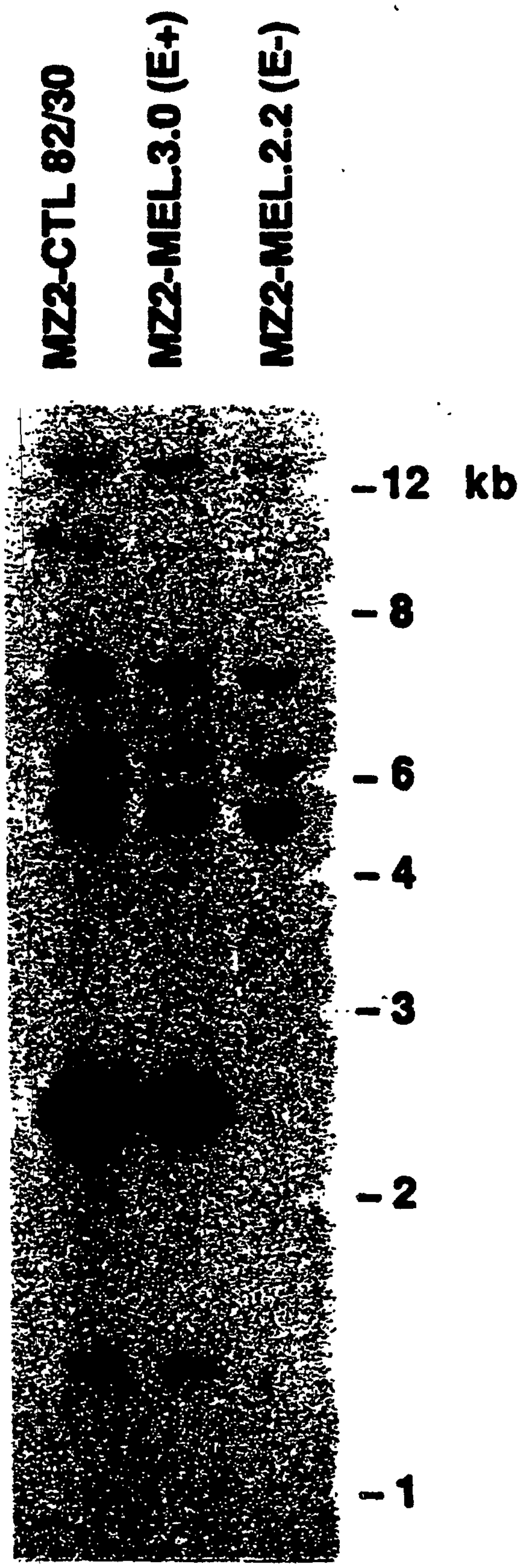

FIG. 13

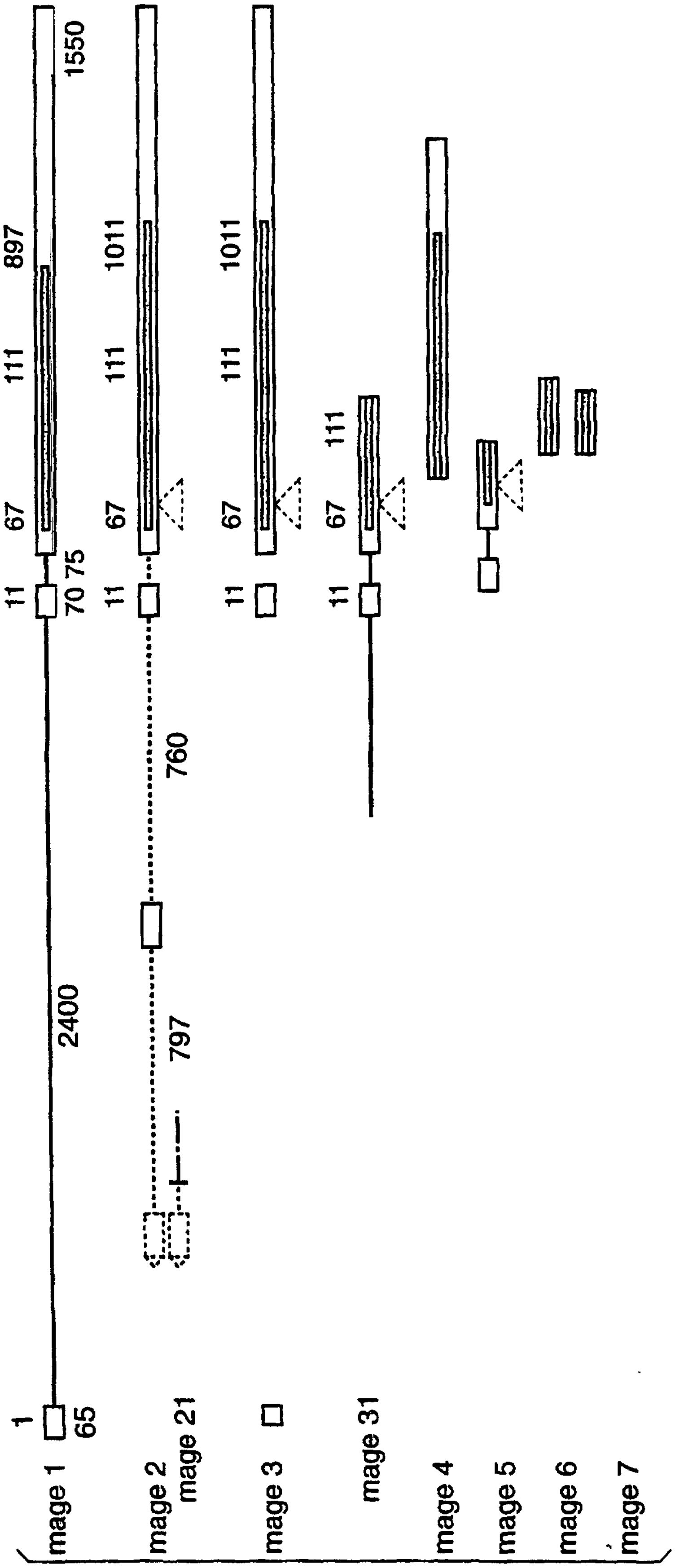
mage 1
mage 2
mage 21
mage 3
mage 31
mage 4
mage 5
mage 6
mage 7
1
65
2400
11
70 75
67
111
897
1550
797
760
1011

FIG. 14A

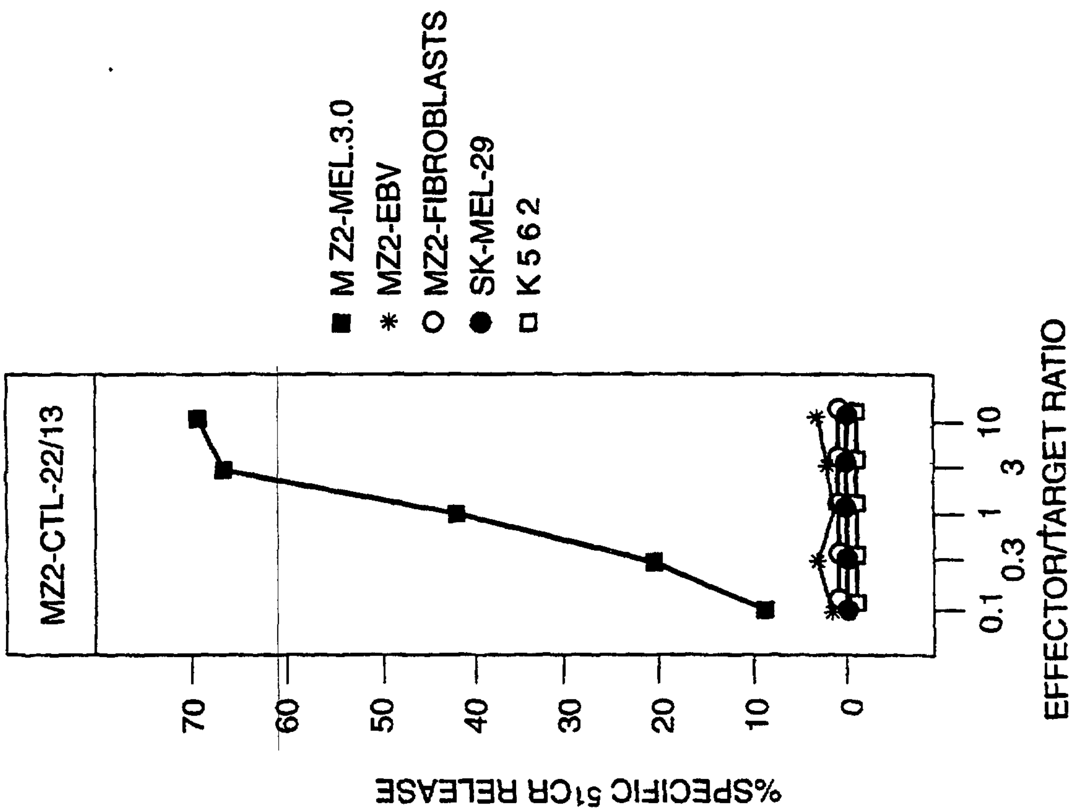


FIG. 14B

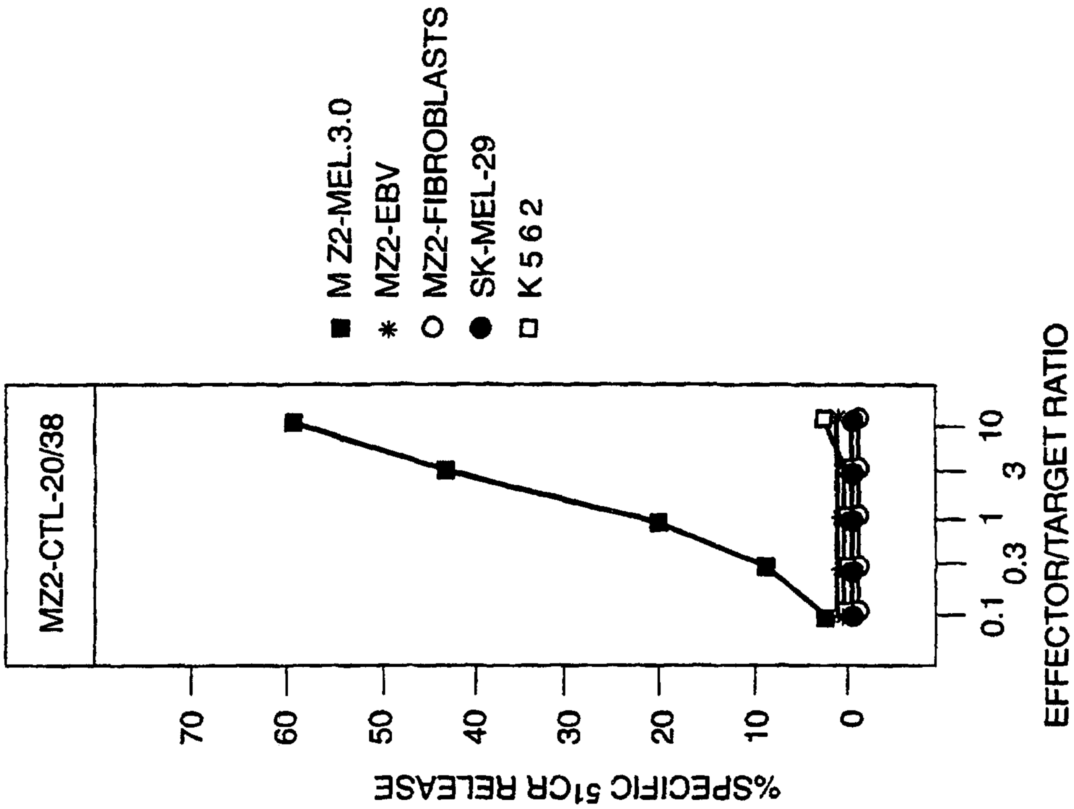

# FIG. 15A

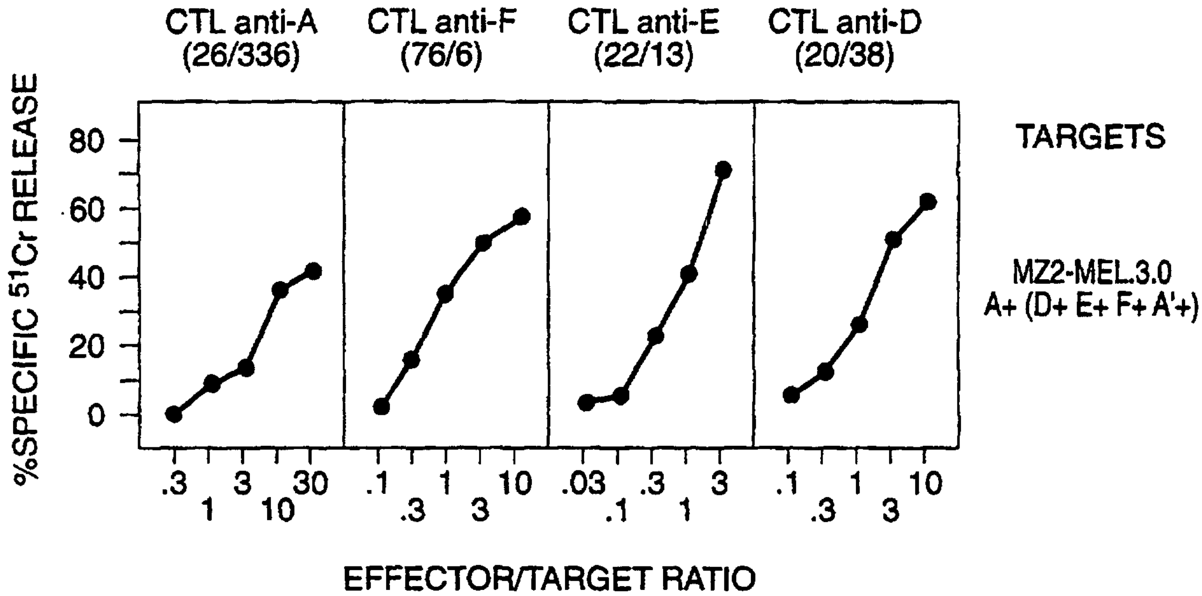


# FIG. 15B

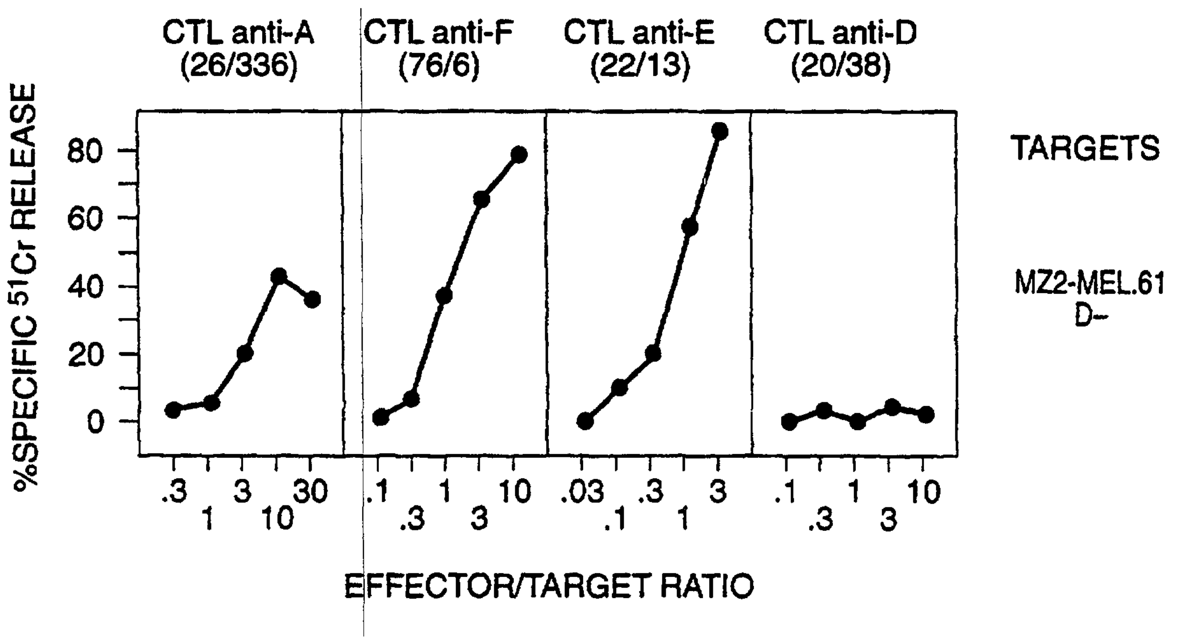

# FIG. 15C

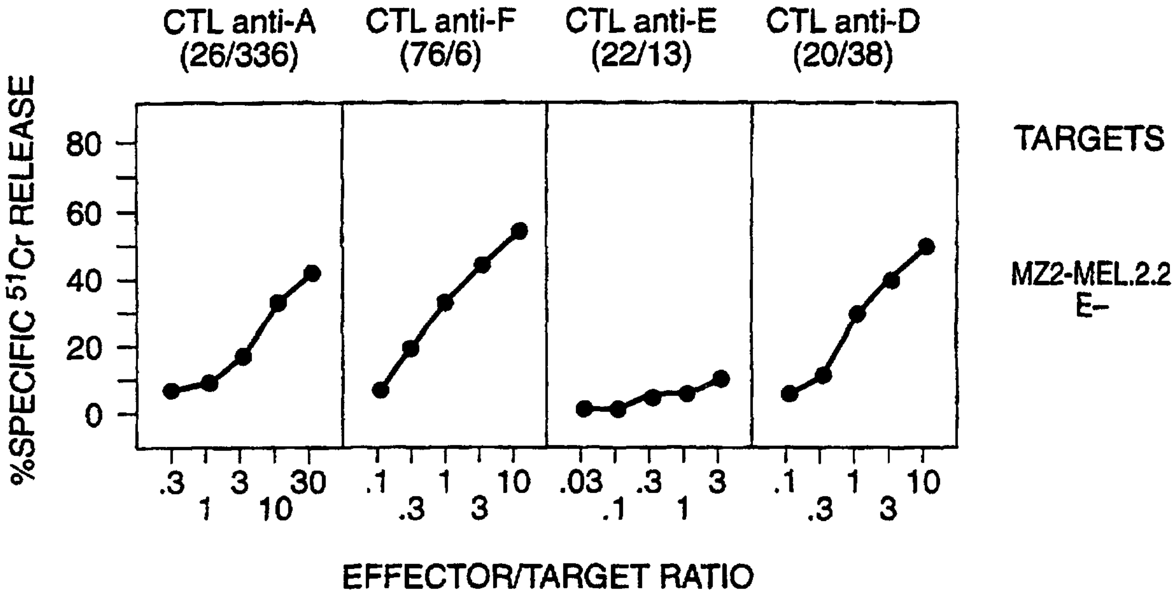


# FIG. 15D

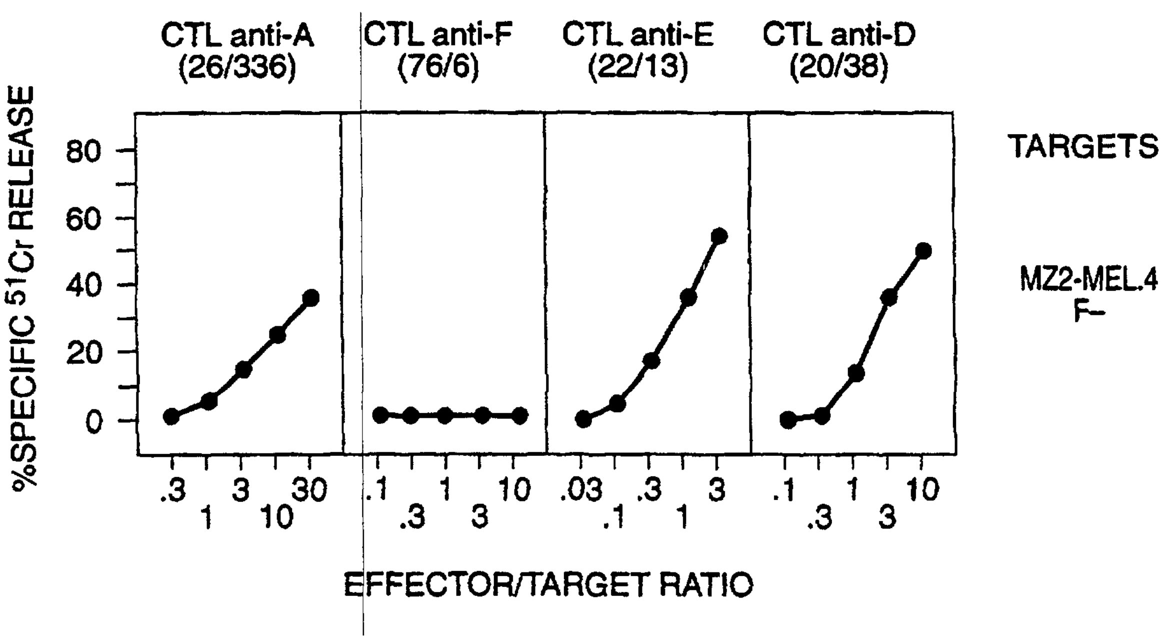

FIG. 16

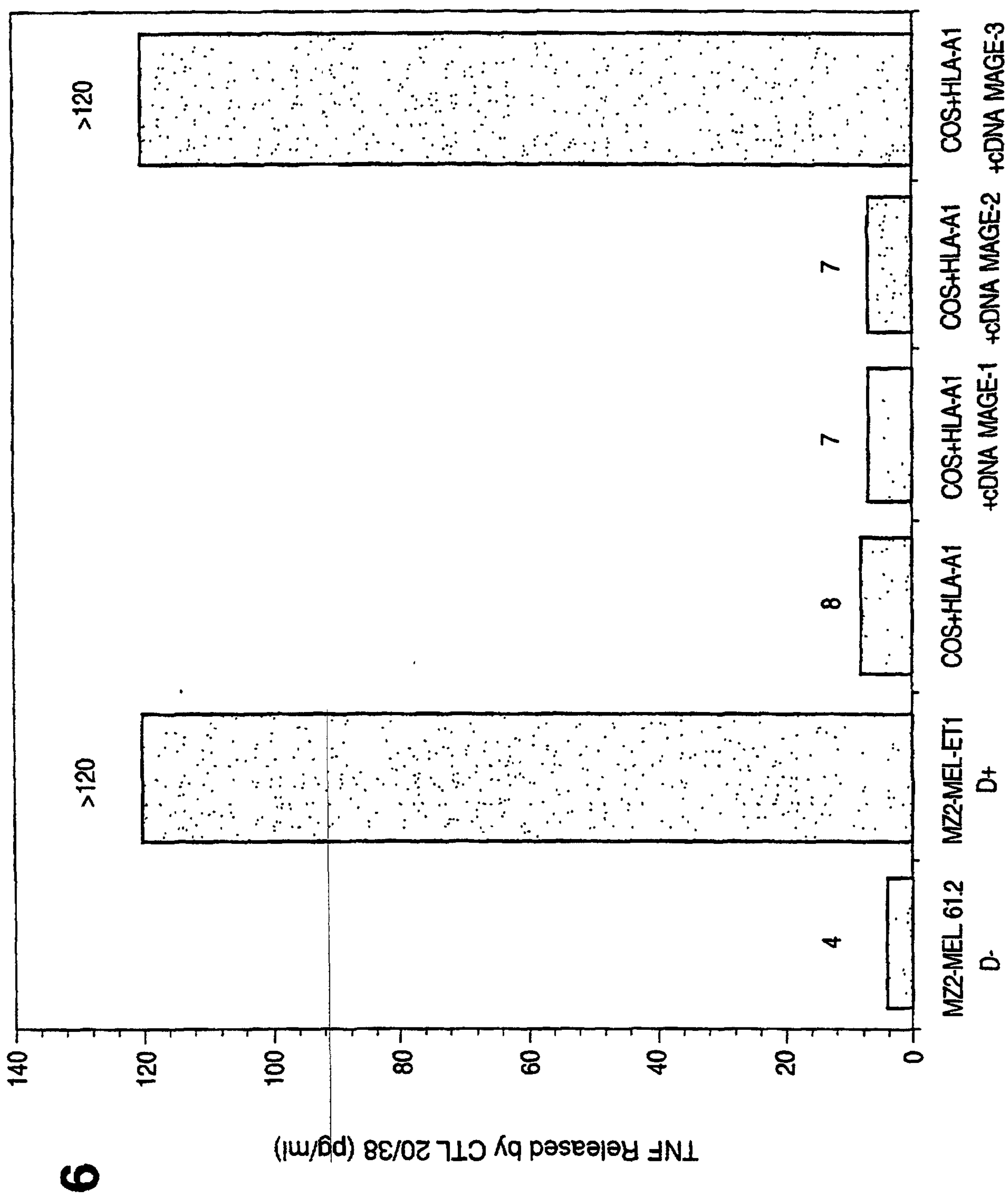
TNF Released by CTL 20/38 (pg/ml)
0
20
40
60
80
100
120
140
4
>120
8
7
7
>120
MZ2-MEL 61.2
D-
MZ2-MEL-ET1
D+
COS+HLA-A1
COS+HLA-A1
+cDNA MAGE-1
COS+HLA-A1
+cDNA MAGE-2
COS+HLA-A1
+cDNA MAGE-3

FIG. 17

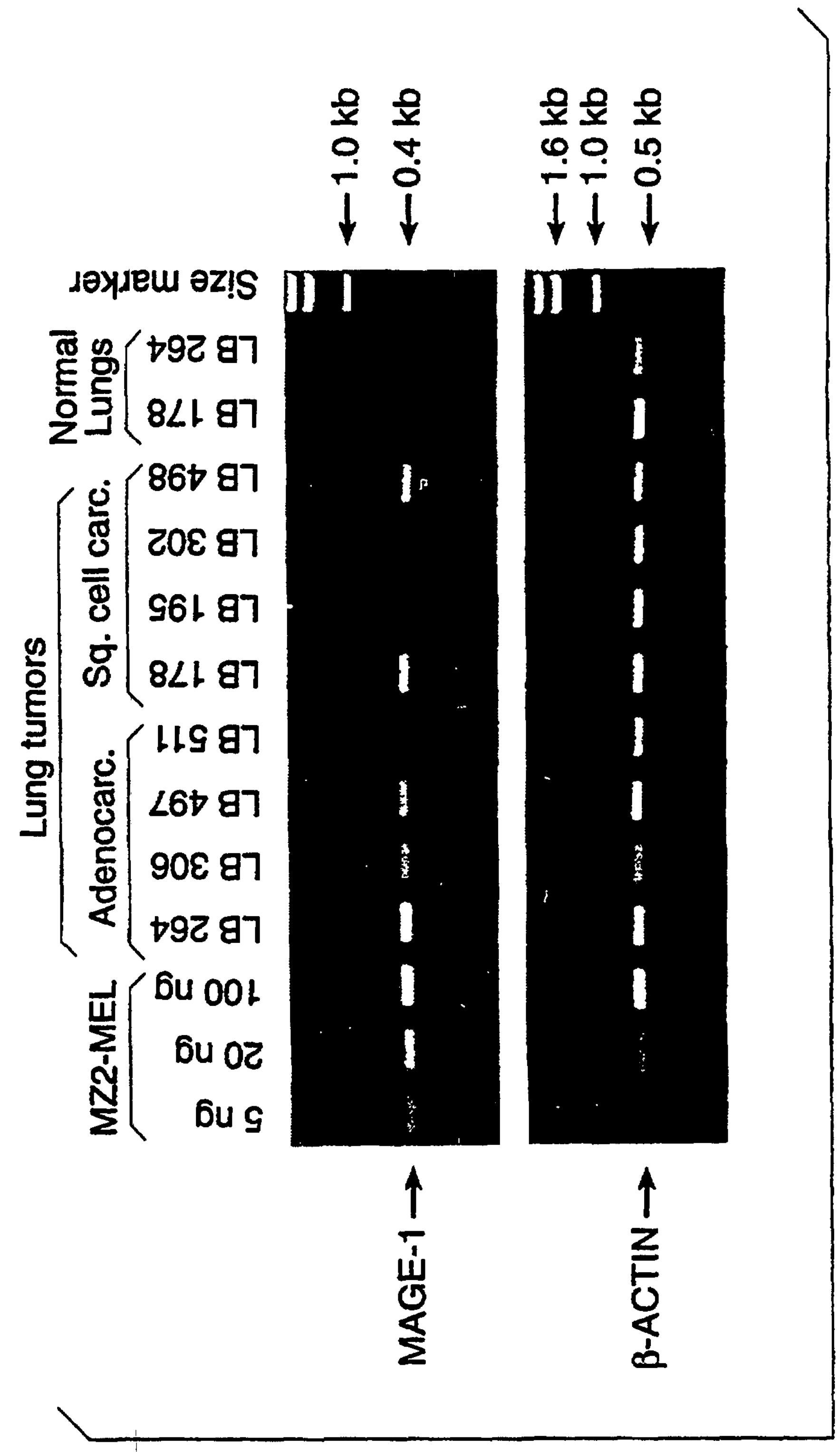
MAGE-1
β-ACTIN
MZ2-MEL
5 ng
20 ng
100 ng
Lung tumors
Adenocarc.
LB 264
LB 306
LB 497
LB 511
Sq. cell carc.
LB 178
LB 195
LB 302
LB 498
Normal Lungs
LB 178
LB 264
Size marker
1.0 kb
0.4 kb
1.6 kb
1.0 kb
0.5 kb

# FIG. 18

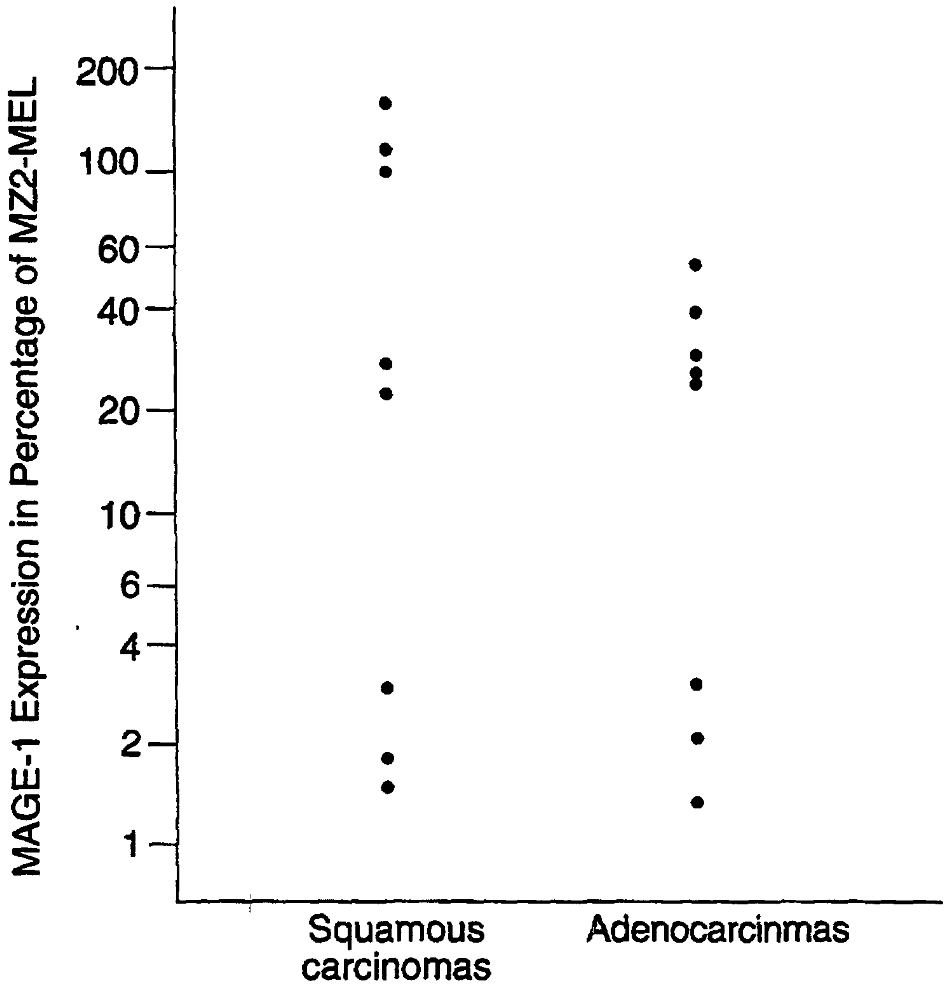
MAGE-1 Expression in Percentage of MZ2-MEL
200
100
60
40
20
10
6
4
2
1
Squamous carcinomas
Adenocarcinmas

# FIG. 19

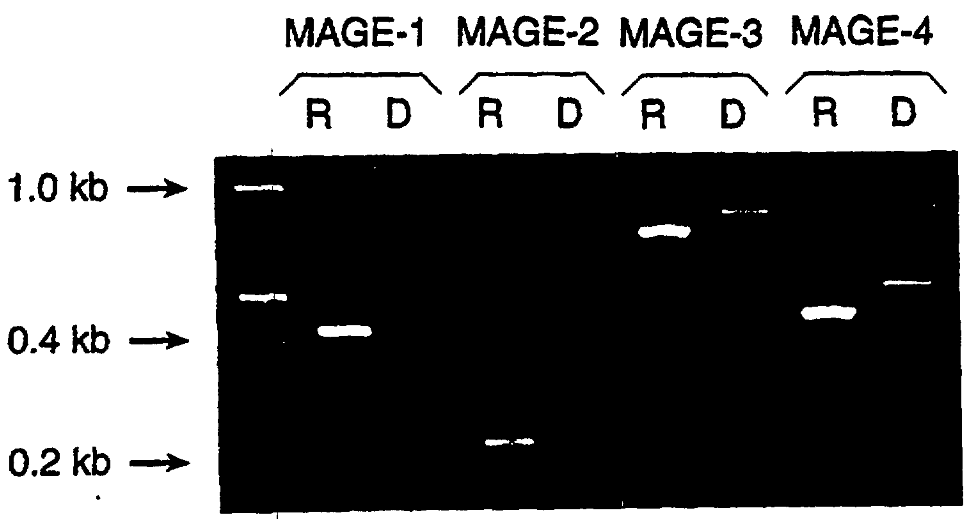

# FIG. 20

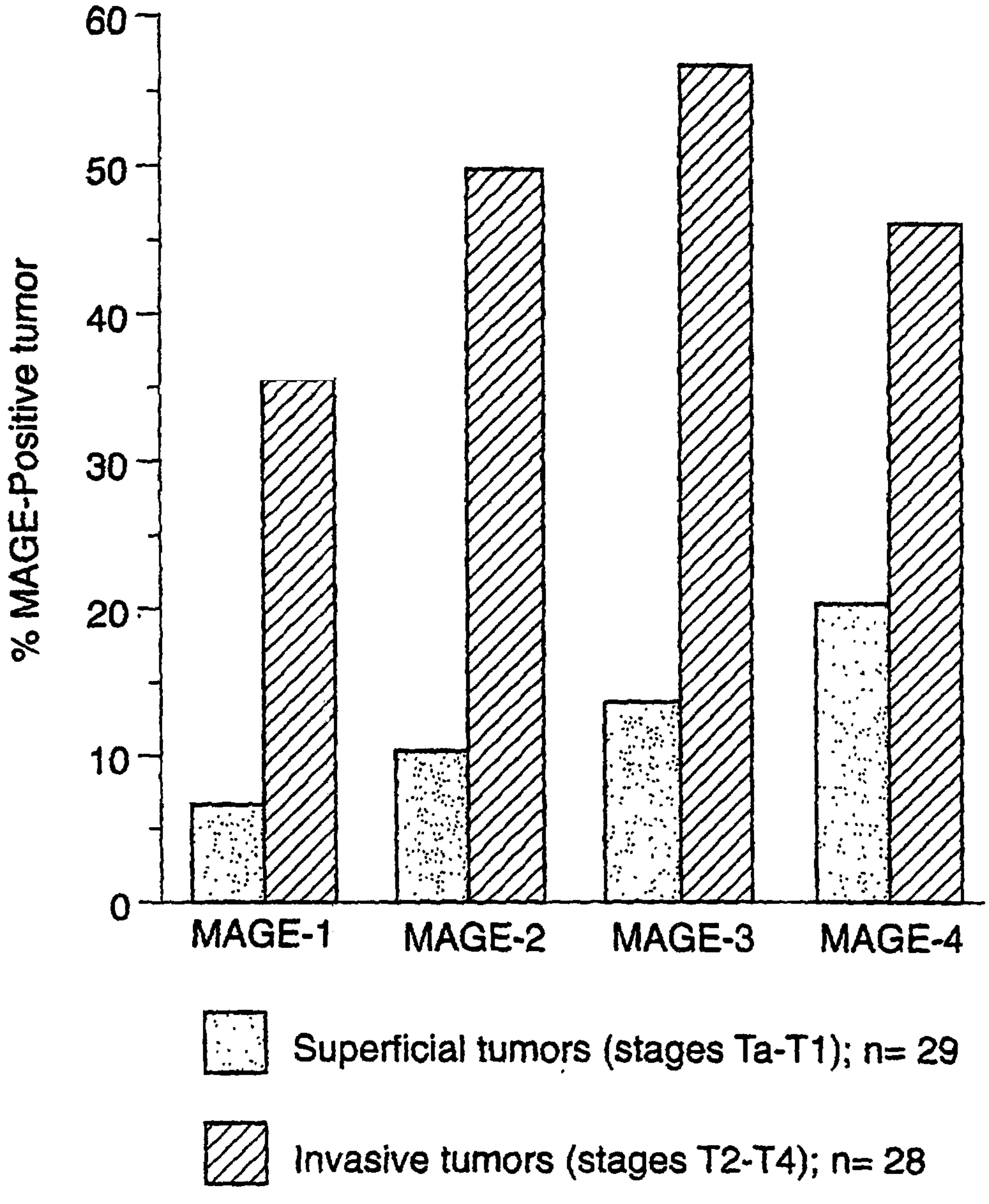
% MAGE-Positive tumor
0
10
20
30
40
50
60
MAGE-1
MAGE-2
MAGE-3
MAGE-4
Superficial tumors (stages Ta-T1); n= 29
Invasive tumors (stages T2-T4); n= 28